(19) Europäisches Patentamt
European Patent Office
Office européen des brevets

(11) EP 4 759 822 A1

(12) EUROPEAN PATENT APPLICATION
published in accordance with Art. 153(4) EPC

(43) Date of publication:
**17.06.2026 Bulletin 2026/25**

(21) Application number: **24851028.1**

(22) Date of filing: **07.08.2024**

(51) International Patent Classification (IPC):
***C07K 5/027*** *(2006.01)* ***A61K 47/68*** *(2017.01)*
***A61K 38/07*** *(2006.01)* ***A61P 35/00*** *(2006.01)*
***A61P 35/02*** *(2006.01)* ***C07K 5/062*** *(2006.01)*
***C07K 5/083*** *(2006.01)*

(86) International application number:
**PCT/CN2024/110323**

(87) International publication number:
**WO 2025/031383 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.08.2023 CN 202310988707**

(71) Applicant: **Hangzhou Adcoris Biopharma Co., Ltd**
**Hangzhou, Zhejiang 310052 (CN)**

(72) Inventors:
- **MIAO, Zhenwei**
**Hangzhou, Zhejiang 310052 (CN)**
- **HUANG, Yunsheng**
**Hangzhou, Zhejiang 310052 (CN)**
- **LI, Yaowu**
**Hangzhou, Zhejiang 310052 (CN)**
- **WENG, Shanhui**
**Hangzhou, Zhejiang 310052 (CN)**

(74) Representative: **Elzaburu S.L.P.**
**Edificio Torre de Cristal**
**Paseo de la Castellana 259 C, planta 28**
**28046 Madrid (ES)**

# (54) BIFUNCTIONAL LINKER COMPOUND, ANTIBODY DRUG CONJUGATE, PREPARATION METHOD FOR ANTIBODY DRUG CONJUGATE, AND USE OF ANTIBODY DRUG CONJUGATE

(57) The present application relates to a bifunctional linker compound, an antibody drug conjugate, a preparation method for an antibody drug conjugate, and a use of an antibody drug conjugate. The bifunctional linker compound is a compound represented by formula (I), or a pharmaceutically acceptable salt, stereoisomer or prodrug thereof. All groups in the formula are defined in the description. The compound of the present application not only can be used for preparing an antibody drug conjugate having improved homogeneity, but also can improve the structural stability of an antibody. M-L-D (I)

## Description

The present application claims the priority of Chinese patent application 2023109887077, filed on August 8, 2023, which is incorporated in the present application in its entirety.

Technical Field

The present disclosure relates to the field of antibody-drug conjugates. **In** particular, the present disclosure provides a range of drug-bifunctional linker compounds, drug-antibody conjugates, as well as preparation methods and use thereof.

Background Art

An antibody-drug conjugate or ADC is a novel and effective targeted drug composed of three moieties: an antibody, a linker, and a toxin. **In** the antibody-drug conjugate, the toxin (payload) is a key component for exerting efficacy, the antibody is a key moiety having a targeting function, and the linker not only links the two components together, but also affects the stability of the ADC, the release mechanism of the drug, the method of conjugating to the antibody, etc.

For traditional ADC products, two conjugation methods are used: the formation of an amide bond between a lysine (Lys) residue on the antibody and a carboxyl group on the toxin-linker (payload-linker), or a Michael addition between a maleimide linker and eight cysteine residues of four disulfide bonds in the reduced antibody. Therefore, most ADCs that have been available on the market to date with a drug-to-antibody ratio (DAR) of about 4 are mixtures with an average DAR value obtained by random conjugation, rather than homogeneous conjugates.

Summary of the Invention

The inventors of the present disclosure, through extensive research, have surprisingly discovered a range of bifunctional linker compounds that can be crosslinked with thiol groups of vicinal cysteine residues in reduced antibodies to form homogeneous antibody-drug conjugates. ADC drugs formed by the conjugation of these bifunctional linkers and antibodies not only improve the stability of the drug but also enhance efficacy, and have important application value for the treatment of tumors.

The bifunctional linker can simultaneously link two chemical functional groups, such as thiol groups of two cysteine residues formed by the reduction of a disulfide bond. Therefore, the thiol groups of eight cysteine residues formed by the reduction of four disulfide bonds can be linked to four bifunctional linkers in a saturated manner to form site-specific and stoichiometric antibody-drug conjugates.

In one aspect, the present disclosure relates to a compound of formula (I) or a pharmaceutically acceptable salt, stereoisomer, or prodrug thereof.

In another aspect, the present disclosure provides a drug-antibody conjugate or a pharmaceutically acceptable salt thereof.

In another aspect, the present disclosure provides a method for preparing the drug-antibody conjugate or the pharmaceutically acceptable salt thereof.

In another aspect, the present disclosure provides a bifunctional linker compound.

In another aspect, the present disclosure provides a pharmaceutical composition.

In another aspect, the present disclosure provides use of the drug-antibody conjugate or the pharmaceutically acceptable salt thereof, as described above, in the manufacture of a medicament for treating cancer.

In another aspect, the present disclosure provides a method for treating cancer, comprising the step of administering to a patient in need thereof a therapeutically effective amount of the compound or the pharmaceutically acceptable salt, stereoisomer, or prodrug thereof according to the present disclosure, the drug-antibody conjugate according to the present disclosure, the bifunctional linker compound according to the present disclosure, or the pharmaceutical composition according to the present disclosure.

The present disclosure can not only prepare an antibody-drug conjugate having improved homogeneity, but can also enhance the structural stability of the antibody.

Brief Description of the Drawings

FIG. 1 is a graph showing the effect of a drug-antibody conjugate in inhibiting tumor (pancreatic cancer) in Effect Example 2.

FIG. 2 is an image showing the effect of a drug-antibody conjugate in inhibiting tumor in a CFPAC-1 model.

FIG. 3 is a graph showing the effect of a drug-antibody conjugate in inhibiting tumor (breast cancer) in Effect Example 2.

FIG. 4 is an image showing the effect of a drug-antibody conjugate in inhibiting tumor in an MCF-7 model.

Detailed Description of Embodiments

**I. Definition**

The term "antibody" used in the present application is used in the broadest sense and specifically encompasses monoclonal antibodies, polyclonal antibodies, dimers, multimers, multispecific antibodies (e.g., bispecific antibodies), and antibody fragments, as long as they exhibit the desired biological activity. Antibodies can be mouse antibodies, human antibodies, humanized antibodies, chimeric antibodies, or antibodies derived from other species. Antibodies are proteins generated by the immune system and can recognize and bind to specific antigens. Target antigens typically have multiple binding sites, also known as epitopes, which are recognized by CDRs (complementarity determining regions) on various antibodies. Each antibody that specifically binds to a different epitope has a different structure. Therefore, an antigen can have more than one corresponding antibody. Antibodies include full-length immunoglobulin molecules or immunologically active portions of the full-length immunoglobulin molecules, i.e. molecules containing antigen-binding sites that immunospecifically bind to target antigens or portions thereof, including but not limited to cancer cells or cells that produce autoimmune antibodies associated with autoimmune diseases.

The term "antibody fragment" used in the present application comprises a portion of a full-length antibody, typically an antigen-binding region or variable region thereof. Examples of antibody fragments include Fab, Fab', F(ab')2, and Fv fragments; diabodies; linear antibodies; minibodies; fragments produced by an Fab expression library; anti-idiotypic (anti-Id) antibodies; CDRs (complementarity determining regions) and epitope-binding fragments of any of the antibodies described herein that immunospecifically bind to cancer cell antigens, viral antigens, or microbial antigens; single-chain antibody molecules; and multispecific antibodies formed from antibody fragments.

The term "monoclonal antibody" used in the present application refers to an antibody obtained from a population of substantially homogeneous antibodies, that is, individual antibodies that make up the population are the same, except for those with potentially naturally occurring mutations that may exist in small quantities. Monoclonal antibodies are highly specific and thus target single antigen sites. In addition, unlike polyclonal antibody preparations that comprise different antibodies targeting different determinants (epitopes), each monoclonal antibody targets a single determinant on an antigen. In addition to specificity, the advantage of monoclonal antibodies also lies in that the synthesis thereof is free from contamination by other antibodies. The modifier "monoclonal" indicates that the antibody is characterized by being obtained from a substantially homogeneous antibody population and should not be interpreted as requiring any specific method to produce the antibody. For example, the monoclonal antibodies used according to the present disclosure can be prepared first by using a hybridoma method, or can be prepared by a recombinant DNA method. Monoclonal antibodies can also be isolated from phage antibody libraries.

Monoclonal antibodies in the present description especially include "chimeric" antibodies, in which a portion of the heavy chain and/or light chain is identical or homologous to a corresponding sequence in an antibody from a specific species or belonging to a specific antibody class or subclass, while the rest of the chain is identical or homologous to a corresponding sequence in an antibody from another species or belonging to another antibody class or subclass, and fragments of such antibodies, as long as they exhibit the desired biological activity. Target chimeric antibodies herein include "primatized" antibodies, which comprise antigen-binding sequences of variable domains derived from non-human primates (e.g., Old World monkeys, and apes) and human constant region sequences.

The term "chimeric" antibody used in the present application refers to an antibody in which a portion of the heavy chain and/or light chain is derived from a specific source or species, while the rest of the heavy chain and/or light chain is derived from a different source or species. The term "intact antibody" used in the present application refers to an antibody that comprises VL and VH domains, as well as light chain constant domains (CL) and heavy chain constant domains, CH1, CH2, and CH3. The constant domain can be a native sequence constant domain (e.g., a human native sequence constant domain) or an amino acid sequence variant thereof. An intact antibody may have one or more "effector functions", which refer to those biological activities attributable to the Fc constant region of the antibody (a native sequence Fc region or an amino acid sequence variant Fc region). Examples of effector functions of antibodies include C1q binding; complement dependent cytotoxicity; Fc receptor binding; antibody dependent cell-mediated cytotoxicity (ADCC); phagocytosis; and down-regulation of cell surface receptors (such as B cell receptors and BCR).

The term "antibody-drug conjugate (ADC)" used in the present application means that a biologically active small molecule drug is linked to a monoclonal antibody via a chemical linkage. The monoclonal antibody serves as a carrier to transport the small molecule drug to the target cell in a targeted manner.

The term "bifunctional linker" used in the present application refers to a structure that can simultaneously link two

chemical functional groups (specifically, an antibody and a cytotoxic drug moiety). The structure has a group linked to an antibody and a group linked to a cytotoxic drug moiety. The specific structure thereof is as represented by M hereinafter.

The term "spacer unit E" used in the present application, also known as self-immolative structure, is a linker group located between L and D. The spacer unit (E) is selected from one of p-aminobenzyl carbamate (PAB carbamate), bis(p-aminobenzyl) carbonate (PAB carbonate), hemiaminal, and methylenediamine.

The term "spacer unit F" used in the present application refers to a linker group located between M and L. The spacer unit F is $-NH-(CH_2CH_2-O)_q-CH_2CH_2-C(=O)-$ or a single bond, wherein q represents an integer from 1 to 6.

The term "cytotoxic drug moiety" used in the present application, also known as "small molecule drug", refers to a compound that has a killing effect on tumor cells. As the cytotoxic drug moiety, at least one of an anti-tubulin agent, a DNA intercalator, a DNA topoisomerase inhibitor, a DNA synthesis inhibitor, an RNA polymerase inhibitor, a spliceosome inhibitor, proteolysis-targeting chimera (PROTAC), and an immunomodulator can be listed.

The immunomodulator includes but is not limited to immunosuppressants and agonists against targets including PD-1/PD-L1, PD-L2, CTLA-4, LAG-3, IDO, TIM3, TIGIT, CD47, SIRPα, 4-1BB, CSF-1/CSF1R, GITR, OX40, CD40, CD27, CD28, B7H4, B7H3, TGFβ, BTLA, VISTA, ICOS, CD39, CD73, A2AR, KIR, NKG2A, etc.; and cell therapies associated with immunotherapy.

The small molecule drugs used in the present application may be asymmetric, for example, having one or more stereoisomers. Unless otherwise specified, all stereoisomers include, for example, enantiomers and diastereomers. The stereoisomers include geometric isomers (such as cis and trans structures) and optical isomers (such as enantiomers), as well as therapeutics composed of monomers, racemates, racemic mixtures, and pharmaceutically acceptable salts thereof. The compound containing asymmetric carbon atoms in the present application can be isolated in an optically active pure form or a racemic form. The optically active pure form can be resolved from a racemic mixture or synthesized using a chiral raw material or a chiral reagent. Racemates, diastereomers, and enantiomers are all included within the scope of the present application.

The small molecule drugs used in the present application also include tautomeric forms. The tautomeric form originates from the exchange of a single bond with an adjacent double bond, accompanied by the migration of a proton.

The term "precursor" used in the present application means that after the compound enters the human body by an appropriate mode of administration, the precursor compound undergoes metabolism or simple chemical change in a patient and transforms into a compound encompassed by the general formula (I) and the form of a corresponding salt thereof according to the present disclosure. The precursor of the compound includes, but is not limited to, various carboxylates, carbonates, phosphates, sulfates, sulfonates, amino acid esters, and gluconates, as well as various forms such as amides, acetals, hemiacetals, and carbamates.

The numerical ranges herein refer to all integers within given ranges. For example, "$C_1$-$C_6$" means that the group can have 1 carbon atom, 2 carbon atoms, 3 carbon atoms, 4 carbon atoms, 5 carbon atoms, or 6 carbon atoms; and "$C_3$-$C_6$" means that the group can have 3 carbon atoms, 4 carbon atoms, 5 carbon atoms, or 6 carbon atoms.

When any variable (e.g., $R_n$) occurs more than once in the composition or structure of a compound, the definition thereof at each occurrence is independent. Therefore, for example, if a group is substituted with 1-5 R groups, the group can optionally be substituted with at most 5 R groups, and R at each occurrence is independently selected. In addition, a combination of substituents and/or variants thereof is allowed only if such a combination results in a stable compound.

The term "alkyl" used in the present application refers to a saturated aliphatic hydrocarbon group, which is a linear or branched group containing 1 to 20 carbon atoms, preferably an alkyl group containing 1 to 8 carbon atoms, more preferably an alkyl group containing 1 to 6 carbon atoms, and most preferably an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, various branched isomers thereof, etc. More preferably, the term refers to lower alkyl groups containing 1 to 6 carbon atoms, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. The alkyl can be substituted or unsubstituted. When the alkyl is substituted, the substituent can be substituted at any available point of attachment. The substituent is preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group. In the present application, methyl, ethyl, isopropyl, tert-butyl, haloalkyl, deuterated alkyl, alkoxy-substituted alkyl, and hydroxyl-substituted alkyl are preferred.

The term "heteroalkyl" used in the present application, by itself or in combination with another term, means a stable linear or branched hydrocarbon radical or a combination thereof, consisting of a certain number of carbon atoms and at least one heteroatom. In a typical embodiment, the heteroatom is selected from B, O, N, and S, where the nitrogen and sulfur atoms are optionally oxidized, and the nitrogen heteroatom is optionally quaternized. The heteroatom or heteroatom radical can be located at any internal position of the heterohydrocarbyl group, including the position where the hydrocarbon group is attached to the rest of the molecule. Examples include but are not limited to $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-S-CH_2$, $-S(O)-CH_3$, and $-CH_2-CH_2-S(O)_2-CH_3$. However, the terms "alkoxy", "alkylamino", and "alkylthio" are customary expressions and refer to those alkyl groups that are attached to the rest of the molecule via an oxygen atom, amino, or sulfur atom, respectively. "Alkoxy" refers to -O-alkyl, "alkylamino" refers to -NH-alkyl, and "alkylthio" refers to -S-alkyl, wherein the alkyl is defined as previously.

The term "alkenyl" used in the present application is an unsaturated linear or branched hydrocarbon group, especially alkenyl having 2 to 10 carbon atoms and a double bond at any desired position, such as $C_2$-$C_6$ alkenyl, e.g., vinyl, 1-propenyl, 2-propenyl, 1-methyl-vinyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-vinyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, and 1-ethyl-2-methyl-2-propenyl.

The term "heterocyclyl" or "heterocycloalkyl ring" used in the present application refers to a saturated or partially unsaturated monocyclic or polycyclic cyclic hydrocarbon substituent, which contains 3 to 20 ring atoms, wherein one or more ring atoms are heteroatoms selected from nitrogen, oxygen, or S(O)m (in which m is an integer from 0 to 2), exclusive of the ring moiety -O-O-, -O-S-, or -S-S-, and the remaining ring atoms are carbon. The term preferably contains 3 to 12 ring atoms, of which 1-4 are heteroatoms; more preferably 3 to 8 ring atoms; most preferably 3 to 8 ring atoms; further preferably, the term refers to a 3- to 8-membered heterocyclyl group containing 1-3 nitrogen atoms, optionally substituted with 1-2 oxygen atoms, sulfur atoms, or oxo groups, including nitrogen-containing monocyclic heterocyclyl groups, nitrogen-containing spiroheterocyclic groups, or nitrogen-containing fused heterocyclic groups.

The term "aryl" used in the present application refers to a 6- to 14-membered all-carbon monocyclic or fused polycyclic (i.e., rings sharing a pair of adjacent carbon atoms) group having a conjugated $\pi$-electron system, preferably 6- to 12-membered, e.g., phenyl and naphthyl.

The aryl can be substituted or unsubstituted. When the aryl is substituted, the substituent is preferably one or more groups independently selected from (halo)alkyl, alkenyl, alkynyl, alkoxy, alkylthio, alkylamino, halogen, mercapto, hydrogen, nitro, cyano, cycloalkyl, heterocycloalkyl, aryl, heteroaryl, cycloalkoxy, heterocycloalkoxy, cycloalkylthio, heterocycloalkylthio, carboxyl, or carboxylate group.

The term "alkoxy" used in the present application refers to -O-(alkyl) and -O-(unsubstituted cycloalkyl), wherein the alkyl is defined as previously. Non-limiting examples of alkoxy include: methoxy, ethoxy, propoxy, butoxy, cyclopropoxy, cyclobutoxy, cyclopentyloxy, and cyclohexoxy. The alkoxy can be optionally substituted or unsubstituted. When the alkoxy is substituted, the substituent is preferably one or more groups independently selected from alkyl, alkenyl, alkynyl, alkoxy, alkylthio, alkylamino, halogen, mercapto, hydrogen, nitro, chloro, cycloalkyl, heterocycloalkyl, aryl, heteroaryl, cycloalkoxy, heterocycloalkoxy, cycloalkylthio, heterocycloalkylthio, carboxyl, or carboxylate group.

The term "heteroaryl" used in the present application is used interchangeably in the present application and refers to a saturated or partially unsaturated (i.e., having one or more double and/or triple bonds in the ring) carbocyclic group having 3 to about 20 ring atoms, wherein at least one ring atom is a heteroatom selected from nitrogen, oxygen, phosphorus, and sulfur, and the remaining ring atoms are C, wherein one or more ring atoms are optionally independently substituted with one or more of the following substituents. A heterocycle may be a monocyclic ring having 3 to 7 ring atoms (2 to 6 carbon atoms and 1 to 4 heteroatoms selected from N, O, P, and S) or a bicyclic ring having 7 to 10 ring atoms (4 to 9 carbon atoms and 1 to 6 heteroatoms selected from N, O, P, and S), such as a bicyclo[4,5], [5,5], [5,6], or [6,6] system. The "heterocyclyl" also includes groups in which a heterocyclyl group is fused to a saturated ring, a partially unsaturated ring, or an aromatic carbocyclic, or a heterocyclyl ring. Examples of heterocyclyl include, but are not limited to, morpholin-4-yl, piperidin-1-yl, piperazinyl, piperazin-4-yl-2-one, piperazin-4-yl-3-one, pyrrolidin-1-yl, thiomorpholin-4-yl, S,S-dioxothiomorpholin-4-yl, azocan-1-yl, azetidin-1-yl, octahydropyrido[1,2-a]pyrazin-2-yl, [1,4]diazepan-1-yl, pyrrolidinyl, tetrahydrofuranyl, dihydrofuranyl, tetrahydrothiophenyl, tetrahydropyranyl, dihydropyranyl, tetrahydrothiopyranyl, piperidinyl, morpholinyl, thiomorpholinyl, thiaoxacyclohexyl, piperazinyl, homopiperazinyl, azetidinyl, oxetanyl, thietanyl, homopiperidinyl, oxepanyl, thiepanyl, oxa-aza-heterocyclyl, diaza-heterocyclyl, thia-aza-heterocyclyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothiophenyl, dihydrofuranyl, pyrazolidinyl, imidazolinyl, imidazolidinyl, 3-azabicyclo[3.1.0]hexyl, 3-azabicyclo[4.1.0]heptyl, azabicyclo[2.2.2]hexyl, 3H-indolyl, quinolizidinyl, and N-pyridinylurea. Spirocyclic moieties are also encompassed within the scope of this definition. Examples of heterocyclyl in which two ring atoms are substituted with an oxo (=O) moiety include pyrimidinonyl and 1,1-dioxo-thiomorpholinyl. In the present application, heterocycles are optionally independently substituted with one or more substituents described in the present application.

The term "substituted" used in the present application means that one or more hydrogen atoms in a group, preferably at most 5, more preferably 1-3 hydrogen atoms, are each independently replaced by a corresponding number of substituents. It goes without saying that substituents are only in their possible chemical positions, and those skilled in the art can determine (by experiment or theory) possible or impossible substitutions without excessive efforts. For example, amino or hydroxyl having free hydrogen may be unstable when combined with carbon atoms having unsaturated (such as olefinic) bonds.

"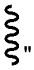" refers to a site to which a chemical bond is attached.

Although the term "optionally substituted" does not appear, if any position on a ring (e.g., an aliphatic ring or aromatic ring) is substituted, it should be understood that the positions and number of substitutions are arbitrary, as long as they are chemically feasible.

The term "pharmaceutically acceptable salt" used in the present application refers to a salt formed between a corresponding amine compound and an inorganic or organic acid, or a salt formed between a corresponding carboxylic acid compound and an alkali metal or alkaline earth metal, or a salt formed with an organic amine. The inorganic acid includes but is not limited to hydrochloric acid, hydrobromic acid, hydroiodic acid, sulfuric acid, phosphoric acid, etc. The organic acid includes but is not limited to acetic acid, propionic acid, butyric acid, benzoic acid, methanesulfonic acid, benzenesulfonic acid, p-toluenesulfonic acid, oxalic acid, succinic acid, lactic acid, citric acid, succinic acid, gluconic acid, maleic acid, fumaric acid, tartaric acid, etc. The alkali or alkaline earth metal salt includes but is not limited to sodium, potassium, calcium, magnesium salts, etc. The organic amine salt includes but is not limited to salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids, etc.

The drug or pharmaceutical composition of the present application can be administered orally, topically, parenterally, or mucosally (for example, buccally, by inhalation, or rectally) in dosage unit formulations containing conventional non-toxic pharmaceutically acceptable carriers. Oral administration is generally desired. The active agent can be administered orally in the form of capsules, tablets, etc. (see Remington: The Science and Practice of Pharmacy, 20th Edition).

For oral administration in the form of tablets or capsules, the active pharmaceutical component may be combined with non-toxic, pharmaceutically acceptable excipients, such as binders (e.g., pregelatinized corn starch, polyvinylpyrrolidone, or hydroxypropyl methylcellulose); fillers (e.g., lactose, sucrose, glucose, mannitol, sorbitol, and other reducing and non-reducing sugars, microcrystalline cellulose, calcium sulfate, or calcium hydrogen phosphate); lubricants (e.g., magnesium stearate, talc or silica, stearic acid, sodium stearyl fumarate, glyceryl behenate, and calcium stearate); disintegrants (e.g., potato starch or sodium starch glycolate); or wetting agents (e.g., sodium lauryl sulfate), colorants and flavoring agents, gelatin, sweeteners, natural and synthetic gums (e.g., arabic gum, tragacanth, or alginates), buffering salts, carboxymethyl cellulose, polyethylene glycols, waxes, etc. For oral administration in a liquid form, the drug component can be combined with non-toxic, pharmaceutically acceptable inert carriers (e.g., ethanol, glycerol, and water), anti-settling agents (e.g., sorbitol syrup, cellulose derivatives, or hydrogenated edible fats), emulsifiers (e.g., lecithin or arabic gum), non-aqueous carriers (e.g., almond oil, oil esters, ethanol, or fractionated vegetable oil), preservatives (e.g., methyl p-hydroxybenzoate or propyl p-hydroxybenzoate or sorbic acid), etc. Stabilizers such as antioxidants (BHA, BHT, propyl citrate, sodium ascorbate, and citric acid) can also be added to stabilize the dosage form.

Tablets containing active compounds can be coated by well-known methods in the art. The composition of the present application containing the compound of formula I as an active compound may also incorporate beads, microspheres, or microcapsules, for example, those made of poly(glycolic acid/lactic acid) (PGLA). Preparations of liquids for oral administration may take the form of, for example, solutions, syrups, emulsions, or suspensions, or they may appear as dry products reconstituted with water or other suitable excipients before use. Formulations for oral administration can be appropriately formulated to achieve controlled or delayed release of the active compound.

The term "treatment" used in the present application includes the inhibition, alleviation, prevention, or elimination of one or more symptoms or side effects associated with the disease, condition, or disorder being treated.

The use of the term "inhibition" in the present application is relative to a control. Those skilled in the art will readily determine the appropriate control for each experiment. For example, a reduced response in a subject or cell treated with a compound is compared with a response in a subject or cell not treated with the compound.

The term "pharmaceutical composition" used in the present application means a composition comprising the compound or the pharmaceutically acceptable salt according to the present application, and, depending on the mode of administration and the nature of the dosage form, at least one pharmaceutically acceptable ingredient selected from ingredients including but not limited to: carriers, diluents, adjuvants, excipients, preservatives, fillers, disintegrants, wetting agents, emulsifiers, suspending agents, sweeteners, corrigents, flavoring agents, antibacterial agents, antifungal agents, lubricants, dispersants, thermosensitive materials, temperature regulators, binders, stabilizers, suspending agents, etc.

The term "tumor" or "cancer" used in the present application refers to a disease characterized by pathological proliferation of cells or tissues, and subsequent migration or invasion of other tissues or organs. Tumor growth is usually uncontrolled and progressive, without inducing or inhibiting normal cell proliferation. Tumors can affect various cells, tissues, or organs, including but not limited to those selected from bladder, bone, brain, breast, cartilage, glial cells, esophagus, fallopian tubes, gallbladder, heart, intestine, kidney, liver, lung, lymph nodes, nerve tissue, ovary, pancreas, prostate, skeletal muscle, skin, spinal cord, spleen, stomach, testis, thymus, thyroid, trachea, urethra, ureter, urethra, uterus, vaginal organs, or tissues or corresponding cells. Tumors include cancers such as sarcomas, carcinomas, or plasmacytomas (malignant tumors of plasma cells). The tumors described in the present disclosure may include, but are not limited to, leukemia (such as acute leukemia, acute lymphoblastic leukemia, acute myelogenous leukemia, acute myeloblastic leukemia, acute promyelocytic leukemia, acute myelo-monocytic leukemia, acute monocytic leukemia, chronic leukemia, chronic myeloblastic leukemia, chronic lymphoblastic leukemia, and polycythemia vera), lymphoma (Hodgkin's disease and non-Hodgkin's disease), primary macroglobulinemia, heavy chain disease, solid tumors such as sarcoma and cancer (such as fibrosarcoma, myxosarcoma, liposarcoma, chondrosarcoma, osteosarcoma, chordoma, endothelial sarcoma, lymphangiosarcoma, angiosarcoma, lymphangion endothelial sarcoma, mesothelioma, Ewing's tumor, leiomyosarcoma, rhabdomyosarcoma, colon cancer, rectal cancer, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinoma, bronchial carcinoma, medullary carcinoma, renal cell carcinoma, liver cancer, Nile duct carcinoma, choriocarcinoma, seminoma, embryonal carcinoma, nephroblastoma, cervical cancer, uterine cancer, testicular cancer, lung cancer, small cell lung cancer, bladder cancer, epithelial cancer, glioma, astrocytoma, medulloblastoma, craniopharyngioma, ependymoma, pinealoma, hemangioblastoma, acoustic neuroma, oligodendroglioma, schwannoma, meningioma, melanoma, neuroblastoma, and retinoblastoma), esophageal cancer, gallbladder cancer, renal cancer, and multiple myeloma. Preferably, the "tumor" includes but is not limited to: pancreatic cancer, liver cancer, lung cancer, gastric cancer, esophageal cancer, head and neck squamous cell cancer, prostate cancer, colon cancer, rectal cancer, breast cancer, lymphoma, gallbladder cancer, kidney cancer, leukemia, multiple myeloma, ovarian cancer, cervical cancer, and glioma.

## II Embodiments

In a first aspect, the present application provides a drug-bifunctional linker compound represented by formula (I), or a pharmaceutically acceptable salt, stereoisomer, or prodrug thereof:

M-L-D (I)

wherein,

M is a bifunctional linker linked to an antibody or an antigen-binding fragment thereof;
L is a linker between the bifunctional linker M and D;
D is a cytotoxic drug moiety;
said M represents a structure represented by formula (II) below:

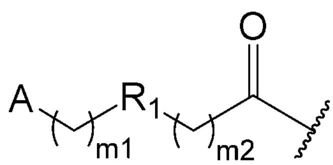
(II)

in formula (II), A is selected from alkenyl, a monocyclic group, or a fused ring group, wherein the monocyclic group is

aryl or heteroaryl, and the fused ring group is formed by fusing heteroaryl to a group selected from aryl and heteroaryl; and $R_1$ represents a single bond, O, N, S, or alkylene; said A is optionally substituted with one or more substituents selected from X, wherein X is selected from halogen, halogen-substituted or unsubstituted alkyl, -NH-S(O)$_2$-alkenyl, or -$C_1$-$C_6$ heteroalkyl-heterocycloalkyl, the heterocycloalkyl being optionally substituted with one or more halogen-substituted alkyl or -C(O)-halogen-substituted alkyl; and one or more X groups can be the same or different.

In formula (II), m1 and m2 are each independently selected from an integer from 0 to 10 and can be, for example, 0, 1, 2, 3, 4, 5, or 6.
Preferably, A is optionally substituted with 1, 2, or 3 substituents selected from X.
Preferably, m1 is 0 or 1, and m2 is 0 or 1.
In one embodiment, A is selected from $C_2$-$C_6$ alkenyl, a monocyclic group, or a fused ring group, wherein the monocyclic group is 6- to 12-membered aryl or 5- to 12-membered heteroaryl, the 5- to 12-membered heteroaryl containing 1-3 nitrogen atoms; and the fused ring group is a ring formed by fusing 5- to 12-membered heteroaryl to a group selected from 6- to 12-membered aryl and 5- to 12-membered heteroaryl, the 5- to 12-membered heteroaryl containing 1-3 nitrogen atoms as ring atoms;
preferably, A is selected from a monocyclic group or a fused ring group, wherein the monocyclic group is a 6-membered aromatic ring, a 5-membered heteroaromatic ring, or a 6-membered heteroaromatic ring, and the fused ring group is formed by fusing 6-membered heteroaryl to 6-membered aryl, the 5-membered heteroaryl or 6-membered heteroaryl containing 1-3 nitrogen atoms;
Preferably, A is selected from vinyl,

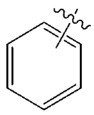, 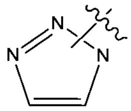, 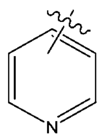, 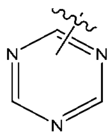

or

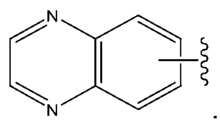.

Preferably, A is selected from vinyl,

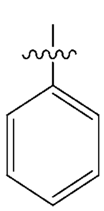, 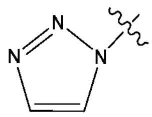, 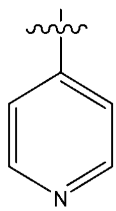, 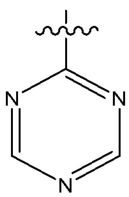

or

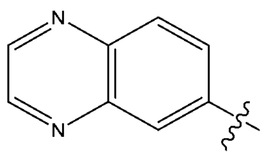.

In one embodiment, X represents halogen, halogen-substituted $C_1$-$C_6$ alkyl, -NH-S(O)$_2$-$C_2$-$C_6$ alkenyl, or -$C_1$-$C_6$ heteroalkyl-3- to 8-membered heterocycloalkyl, the 3- to 8-membered heterocycloalkyl being optionally substituted with one or more halogen-substituted $C_1$-$C_6$ alkyl or -C(O)-halogen-substituted $C_1$-$C_6$ alkyl.
In one embodiment, M is selected from:

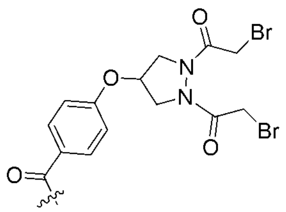, 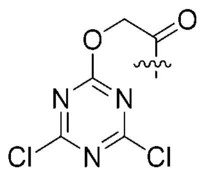, 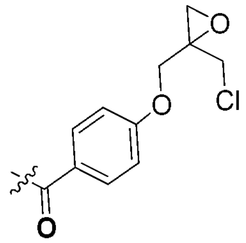, 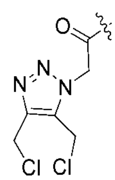, 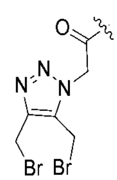,

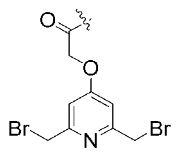, 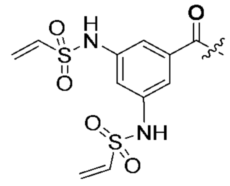, 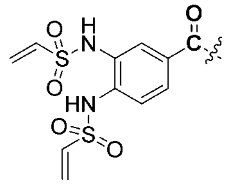, 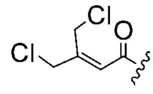, 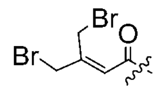;

and

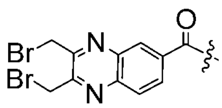.

In one embodiment, L is a divalent structure consisting of one or more selected from: Val-Cit, Val-Ala, Val-Lys, Val-Lys(Ac), Phe-Lys, Phe-Lys(Ac), Gly -Lys, Gly-Phe, Gly-Leu, Gly-Gly-Lys, Gly-Val-Ala, Gly-Val-Cit, Gly-Phe-Gly, Val-Ala-Gly, D-Val-Leu-Lys, Gly-Gly-Arg, Ala-Ala-Asn, Ala-Ala-Ala, Val-Lys-Ala, Gly-Gly-Gly, Gly-Leu-Gly, Gly-Gly-Phe-Gly, Gly-Gly-Leu-Gly, Gly-Val-Ala-Gly, Gly-Val-Cit-Gly, and Gly-Gly-Gly-Gly-Gly;

preferably, L is selected from Val-Cit, Val-Ala, Gly-Gly-Lys, Gly-Phe-Gly, Gly-Lys, Gly-Phe, Gly-Leu, Val-Ala-Gly, Gly-Gly-Phe-Gly, Gly-Val-Ala-Gly, Ala-Ala-Asn, and Gly-Leu-Gly; and
preferably, L is selected from Gly-Lys and Gly-Val-Ala.

In one embodiment, D is at least one agent selected from an anti-tubulin agent, a DNA intercalator, a DNA topoisomerase inhibitor, a DNA synthesis inhibitor, an RNA polymerase inhibitor, and a spliceosome inhibitor.

Preferably, the tubulin inhibitor is an auristatin compound or a maytansinoid compound. Auristatin interferes with microtubule dynamics, GTP hydrolysis, and nuclear and cellular division, and has anticancer and antifungal activities. For example, auristatin E can react with p-acetylbenzoic acid or benzoylpentanoic acid to produce AEB and AEVB, respectively. Other typical auristatin derivatives include AFP, MMAF (monomethyl auristatin F), and MMAE (monomethyl auristatin E).

Preferably, the DNA intercalator is pyrrolobenzodiazepine (PBD).

Preferably, non-limiting examples of the DNA topoisomerase inhibitor include topoisomerase I inhibitors or topoisomerase II inhibitors. Preferably, non-limiting examples of the topoisomerase I inhibitors include camptothecin, hydroxycamptothecin, 9-aminocamptothecin, 10,11-methylenedioxycamptothecin, SN-38, irinotecan, topotecan, belotecan, exatecan or rubitecan, and Genz-644282. Preferably, non-limiting examples of the topoisomerase II inhibitors include adriamycin, doxorubicin, PNU-159682, duocarmycin, daunomycin, mitoxantrone, podophyllotoxin, or etoposide. Preferably, non-limiting examples of the DNA synthesis inhibitor include gemcitabine and other pyrimidine nucleotide analogues. Preferably, non-limiting examples of the RNA polymerase inhibitor include α-amanitin. Preferably, non-limiting examples of the spliceosome inhibitor include thailanstatin series toxins and pharmaceutically acceptable salts, esters, and analogues thereof.

In one embodiment, said L is directly linked to D, or a spacer unit E is linked between said L and said D, the spacer unit E being selected from one of p-aminobenzyl carbamate, bis(p-aminobenzyl) carbonate, hemiaminal (e.g., aminomethyl ether (also known as alkoxyamine)), and methylenediamine.

In one embodiment, said M is directly linked to L, or a spacer unit F is linked between said M and said L, the spacer unit F being $-NH-(CH_2CH_2-O)_q-CH_2CH_2-C(=O)-$ or a single bond, wherein q represents an integer from 1 to 6 and can be, for example, 0, 1, 2, 3, 4, 5, or 6.

In one embodiment, the compound is selected from the group consisting of:

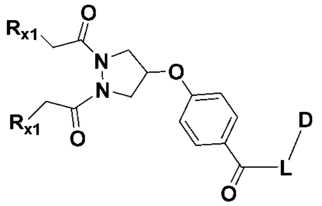, 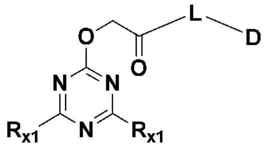, 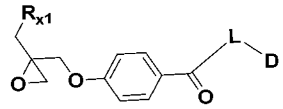,

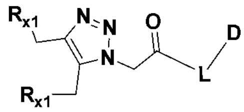, 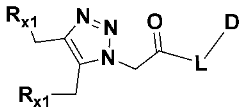, 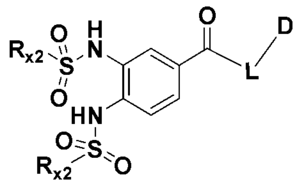, 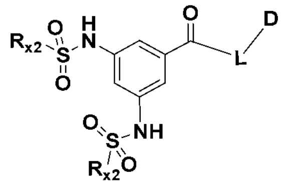,

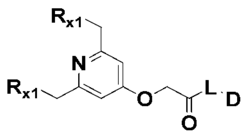, 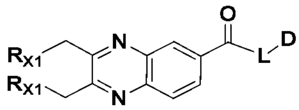, and 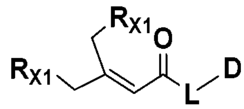,

wherein, L and D are as previously defined;
$R_{X1}$ and $R_{X2}$ are each independently selected from halogen or $C_2$-$C_6$ alkenyl;
preferably, $R_{X1}$ is selected from bromine or chlorine, and $R_{X2}$ is vinyl.

In one embodiment, the compound is selected from the group consisting of:

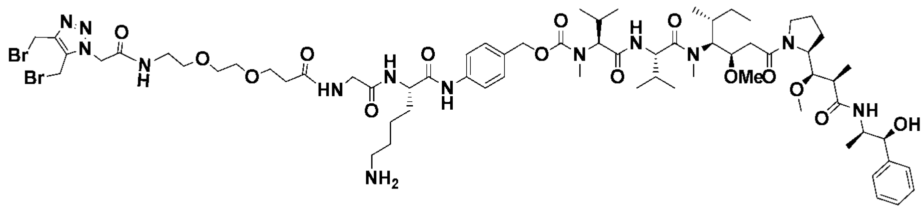

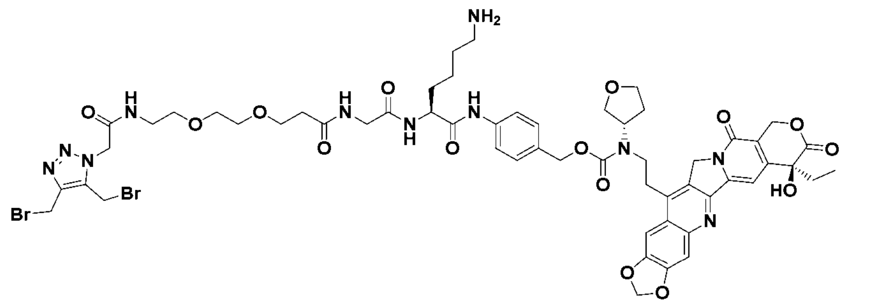

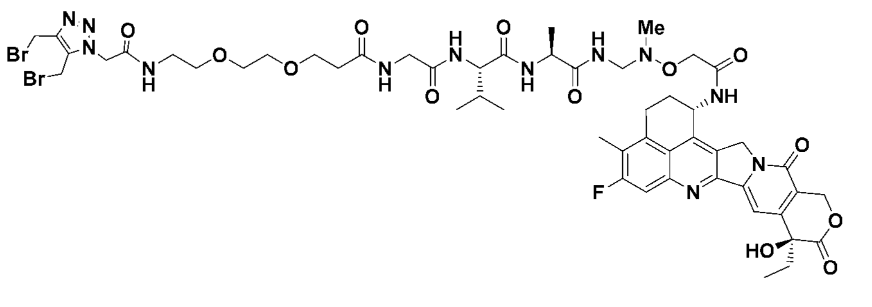

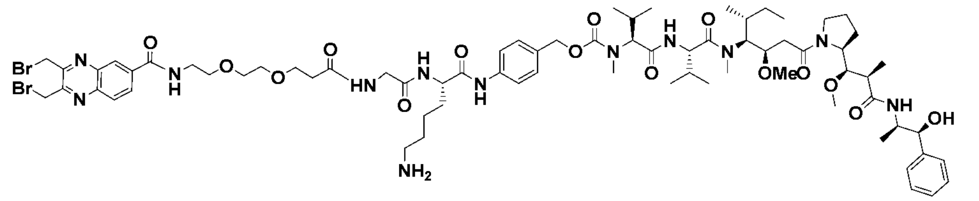

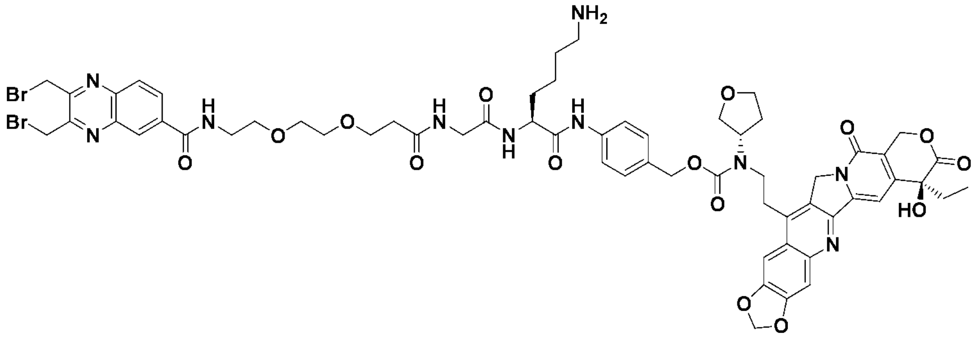

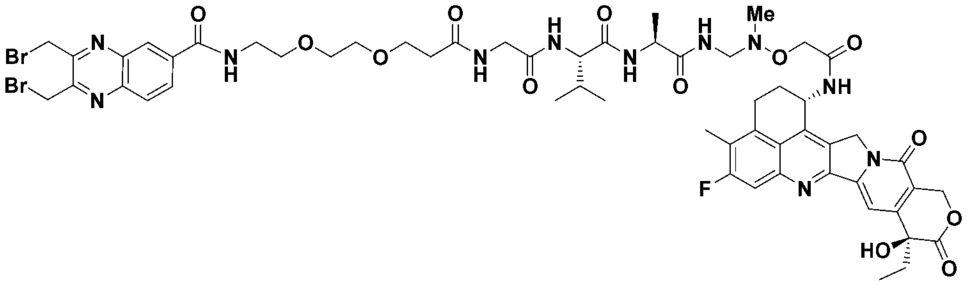

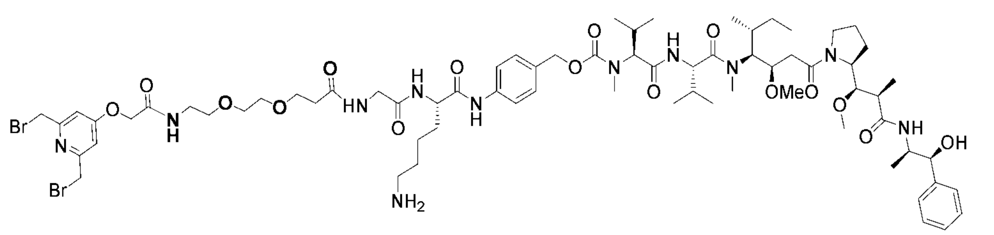

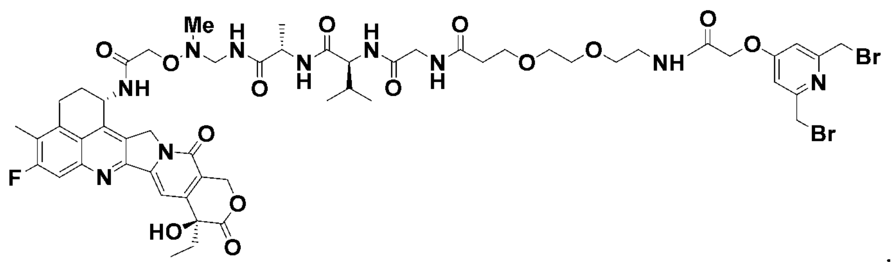

.

In a second aspect, the present application provides a drug-antibody conjugate represented by formula (III) or a pharmaceutically acceptable salt thereof:

Ab-M-L-D (III)

wherein, M, L, and D are each as defined for the compound of formula (I); and Ab is an antibody or an antigen-binding fragment thereof;

In one embodiment, the drug-antibody conjugate is further linked to an additional drug having cytotoxicity;

preferably, the drug-antibody conjugate has a structure represented by formula (IV):

D'-L'-Ab-M-L-D (IV)

wherein, said L' is a linker between the antibody and D', and is the same as or different from L;
D' is a cytotoxic drug moiety that is the same as or different from D.

Preferably, D' is exatecan or monomethyl auristatin E. Preferably, the antibody is a monoclonal antibody. Preferably, the monoclonal antibody is a human antibody or a humanized antibody. Preferably, the monoclonal antibody is IgG. Preferably, the monoclonal antibody is IgG1, IgG2, IgG3, or IgG4. Preferably, the monoclonal antibody is an IgG1 or IgG4 monoclonal antibody. Preferably, the IgG1 monoclonal antibody is Herceptin (trastuzumab (U.S. Patent No. 5821337)).
In one embodiment, Ab is an antibody against a tumor-associated antigen.
Preferably, Ab is selected from an anti-Her2 antibody, an anti-Trop2 antibody, an anti-B7H3 antibody, an anti-5T4 antibody, an anti-Nectin-4 antibody, an anti-CD20 antibody, and an anti-ROR1 antibody.
Preferably, Ab is pertuzumab HS627.
In one embodiment, D or D' is selected from:

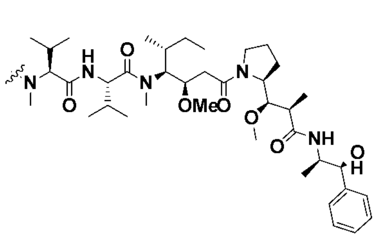
,
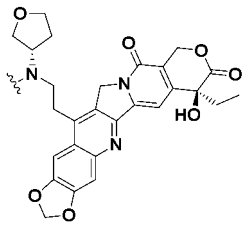
and
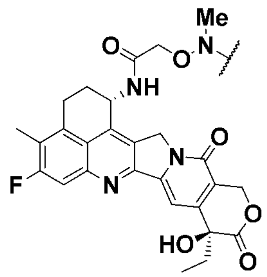
.

In one embodiment, the present application provides the following drug-antibody conjugates:

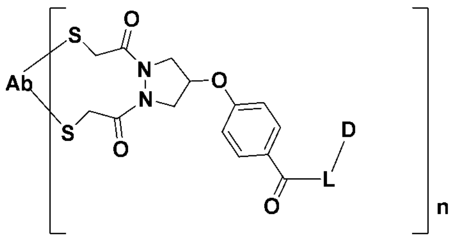
,
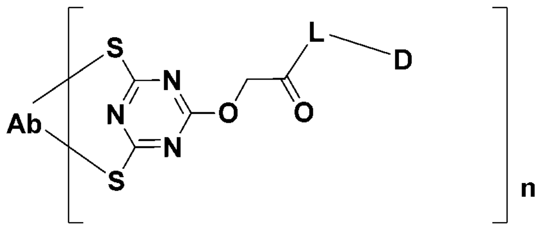
,

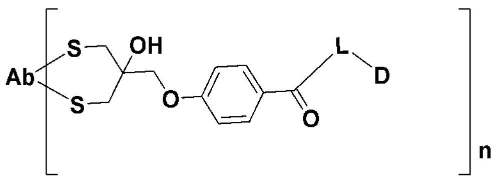
,
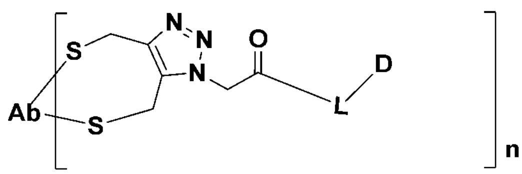
,

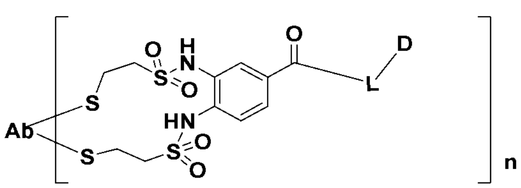
,
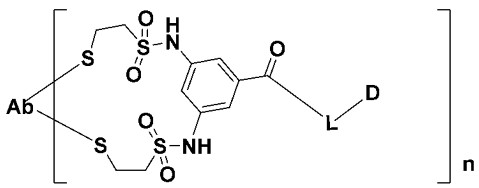
,

and

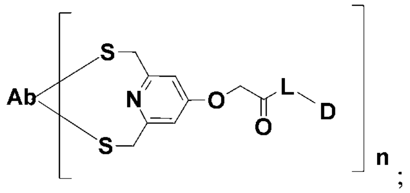
;

wherein, L and D are each as defined for the compound of formula (I), and Ab is an antibody or an antigen-binding fragment thereof.

n is an integer selected from 1 to 12, preferably an integer selected from 2 to 4; preferably, the antibody is a monoclonal antibody or a polyclonal antibody.

In a third aspect, the present disclosure provides the following bifunctional linker compounds:

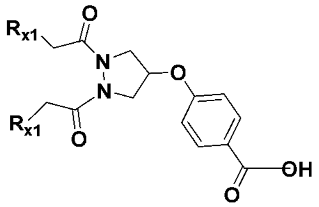
,
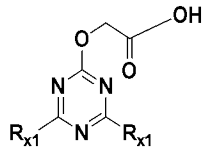
,
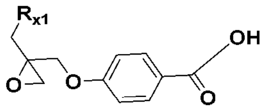
,
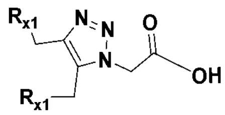
,

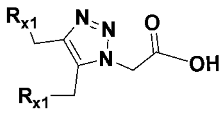
,
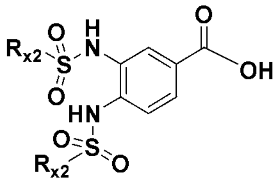
,
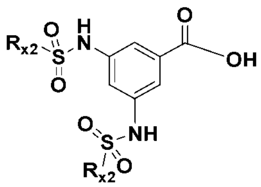
,
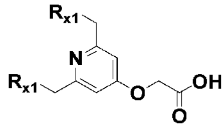
,

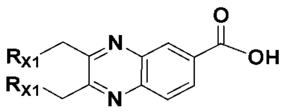
, or
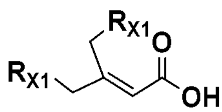
;

$R_{X1}$ and $R_{X2}$ are each independently selected from halogen or $C_2$-$C_6$ alkenyl;
preferably, $R_{X1}$ is selected from bromine or chlorine, and $R_{X2}$ is vinyl.

The present application provides a method for preparing the drug-antibody conjugate represented by formula (III) or the pharmaceutically acceptable salt thereof, as described above, comprising the following step:
linking a compound represented by formula (I) or a pharmaceutically acceptable salt, stereoisomer, or prodrug thereof to an antibody or an antigen-binding fragment thereof.

The present application provides a pharmaceutical composition, comprising the compound or the pharmaceutically acceptable salt, stereoisomer, or prodrug thereof, as described above, or the antibody-drug conjugate, as described above, and a pharmaceutically acceptable excipient.

The present application provides use of the compound or the pharmaceutically acceptable salt, stereoisomer, or prodrug thereof, as described above, the antibody-drug conjugate as described above, and the pharmaceutical composition as described above in the manufacture of a medicament for treating cancer.

The present application provides a method for treating cancer, comprising the step of administering to a patient in need thereof the compound or the pharmaceutically acceptable salt, stereoisomer, or prodrug thereof, as described above, the antibody-drug conjugate as described above, or the pharmaceutical composition as described above.

In some embodiments, the cancer comprises gastric cancer, esophageal cancer, cardia cancer, breast cancer, ovarian cancer, colon cancer, rectal cancer, primary liver cancer, pancreatic cancer, acute and chronic granulocytic leukemia, chorionic epithelial cancer, lung cancer, bladder cancer, intestinal cancer, or small cell lung cancer; preferably, the cancer is esophageal cancer, pancreatic cancer, or gastric cancer, and further preferably, the cancer is pancreatic cancer.

Examples

Abbreviation:

Fmoc: 9-fluorenylmethoxycarbonyl; Val: valine, the structural formula of which is

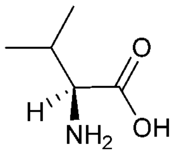;

Ala: alanine, the structural formula of which is

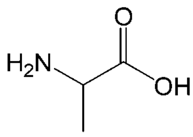;

Cit: citrulline, the structural formula of which is

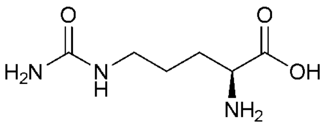;

Lys: lysine, the structural formula of which is

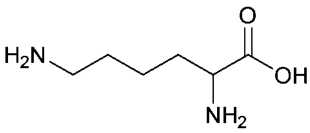;

Gly: glycine, the structural formula of which is

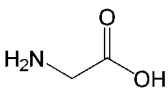;

Phe: phenylalanine, the structural formula of which is

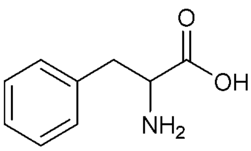;

PAB:

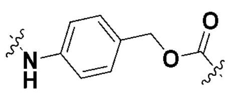;

m:

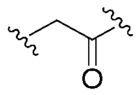
;

MC: 6-maleimidocaproyl; VC: Val-Cit (valine-citrulline); THF: tetrahydrofuran; DCM: dichloromethane; PE: petroleum ether; EA: ethyl acetate; DMF: N,N-dimethylformamide; DIAD: diisopropyl azodicarboxylate; TFA: trifluoroacetic acid; LAH: lithium aluminum hydride; EEDQ: 2-ethoxy-1-ethoxycarbonyl-1,2-dihydroquinoline; DBU: 1,8-diazabicyclo[5.4.0] undec-7-ene; TEA: triethylamine; MMAE: monomethyl auristatin E; HOBT: 1-hydroxybenzotriazole; HATU: 2-(7-azabenzotriazole)-N,N,N',N'-tetramethyluronium hexafluorophosphate; DIPEA: N,N-diisopropylethylamine; DMA: N,N-dimethylacetamide; mCPBA: m-chloroperoxybenzoic acid; DIC: N,N-diisopropylcarbodiimide; TFA: trifluoroacetic acid; NMP: N-methylpyrrolidone; DMA: N,N-dimethylaniline; and NAC: N-acetylcysteine.

During the preparation of ADCs in the examples, pertuzumab (HS627, PERTUZUMAB, purchased from BrightGene, batch No. 0362721003) is used without limitation.

**Example 1: Synthesis of 4-((1,2-bis(2-bromoacetyl)pyrazolidin-4-yl)oxy)benzoic acid (CL-01)**

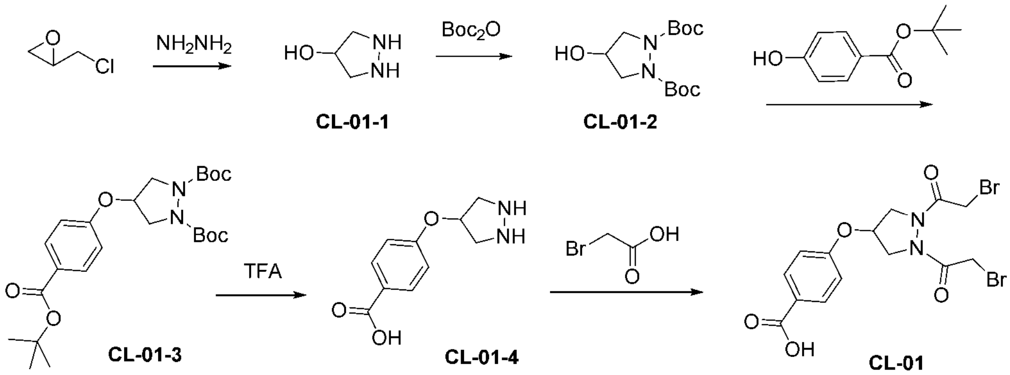

CL-01-1 CL-01-2 CL-01-3 CL-01-4 CL-01

Under ice bath cooling condition, chloromethyloxirane (12 g, 129.70 mmol, 10.14 mL) was slowly dropwise added to a solution containing 85% hydrazine hydrate ($N_2H_4.H_2O$) (8.12 g, 162.12 mmol, 7.90 mL) in water (10 mL). After the addition was complete, the ice bath was removed, and stirring was continued for 2 hours. Acetone (30 mL) was added, and stirring was continued for 2 hours. The mixture was extracted with ethyl acetate (3×50 mL) to remove a nonpolar material. An aqueous phase was separated. Under ice bath cooling condition, potassium carbonate (22.41g, 162.12 mmol), THF (30 mL), and $Boc_2O$ anhydride (35.38 g, 162.12 mmol, 37.21 mL) were added to the aqueous phase. Stirring was continued for 2 hours. The mixture was extracted with ethyl acetate (100 mL). The organic phase was washed separately with 1N HCl (50 mL), a saturated $NaHCO_3$ aqueous solution (100 mL), and a saturated aqueous sodium chloride solution, dried over anhydrous sodium sulfate, filtered, and concentrated to give bis-Boc-substituted intermediate **CL-01-2** (30 g, yield 83%). To a reaction flask were added **CL-01-2** (6 g, 13.82 mmol), THF (40 mL), tert-butyl 4-hydroxybenzoate (2.67 g, 13.76 mmol), and triphenylphosphine (1.69 g, 24.87 mmol). Under stirring at 0°C, DIAD (5.04 g, 24.87 mmol, 4.89 mL) was dropwise added to the reaction flask. After the addition was complete, the mixture was stirred overnight at room temperature. Water (50 mL) was added. The mixture was extracted with ethyl acetate, dried over anhydrous sodium sulfate, filtered, concentrated, and purified by silica gel column chromatography (PE/EA = 4:1) to give intermediate **CL-01-3** (8 g, yield 81.93%, HPLC 99%); $^1$H NMR(500 MHz, $CDCl_3$)δ 7.88 - 7.84(m, 2H), 6.76-6.72(m, 2H), 5.01(t, J=4.7 Hz, 1H),4.23(d, J=12.9 Hz, 1H), 4.03-3.98(m, 1H), 3.51(d, J=12.4 Hz, 1H), 3.22(d, J=12.4 Hz, 1H), 1.50(s, 9H), 1.42(s, 9H), 1.38(s, 9H); $^{13}$C NMR(126 MHz, $CDCl_3$)δ 165.32, 159.82, 131.54, 125.45, 114.73, 81.74, 81.43, 80.76,77.32, 77.06, 76.81, 28.25, 28.22, 28.05; LCMS: m/z 487.30 $[M+H]^+$ (theoretical value 465.56).

To a reaction flask were added **CL-01-3** (8 g, 17.22 mmol), DCM (50 mL), and TFA (15 mL), which were reacted under stirring for 5 hours at room temperature. The reaction mixture was concentrated. Tert-butyl methyl ether was added and stirred. **CL-01-4** (3 g, yield 83%, HPLC 99%) precipitated out as a white solid product; $^1$H NMR(500 MHz, DMSO-$d_6$)δ 7.93-7.91(d, J=10 Hz, 2H), 7.07-7.05(d, J=10 Hz, 2H), 5.36(q, J=3.5, 1.9 Hz, 1H), 3.52(d, J=5.2 Hz, 1H), 3.49(d, J=5.1 Hz, 1H), 3.37(d, J=1.6 Hz, 1H), 3.36-3.33(d, J=1.6 Hz, 1H); $^{13}$C NMR(126 MHz, DMSO-$d_6$)δ 167.32, 160.45, 131.92, 124.31, 115.66, 77.38, 68.73, 52.6; LCMS: m/z 209.61 $[M+H]^+$ (theoretical value 209.22).

To a reaction flask was added **CL-01-4** (1.5 g, 7.2 mmol), followed by THF (50 mL) and solid potassium carbonate (2.99 g, 21.61 mmol). Under stirring at 0°C, a solution of bromoacetyl bromide (3.64 g, 18.01 mmol, 1.56 mL) in THF (10 mL) was dropwise added. The mixture was reacted under stirring at room temperature for 2 hours. The reaction mixture

was poured into ice water under stirring. The pH was adjusted to an acidic value with hydrochloric acid. After filtration, **CL-01** (2 g, yield 61%, HPLC 98%) precipitated out as a white solid product; $^1$H NMR(500 MHz, DMSO-$d_6$)δ 7.91(s, 1H), 7.89(s, 1H), 7.02(s, 2H), 5.46-5.38(m, 1H), 4.52-4.45(m, 2H), 4.17-4.14(m, 1H), 3.51-3.44(m, 1H); LCMS: m/z 451.08 $[M+H]^+$ (theoretical value 451.08).

**Example 2: Synthesis of 2-((4,6-dichloro-1,3,5-triazin-2-yl)oxy)acetic acid (CL-02)**

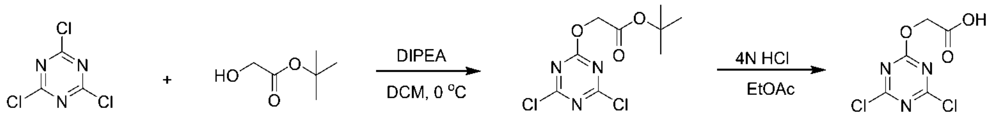


**CL-02**

At 0°C, to a reaction flask were added DIPEA (3.07 g, 23.73 mmol, 4.13 mL), tert-butyl 2-hydroxyacetate (2.87 g, 21.58 mmol), DCM (50 mL), and a solution of 2,4,6-trichloro-1,3,5-triazine (4 g, 21.57 mmol) in DCM (150 mL). The reaction liquid was stirred for 1 h at 0°C, poured into ice water, and separated to give an organic phase. The organic phase was washed with a saturated sodium chloride solution. The solvent was concentrated. Purification by silica gel column chromatography (EA/PE = 2-8% EA) afforded tert-butyl 2-((4,6-dichloro-1,3,5-triazin-2-yl)oxy)acetate as a viscous product (4.9 g, 17.43 mmol, yield 80.80%, HPLC 99.9%), which was turned into a solid upon standing; $^1$H NMR(600 MHz, $CDCl_3$)δ 4.81(s, 2H), 1.41(s, 9H); $^{13}$C NMR(151 MHz, $CDCl_3$)δ 172.59, 170.98, 165.26, 83.55, 77.25, 77.04, 76.82, 65.40, 27.98.

Tert-butyl 2-((4,6-dichloro-1,3,5-triazin-2-yl)oxy)acetate (6.5 g, 23.12 mmol) was dissolved in a solution of 4N HCl in ethyl acetate and stirred overnight at room temperature. The solvent was concentrated. Tert-butyl methyl ether was added. After filtration, a solid precipitated out, which was dried to give the product 2-((4,6-dichloro-1,3,5-triazin-2-yl)oxy) acetic acid **CL-02** (3.5 g, 15.24 mmol, yield 65.93%, HPLC 98%); LC/MS m/z 223.38 $[M+H]^+$ (theoretical value 222.96).

**Example 3:**

**Synthesis of 4-(2-chloromethyloxiran-2-ylmethoxy)benzoic acid (CL-03)**

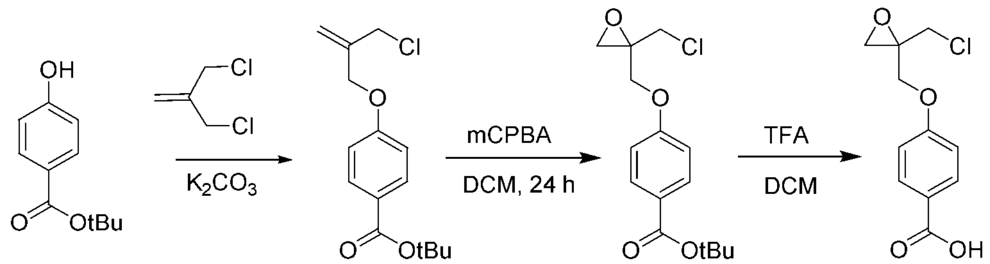


**CL-03-1** **CL-03-2** **CL-03**

To a reaction flask were added tert-butyl 4-hydroxybenzoate (0.78 g, 4.00 mmol), 3-chloro-2-chloromethyl-1-propene (998.77 mg, 7.93 mmol), $K_2CO_3$ (1.66 g, 12 mmol), and acetonitrile (10 mL). The reaction liquid was heated to 70°C under stirring and reacted for 5 h. After filtration, the filtrate was concentrated to dryness and purified by silica gel column chromatography (EA/PE = 3-10 % EA) to give tert-butyl 4-((2-chloromethyl)allyloxy)benzoate CL-03-1 (0.6 g, 2.09 mmol, yield 52.39%, HPLC 99%); $^1$H NMR(600 MHz, $CDCl_3$)δ 7.90-7.83(m, 1H), 6.90-6.81(m, 1H), 5.33(s, 1H), 5.29(s, 1H), 4.60(s, 1H), 4.11(s, 1H), 1.50(s, 5); $^{13}$C NMR(151 MHz, $CDCl_3$)δ 165.50, 161.73, 140.30, 131.42, 124.99, 117.81, 114.17, 80.61, 77.27, 77.06, 76.85, 67.95, 44.97, 34.94, 31.52, 30.15, 29.71, 28.26.

To a reaction flask were added mCPBA (1.09 g, 6.31 mmol), CL-03-1 (0.6 g, 2.11 mmol), and DCM (10 mL), which were reacted under stirring at room temperature for 36 h. A saturated aqueous sodium sulfite solution (10 mL) was added. An organic phase was separated and purified by silica gel column chromatography (EA/PE = 5-12% EA) to give tert-butyl 4-((2-chloromethyl)oxiran-2-ylmethoxy)benzoate CL-03-2 (0.42 g, 1.40 mmol, yield 66.00%, HPLC 99.6%) as a white solid product; $^1$HNMR(600MHz,$CDCl_3$)δ8.01-7.76(m,2H),6.96-6.72(m,2H),4.22(dd,J=10.7,1.1 Hz, 1H), 4.11(d, J=10.5

Hz, 1H), 3.79(dt, J=11.8, 1.1 Hz, 1H), 3.58(d, J=11.7 Hz, 1H), 2.93(dd, J=4.4, 1.0 Hz, 1H), 2.87(dd, J=4.3, 1.2 Hz, 1H), 1.52(d, J=13.1 Hz, 8H); $^{13}$C NMR(151 MHz, $CDCl_3$)δ 165.41, 161.55, 131.46, 125.35, 114.06, 80.69, 77.28, 77.07, 76.85, 67.47, 57.46, 51.28, 44.81, 29.70, 28.24 LCMS: m/z 298.86 $[M+H]^+$ (theoretical value 298.10).

To a reaction flask were added TFA (1.5 mL), CL-03-2 (0.2 g, 667.18 μmol), and DCM (6 mL), which were stirred at room temperature for 2 h. The solvent was removed under reduced pressure to give 4-((2-chloromethyl)oxiran-2-ylmethoxy)benzoic acid **CL-03** (0.16 g, 430.64 μmol, yield 64.55%, HPLC 96%); $^1$H NMR(600 MHz, DMSO-$d_6$)δ 7.86-7.79(m, 2H), 6.99(d, J=8.6 Hz, 2H), 4.23(d, J=11.0 Hz, 1H), 4.17(d, J=11.0 Hz, 1H), 3.87-3.79(m, 2H), 2.96(d, J=4.9 Hz, 1H), 2.92(d, J=4.9 Hz, 1H); $^{13}$C NMR(151 MHz, DMSO)δ 167.47, 162.07, 131.84, 124.08, 114.85, 67.34, 57.53, 50.50, 45.76. LCMS: m/z $[M+H]^+$ 243.45 (theoretical value 242.03).

**Example 4: Synthesis of 2-(4,5-dichloromethyl-1-H-1,2,3-triazol-1-yl)acetic acid (CL-04)**

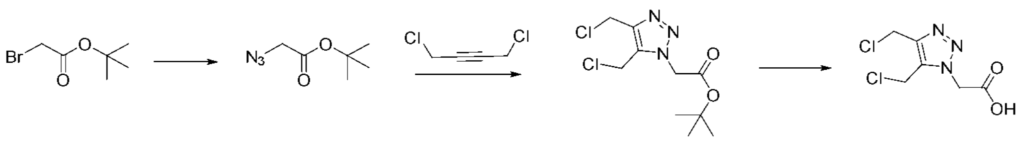

CL-04-1 CL-04-2 CL-04

Tert-butyl bromoacetate (2 g, 10.20 mmol, 1.50 mL) was dropwise added to a reaction flask containing $NaN_3$ (663.06 mg, 10.20 mmol) and DMF (10 mL). The mixture was heated to 70°C and reacted overnight under stirring. The reaction mixture was cooled. Water (25 mL) was added. The mixture was extracted with ethyl acetate, dried over anhydrous sodium sulfate, filtered, and concentrated to give tert-butyl azidoacetate as a light yellow oil (1.2 g, yield 75%). Tert-butyl azidoacetate **CL-04-1** (500 mg, 3.16 mmol), toluene (10 mL), and 1,4-dichloro-2-butyne (777.48 mg, 6.27 mmol) were added to a reaction flask and heated overnight under stirring. The solvent was concentrated under reduced pressure. Tert-butyl methyl ether was added. After stirring, tert-butyl 2-(4,5-dichloromethyl-1-H-1,2,3-triazol-1-yl)acetate **CL-04-2** as a grey-white solid product (500 mg, yield 56%) precipitated out; $^1$H NMR(600 MHz,$CDCl_3$)δ 5.16(s, 2H), 4.75(s, 2H), 4.73(s, 2H), 1.50(s, 9H); LCMS: m/z 280.41 $[M+H]^+$ (theoretical value 280.15). Tert-butyl 2-(4,5-dichloromethyl-1-H-1,2,3-triazol-1-yl)acetate (200 mg, 0.7 mmol), a 4.0 M ethyl acetate solution in HCl (25.94 mg, 0.7 mmol, 4 mL), and ethyl acetate (5 mL) were added to a reaction flask and reacted under stirring at room temperature for 6 hours. The solvent was concentrated under reduced pressure. Tert-butyl methyl ether was added. After stirring and filtration, 2-(4,5-dichloromethyl-1-H-1,2,3-triazol-1-yl)acetic acid **CL-04** as a grey solid product (120 mg, yield 75%, HPLC 98%) precipitated out; $^1$H NMR(600 MHz, DMSO-$d_6$)δ 5.34(s, 2H), 5.06(s, 2H), 4.94(s, 2H); LCMS: m/z 224.45 $[M+H]^+$ (theoretical value 224.04).

**Example 5: Synthesis of 2-(4,5-dibromomethyl-1-H-1,2,3-triazol-1-yl)acetic acid (CL-05)**

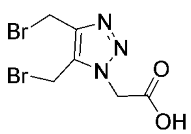

2-(4,5-Dibromomethyl-1-H-1,2,3-triazol-1-yl)acetic acid could be synthesized following the method for the synthesis of 2-(4,5-dichloromethyl-1-H-1,2,3-triazol-1-yl)acetic acid (CL-04). Tert-butyl 2-(4,5-dibromomethyl-1-H-1,2,3-triazol-1-yl)acetate as a grey solid product (16 g, yield 58%, HPLC 98%) was obtained; $^1$H NMR(600 MHz, $CDCl_3$)δ 5.14(s, 2H), 4.60(s, 2H), 4.53(s, 2H), 1.50(s, 9H); LCMS: m/z 370.19 $[M+H]^+$ (theoretical value 370.06). Next, the grey solid product was treated with trifluoroacetic acid to give 2-(4,5-dibromomethyl-1-H-1,2,3-triazol-1-yl)acetic acid **CL-05** as a white solid product (10 g, yield 72%, HPLC 98%); $^1$H NMR(600 MHz, DMSO-$d_6$)δ 5.34(s, 2H), 5.06(s, 2H), 4.94(s, 2H); LCMS: m/z 314.22 $[M+H]^+$ (theoretical value 313.95).

**Example 6: Synthesis of 2-((2,6-dibromomethyl)pyridin-4-yloxy)acetic acid (CL-06)**

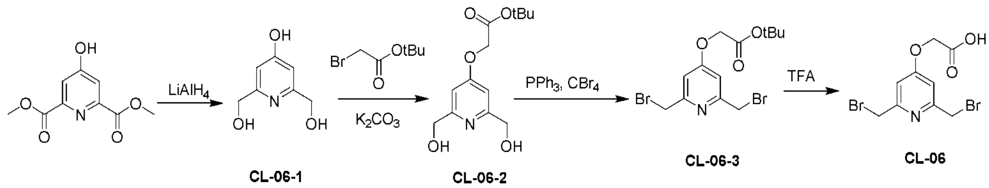

CL-06-1 CL-06-2 CL-06-3 CL-06

4-Hydroxypyridine-2,6-dicarboxylic acid (5 g, 27.16 mmol) was dissolved in methanol (50 mL). Concentrated sulfuric acid (0.6 mL, 27.16 mmol) was added. The reactants were heated to reflux under stirring for 4 h, cooled to room temperature, poured into ice water, and adjusted to pH 5. After filtration, a solid product precipitated out, which was recrystallized from ethyl acetate/petroleum ether to give dimethyl 4-hydroxypyridine-2,6-dicarboxylate (5.76 g, yield 70%); $^1$H NMR(600 MHz, DMSO-$d_6$)δ 11.60(s, 1H), 7.59(s, 2H), 3.90(s, 6H); LCMS: m/z 212.5 $[M+H]^+$ (theoretical value 211.05).

Dimethyl 4-hydroxypyridine-2,6-dicarboxylate (1 g, 4.71 mmol) was dissolved in THF (8 mL). A solution of LAH (447.19 mg, 11.78 mmol) in THF (15 mL) was slowly dropwise added under ice bath cooling condition. After the addition was complete, the reaction liquid was reacted under stirring at 50°C for 18 h and cooled to room temperature. A saturated aqueous sodium sulfate solution was added to quench the reaction. The reaction mixture was filtered to remove a solid material. The filtrate was concentrated under reduced pressure to give **CL-06-1** as a solid product (0.45 g, yield 72 %); $^1$H NMR(600 MHz, DMSO-$d_6$)δ 6.16(s, 2H), 4.24(s, 4H); LCMS: m/z 156.65 $[M+H]^+$ (theoretical value 155.06).

To a reaction flask were added tert-butyl 2-bromoacetate (2.62 g, 13.38 mmol, 1.97 mL), **CL-06-1** (2.1 g, 13.45 mmol), $K_2CO_3$ (2.7 g, 20 mmol), and acetonitrile (25 mL). The reaction liquid was heated, stirred to reflux for 2 h, concentrated, extracted with DCM, and purified by silica gel column chromatography (MeOH/DCM (3-10% MeOH) to give **CL-06-2** as a white solid product (1 g, yield 24 %); $^1$H NMR(500 MHz, DMSO-$d_6$)δ 6.81(s, 2H), 5.36(t, J=5.8 Hz, 2H), 4.74(s, 2H), 4.46(d, J=5.7 Hz, 4H), 1.43(s, 9H); LCMS: m/z 270.86 $[M+H]^+$ (theoretical value 269.13).

To a reaction flask were added triphenylphosphine (2.18 g, 8.32 mmol), carbon tetrabromide (2.76 g, 8.32 mmol), **CL-06-2** (0.9 g, 3.33 mmol), and DCM (30 mL), which were stirred at room temperature for 10 h, concentrated under reduced pressure, and purified by silica gel column chromatography (EtOAc/PE = 0-30% EA) to give **CL-06-3** as a white solid product (1.06 g, yield 76 %); $^1$H NMR(500 MHz, $CDCl_3$)δ 6.88(s, 2H), 4.59(s, 2H), 4.47(s, 4H), 1.50(s, 9H); LCMS: m/z 394.23 $[M+H]^+$ (theoretical value 392.96).

To a reaction flask were added TFA (2 mL), **CL-06-3** (1 g, 2.52 mmol), and DCM (8 mL). The reaction liquid was stirred at room temperature for 15 h and concentrated under reduced pressure. Tert-butyl methyl ether was added to give 2-((2,6-dibromomethyl)pyridin-4-yloxy)acetic acid **CL-06** as a solid product (620 mg, yield 53 %); LCMS: m/z 338.27 $[M+H]^+$ (theoretical value 336.89).

### Example 7: Synthesis of 3,4-bis(vinylsulfonamido)benzoic acid (CL-07)

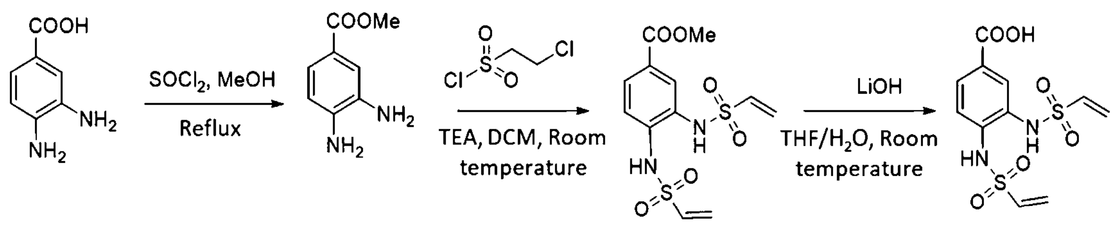

CL-07-1 CL-07-2 CL-07

To a reaction flask were added 3,4-diaminobenzoic acid (2 g, 13.14 mmol) and methanol (80 mL), which were stirred under ice bath cooling condition. $SOCl_2$ (3.13 g, 26.29 mmol, 1.92 mL) was dropwise added. After the addition was complete, the mixture was reacted under stirring at room temperature for 5 h and concentrated under reduced pressure to give methyl 3,4-diaminobenzoate **CL-07-1** (2 g, 12.04 mmol, yield 91.56%), which was directly used in the next reaction.

To a reaction flask were added **CL-07-1** (300 mg, 1.81 mmol) and DCM (8 mL), which were stirred under ice bath condition. TEA (913.39 mg, 9.03 mmol, 1.26 mL) was added, followed by slow dropwise addition of 2-chloroethanesulfonyl chloride (588.61 mg, 3.61 mmol). After the addition was complete, stirring was continued at room temperature for 2 h. The solvent was removed under reduced pressure. THF and water (3 mL, 1:1) were added, followed by $LiOH.H_2O$ (16.96 mg, 404.18 μmol). The mixture was stirred at room temperature for 1 h and adjusted with 2N HCl to pH < 3. After filtration, **CL-07** as a white solid product (22 mg, 62.89 μmol, yield 31.12%, HPLC 95%) precipitated out; $^1$H NMR(500 MHz, DMSO-$d_6$) δ 13.04(s, 1H), 9.41(d, J=42.7 Hz, 2H), 7.88(d, J=2.0 Hz, 1H), 7.75(dd, J=8.5, 2.0 Hz, 1H), 7.47(d, J=8.5 Hz, 1H),

6.92-6.89(m, 1H), 6.88-6.86(m, 1H), 6.21-6.11(m, 2H), 6.10-6.00(m, 2H); $^{13}C$ NMR(126 MHz, DMSO) δ 166.75, 136.30, 135.45, 129.41, 128.90, 128.12, 128.07, 127.52, 126.76, 121.67.

**Example 8: Synthesis of 3,5-bis(vinylsulfonamido)benzoic acid (CL-08)**

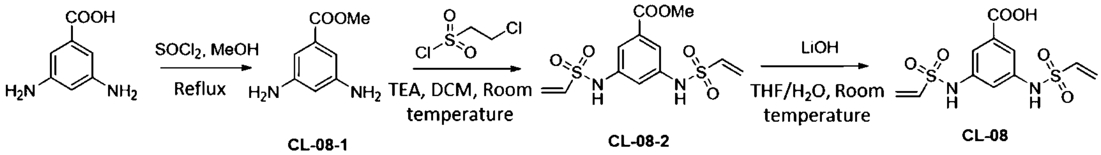


CL-08-1 CL-08-2 CL-08

Following the method described above for the preparation of **CL-07, CL-08** (20 mg, overall yield 26.80% over three steps, HPLC 97%) was successfully obtained; $^{1}H$ NMR(600 MHz, DMSO-$d_6$)δ 13.10(s, 1H), 10.32(d, J=6.7 Hz, 2H), 7.42(d, J=6.1 Hz, 2H), 7.28(d, J=6.2 Hz, 1H), 6.77(dt, J=16.9, 8.4 Hz, 2H), 6.15(dd, J=16.5, 7.2 Hz, 2H), 6.09(t, J=8.4 Hz, 2H); $^{13}C$ NMR(151 MHz, DMSO-$d_6$) δ 166.88, 139.38, 136.26, 132.96, 128.93, 115.36, 113.73.

**Example 9: Synthesis of 4-chloro-3-(chloromethyl)but-2-enoic acid (CL-09)**

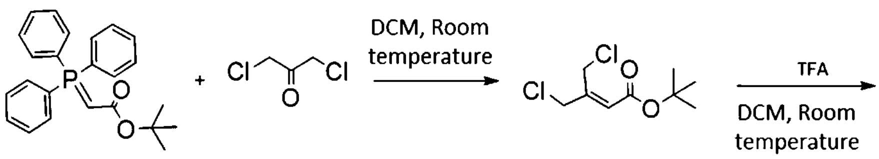


**CL-09**

To a reaction flask were added 1,3-dichloroacetone (678.14 mg, 5.30 mmol), tert-butyl triphenylphosphoacetate (2 g, 5.30 mmol), and DCM (20 mL). The reaction liquid was stirred at room temperature for 12 h. The solvent was removed under reduced pressure. Purification by silica gel column chromatography (PE/EA = 100:1) afforded tert-butyl 4-chloro-3-(chloromethyl)-2-butenoate (1 g, 4.29 mmol, yield 80.96%, HPLC 97 %); $^{1}H$ NMR(500 MHz, $CDCl_3$)δ 5.92(s, 1H), 4.70(s, 2H), 4.17(s, 2H), 1.43(s, 9H); $^{13}C$ NMR(126 MHz, $CDCl_3$)δ 164.04, 147.61, 124.21, 81.76, 45.69, 38.02, 28.08.

To a reaction flask were added tert-butyl 4-chloro-3-(chloromethyl)-2-butenoate (400 mg, 1.77 mmol), TFA (2 mL), and DCM (3 mL), which were stirred at room temperature for 2 h. The solvent was removed under reduced pressure. After filtration, 4-chloro-3-(chloromethyl)-2-butenoic acid **CL-09** as a white solid product (250 mg, 1.41 mmol, yield 79.80%, HPLC 96%) precipitated out; $^{1}H$ NMR(600 MHz, $CDCl_3$)δ 11.55(s, 1H),6.06(s, 1H), 4.72(s, 2H), 4.23(s, 2H); $^{13}C$ NMR(151 MHz, $CDCl_3$)δ 170.23, 152.23, 121.10, 45.37, 37.86.

**Example 10: Synthesis of 4-bromo-3-(bromomethyl)but-2-enoic acid (CL-10)**

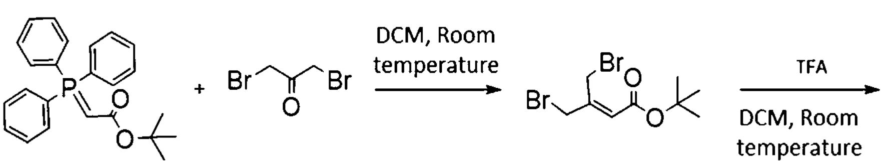


**CL-10**

Following a method similar to that described above for the synthesis of tert-butyl 4-chloro-3-(chloromethyl)-2-butenoate, tert-butyl 4-bromo-3-(bromomethyl)-2-butenoate (1.5 g, 4.67 mmol, yield 88.06%, HPLC 98 %) was obtained; $^{1}H$ NMR(500 MHz, $CDCl_3$)δ 5.89(s, 1H), 4.63(s, 2H), 4.08(s, 2H), 1.43(s, 9H); $^{13}C$ NMR(126 MHz, $CDCl_3$) δ 164.06, 148.37, 123.87, 81.77, 34.02, 28.09, 25.72.

The above product was treated with trifluoroacetic acid to give 4-bromo-3-(bromomethyl)-2-butenoic acid **CL-10** (1.0 g, 3.79 mmol, yield 79.49%, HPLC 98 %); $^{1}H$ NMR(500 MHz, $CDCl_3$)δ 11.32(s, 1H), 6.00(s, 1H), 4.65(s, 2H), 4.12(s,

2H); $^{13}C$ NMR(126 MHz, $CDCl_3$)δ 170.09, 153.05, 120.73, 33.41, 25.20.

**Example 11: Synthesis of 4,5-dibromomethyltriazol-1-ylacetamidoethoxyethoxypropanoyl-Gly-Lys-PAB-MMAE (C1)**

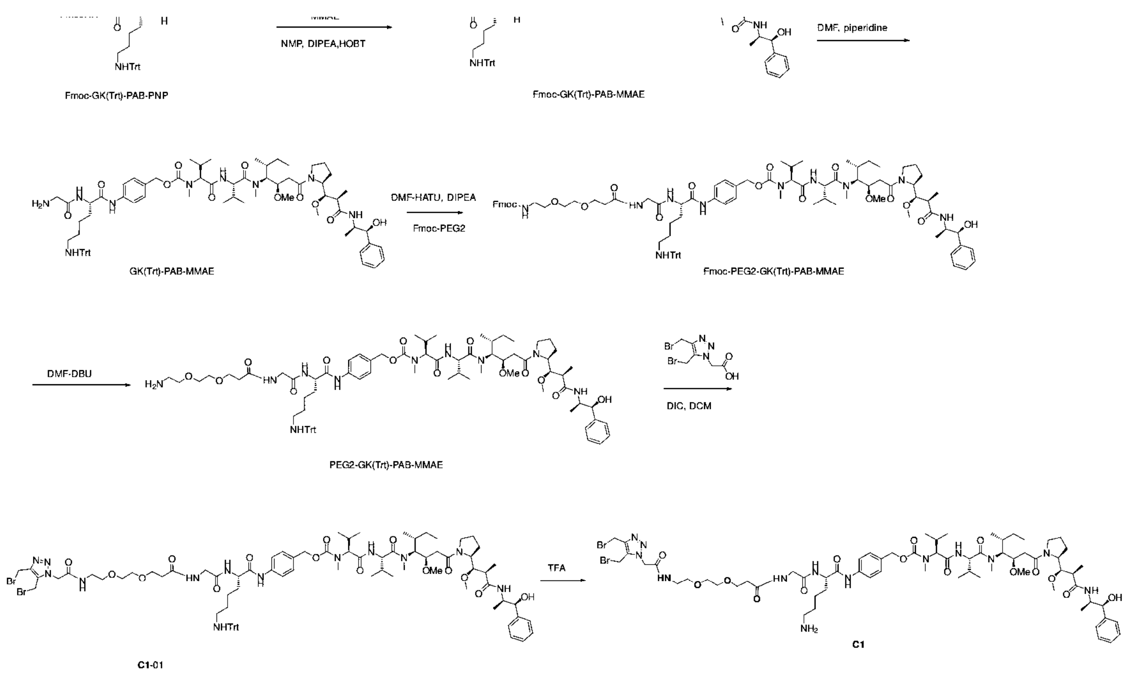


**(1) Synthesis of Fmoc-GK(Trt)-PAB-PNP**

Fmoc-Lys(Trt) (8.01 g, 12.33 mmol) and (4-aminophenyl)methanol (2.00 g, 12.33 mmol) were dissolved in a mixed solvent of DCM (50 mL) and MeOH (50 mL) and cooled to 0°C under stirring. EEDQ (3.05 g, 12.33 mmol) was added. The mixture was reacted under stirring at 0°C for 6 h. DBU (0.94 g, 6.16 mmol) was added to the reaction liquid and stirred at room temperature for 3 h. The reaction liquid was directly concentrated at room temperature, then dissolved in DCM (50 mL), and dropwise added to MTBE (500 mL). After filtration, a white solid precipitated out to give Lys(Trt)-PAB (5.2 g, yield 80%, HPLC 95%); LCMS: $[M+2]^+$ 495.13 (calculated value: 493.27).

To a reaction flask were added Lys(Trt)-PAB (5.2 g, 10 mmol), Fmoc-Gly (3.42 g, 10.2 mmol), and DCM (50 mL), which were stirred until fully dissolved. The mixture was cooled to 0°C. EDC hydrochloride (2.87 g, 15 mmol) was added. The mixture was maintained at 0°C and reacted for 3 h. The reaction liquid was extracted and passed through a column (PE:EA = 1:4) to give Fmoc-GK(Trt)-PAB as a white solid product (6.5 g, yield 84%, HPLC 94%); $^1H$ NMR(600 MHz, DMSO-$d_6$)δ 10.00(s, 1H), 8.12(d, J=7.5 Hz, 1H), 7.95-7.86(m, 2H), 7.74(d, J=7.4 Hz, 2H), 7.68-7.57(m, 3H), 7.53-7.14(m, 20H), 5.16(t, J=5.3 Hz, 1H), 4.47(dd, J=16.1, 5.9 Hz, 3H), 4.28(dd, J=24.2, 6.7 Hz, 3H), 3.72(s, 2H), 2.52(d, J=7.4 Hz, 1H), 1.97(s, 2H), 1.52(ddd, J=109.0, 87.5, 50.6 Hz, 6H); LCMS: $[M+1]^+$ 773.89 (calculated value: 772.36).

Fmoc-GK(Trt)-PAB (5.4 g, 6.65 mmol) was dissolved in DCM (50 mL). TEA (2.02 g, 19.95 mmol, 2.78 mL) and bis(4-nitrophenyl) carbonate (6.07 g, 19.95 mmol) were added and stirred for 2 h. The reaction liquid was directly added to silica gel, blended, and passed through a column (DCM:EA = 5:1) to give Fmoc-GK(Trt)-PAB-PNP as a white solid product (5.5 g, yield 85%, HPLC 97%); $^1H$ NMR(600 MHz, DMSO-$d_6$)δ 10.12(s, 1H), 8.36-8.25(m, 2H), 8.12(t, J=8.3 Hz, 1H), 7.88(d, J=7.5 Hz, 2H), 7.72-7.52(m, 6H), 7.43-6.87(m, 21H), 4.48-4.37(m, 1H), 4.29-4.17(m, 3H), 3.68(d, J=2.6 Hz, 2H), 3.35-3.16(m, 1H), 2.48(t, J=7.9 Hz, 1H), 1.93(d, J=6.9 Hz, 2H), 1.71-1.25(m, 6H); LCMS: $[M+1]^+$ 937.96 (calculated value: 937.37).

**(2) Synthesis of Fmoc-GK(Trt)-PAB-MMAE**

At room temperature, to a 100 mL reaction flask were successively added Fmoc-GK(Trt)-PAB-PNP (1.58 g, 1.68 mmol), MMAE (1.15 g, 1.60 mmol), HOBT (244.88 mg, 1.60 mmol), DIPEA (310.01 mg, 2.40 mmol, 417.80 μL), and N-methylpyrrolidone (10 mL). Stirring was continued at room temperature for a reaction for 3 h. To the reaction liquid was

added 5 g of silica gel powder to prepare a sand-like material, followed by purification by column chromatography using PE and EA to give Fmoc-GK(Trt)-PAB-MMAE as a light brown solid (2.2 g, 1.20 mmol, yield 75.3%, HPLC 93%); LCMS: $[M+1]^+$ 1517.58 (calculated value: 1516.93).

**(3) Synthesis of GK(Trt)-PAB-MMAE**

At room temperature, to a reaction flask were added Fmoc-GK(Trt)-PAB-MMAE (3.00 g, 1.98 mmol), DBU (301.43 mg, 1.98 mmol, 296.10 μL), and DMF (9 mL), which were reacted under stirring at room temperature for 1 h. 5 g of silica gel powder was added to prepare a sand-like material. Purification by column chromatography using DCM and MeOH afforded GK(Trt)-PAB-MMAE as an off-white solid (1.8 g, 1.25 mmol, 63.2%, HPLC 94 %); LCMS: $[M+1]^+$ 1295.35 (calculated value: 1294.69).

**(4) Synthesis of Fmoc-PEG2-GK(Trt)-PAB-MMAE**

At room temperature, to a reaction flask were successively added GK(Trt)-PAB-MMAE (1.8 g, 1.39 mmol), Fmoc-PEG2-COOH (555.35 mg, 1.39 mmol), HATU (528.52 mg, 1.39 mmol), and DIPEA (179.65 mg, 1.39 mmol, 242.11 μL), which were reacted under stirring at room temperature for 1 h. The reaction mixture was extracted with DCM (200 mL), washed with a saturated sodium chloride solution (100 mL * 3), dried over anhydrous magnesium sulfate, and filtered. To the DCM filtrate was added 5 g of silica gel powder to prepare a sand-like material. Purification by column chromatography using petroleum ether and ethyl acetate afforded Fmoc-PEG2-GK(Trt)-PAB-MMAE as a light yellow solid (2 g, 1.15 mmol, yield 82.41%, HPLC 96%); LCMS: $[M+1]^+$ 1677.48 (calculated value: 1676.62).

**(5) Synthesis of PEG2-GK(Trt)-PAB-MMAE**

At room temperature, to a reaction flask were successively added Fmoc-PEG2-GK(Trt)-PAB-MMAE (2 g, 1.19 mmol), DBU (181.66 mg, 1.19 mmol, 178.45 μL), and DMF (6 mL), which were reacted under stirring at room temperature for 1 h. The reaction liquid was purified by chromatography on C18 Spherical 20-35 um 100A 120 g reversed-phase column. The mobile phases were acetonitrile (HPLC grade) and 0.05% TFA aqueous solution as mobile phases B2 and A2, respectively. HPLC preparation method (monitoring at wavelengths 254 nm and 214 nm): 6 mL of a solution of a sample in DMF was injected into a reversed-phase column, the DMF solvent was washed away in 5 min (mobile phase ratio A2:B2 = 95:5), the proportion of acetonitrile was then increased to 40% in 80 min (mobile phase ratio from A2:B2 = 95:5 to A2:B2 = 60:40), the proportion of acetonitrile was then maintained at 40% for continued column washing for 60 min to elute a product, the product was collected, the purity of the product was detected by HPLC (220 nm) and LCMS, and the product was freeze-dried to give PEG2-GK(Trt)-PAB-MMAE as a white solid powder (1.3 g, 867.36 μmol, yield 72.69%, HPLC 97 %); $^1$H NMR(500 MHz, DMSO-$d_6$) δ 9.99(d, J=7.8 Hz, 1H), 8.13(t, J=5.7 Hz, 1H), 8.08(d, J=7.7 Hz, 1H), 7.88(d, J=7.6 Hz, 1H), 7.71-7.56(m, 2H), 7.52(ddd, J=8.5, 4.4, 2.2 Hz, 0H), 7.45(s, 1H), 7.35(dtd, J=34.2, 19.1, 17.0, 9.3 Hz, 10H), 7.23-7.14(m, 1H), 5.76(s, 0H), 5.06(ddd, J=32.9, 23.4, 13.9 Hz, 1H), 4.47(dt, J=29.1, 5.9 Hz, 1H), 4.38(p, J=7.7 Hz, 1H), 4.31-4.17(m, 2H), 4.03(q, J=7.1 Hz, 1H), 4.00(s, 1H), 3.74(d, J=5.6 Hz, 1H), 3.59(t, J=6.5 Hz, 2H), 3.47(s, 2H), 3.39(s, 11H), 3.22(dd, J=22.4, 11.5 Hz, 4H), 3.16-3.09(m, 2H), 2.98(s, 1H), 2.91-2.81(m, 2H), 2.54(s, 1H), 2.45-2.34(m, 2H), 2.27(dd, J=16.0, 9.3 Hz, 1H), 2.17-2.08(m, 1H), 1.99(s, 1H), 1.81(s, 1H), 1.57(s, 3H), 1.55-1.46(m, 2H), 1.32(s, 2H), 1.29-1.22(m, 1H), 1.18(t, J=7.1 Hz, 1H), 1.08-0.96(m, 3H), 0.91-0.72(m, 7H), 0.80(s, 3H); LCMS: $[M+1]^+$ 1454.72 (calculated value: 1453.88).

**(6) Synthesis of C1-01**

At room temperature, to a reaction flask were successively added **CL-07,** 2-[4,5-bis(bromomethyl)triazol-1-yl] acetic acid (86.10 mg, 275.13 μmol), DCM (5 mL), and DIC (17.36 mg, 137.57 μmol, 21.30 μL), which were reacted under stirring at room temperature for 5 min. PEG2-GK(Trt)-PAB-MMAE (200 mg, 137.57 μmol) were added. The reaction was continued at room temperature for 2 h. 5 g of silica gel powder was added to prepare a sand-like material. Purification by column chromatography using DCM and MeOH afforded **C1-01** as a light yellow solid (190 mg, yield 72.66%, HPLC 92 %); LCMS:$[M+1]^+$ 1749.64 (calculated value: 1748.81).

**(7) Synthesis of C1**

At room temperature, to a reaction flask were successively added **C1-01** (190 mg, 108.65 μmol), DCM (5 mL), and TFA (745.00 mg, 6.53 mmol, 500 μL), which were reacted under stirring at room temperature for 1 h. The reaction mixture was concentrated and dissolved with 3 mL of DMF to give a clear solution. The clear solution was purified by C18 reversed-phase column chromatography. The mobile phases were acetonitrile (HPLC grade) and 0.1% TFA aqueous

solution as mobile phases B2 and A2, respectively. HPLC preparation method (monitoring at wavelengths 254 nm and 214 nm) was used. After freeze drying, **C1** as a white solid powder (110 mg, yield 65.86%, HPLC 98 %) was obtained; $^{1}$H NMR(600 MHz, DMSO-$d_6$) δ 10.00(d, J=10.7 Hz, 1H), 8.54(h, J=6.5 Hz, 1H), 8.17(dd, J=17.1, 7.0 Hz, 2H), 8.07(d, J=8.3 Hz, 1H), 7.89(d, J=8.6 Hz, 1H), 7.61(dd, J=17.9, 10.3 Hz, 5H), 7.37-7.27(m, 4H), 7.26(td, J=7.6, 2.7 Hz, 2H), 7.17(q, J=6.0, 4.9 Hz, 1H), 5.14(s, 1H), 5.16-5.09(m, 1H), 5.04(d, J=13.7 Hz, 1H), 4.94-4.80(m, 3H), 4.49(p, J=6.4, 5.8 Hz, 1H), 4.45-4.37(m, 2H), 4.26(dd, J=19.0, 10.8 Hz, 1H), 4.01(dd, J=15.0, 7.2 Hz, 1H), 4.00-3.91(m, 1H), 3.82-3.70(m, 2H), 3.62(t, J=6.4 Hz, 2H), 3.57(s, 5H), 3.57(dd, J=12.1, 5.1 Hz, 1H), 3.48(s, 6H), 3.44(q, J=6.8, 6.3 Hz, 2H), 3.33-3.25(m, 1H), 3.24(t, J=9.0 Hz, 4H), 3.19(d, J=16.0 Hz, 3H), 3.12(s, 2H), 2.97(s, 1H), 2.86(dd, J=22.4, 13.2 Hz, 3H), 2.77(h, J=6.7 Hz, 2H), 2.41(t, J=6.6 Hz, 3H), 2.26(dd, J=15.4, 9.2 Hz, 1H), 2.16-2.03(m, 2H), 1.61(dd, J=9.3, 4.8 Hz, 1H), 1.55(qd, J=13.9, 12.9, 7.1 Hz, 3H), 1.48(s, 1H), 1.36(td, J=16.2, 13.4, 7.1 Hz, 1H), 1.33-1.26(m, 2H), 1.25-1.14(m, 1H), 1.01(ddd, J=18.8, 17.1, 6.7 Hz, 6H), 0.93(s, 2H), 0.86(ddd, J=15.7, 13.5, 6.6 Hz, 6H), 0.79(s, 9H), 0.76(dt, J=11.6, 7.4 Hz, 4H); LCMS: $[M+1]^+$ 1507.31 (calculated value: 1506.49).

**Example 12: Synthesis of 4,5-dibromomethyltriazol-1-ylacetamidoethoxyethoxypropionyl-Gly-Lys-PAB-(10,11-methylenedioxycamptothecin-7-ethyl)-(N-(S)-tetrahydrofuran-3-amine (C2)**

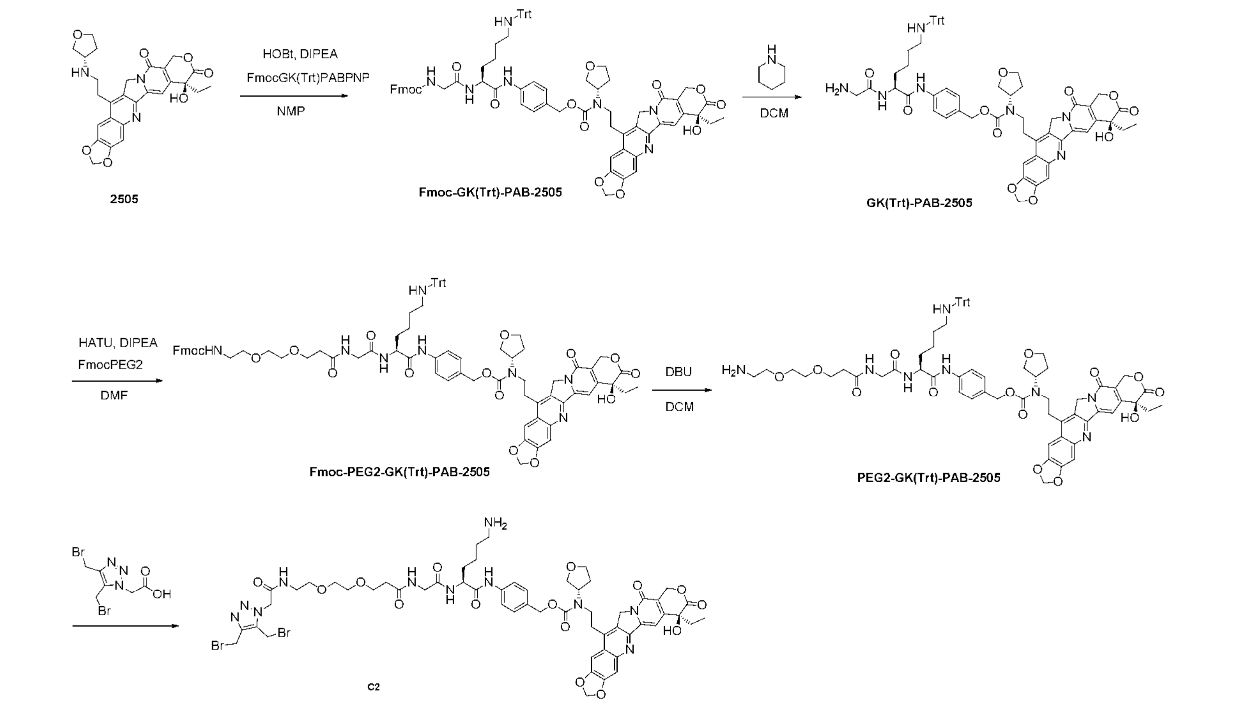


**(1) Synthesis of 2505**

To a reaction flask were added separately (S)-3-aminotetrahydrofuran (64.31 mg, 0.738 mmol), concentrated hydrochloric acid (0.05 mL, 0.6 mmol), DMSO (1.5 mL), and 7-methyl-10,11-methylenedioxycamptothecin (50 mg, 0.12 mmol). The mixture was heated to 120-130°C under stirring and reacted for 1 h. The reaction mixture was cooled to room temperature. Tert-butyl methyl ether was added. After filtration, a solid precipitated out. The solid was purified by silica gel column chromatography to give the product 7-(2-(N-(S)-tetrahydrofuran-3-amino))ethyl-10,11-methylenedioxycamptothecin (18 mg, yield 33%, HPLC 95.9%); $^{1}$H NMR(500 MHz, DMSO-$d_6$) δ 7.61(d, J=3.4 Hz, 1H), 7.47(d, J=3.9 Hz, 1H), 7.22(d, J=4.2 Hz,1H), 6.50(s, 1H), 6.29(d, J=4.0 Hz, 2H), 5.42(d, J=4.0 Hz, 2H), 5.21(d, J=3.2 Hz, 2H), 3.87-3.58(m, 4H), 3.21(s, 2H), 2.85(td, J=11.2, 10.7, 5.8 Hz, 2H), 1.90(ddt, J=29.9, 10.7, 6.2 Hz, 3H), 1.68-1.60(m, 1H), 1.08-0.76(m, 3H); $^{13}$C NMR(126 MHz, DMSO-$d_6$) δ 173.0, 157.3, 151.3, 150.6, 149.7, 149.4, 147. 6, 146.9, 141.2, 128.6, 124.9, 118.5, 105.9, 103.1, 100.0, 96.4, 72.9, 72.7, 66.9, 65.7, 58.4, 50.4, 47.6, 32.6, 30.7, 30.6, 8.2; LC-MS: $[M+H]^+$ 506.31 (theoretical value 505.18).

**(2) Synthesis of GK(Trt)-PAB-2505**

**2505** (4 g, 7.57 mmol) and **Fmoc-GK(Trt)-PAB-PNP** (7.27 g, 7.57 mmol) were dissolved in 40 mL of NMP. HOBT (1.74 g, 11.35 mmol) and DIPEA (1.47 g, 11.35 mmol, 1.98 mL) were added. The mixture was reacted under stirring at room temperature for 4 h. The reaction product **Fmoc-GK(Trt)-PAB-2505** (LCMS: $[M+2]^+$ 1303.66; theoretical value 1303.53) was detected by HPLC. Piperidine (3.45 g, 40.49 mmol, 4 mL) was directly added to the reaction liquid. The mixture was stirred at room temperature for 0.5 h. 300 mL of tert-butyl methyl ether was added, followed by filtration and precipitation. The filter cake was redissolved in 80 mL of DCM. 500 mL of tert-butyl methyl ether was added. A solid precipitated out. After filtration, the filter cake was dried to give **GK(Trt)-PAB-2505** (6.5 g, 2.96 mmol, yield 39.12%, HPLC 93%); LCMS: $[M+1]^+$ 1081.86 (theoretical value 1081.46).

**(3) Synthesis of Fmoc-PEG2-GK(Trt)-PAB-2505**

Fmoc-$PEG_2$ (2.48 g, 5.88 mmol) was dissolved in DCM (100 mL). **GK(Trt)-PAB-2505** (6.5 g, 5.88 mmol), HATU (3.35 g, 8.82 mmol), and DIPEA (1.14 g, 8.82 mmol, 1.54 mL) were added and stirred at room temperature for 2 h. 20 g of silica gel was added. Purification by column chromatography afforded **Fmoc-PEG2-GK(Trt)-PAB-2505** (5 g, 2.97 mmol, yield 50.53%, HPLC 95%); $^1H$ NMR(500 MHz, DMSO-$d_6$)$\delta$ 9.99(s, 1H), 8.13(s, 1H), 8.07(d, J=7.8 Hz, 1H), 7.86(d, J=7.5 Hz, 2H), 7.65(dd, J=18.6, 7.9 Hz, 4H), 7.53-7.47(m, 2H), 7.43-7.23(m, 22H), 6.49(s, 1H), 6.27(s, 2H), 5.76(s, 1H), 5.43(s, 2H), 5.25(s, 2H), 5.14(s, 2H), 4.58(s, 1H), 4.36(s, 1H), 4.27(d, J=6.9 Hz, 2H), 4.18(s, 1H), 4.10(s, 1H), 3.90(s, 1H), 3.76-3.67(m, 3H), 3.65-3.54(m, 5H), 3.46(s, 5H), 3.38(d, J=5.7 Hz, 4H), 3.17(s, 3H), 3.14-3.09(m, 2H), 2.69(s, 1H), 2.37(t, J=6.4 Hz, 2H), 2.18-2.08(m, 1H), 1.85(dq, J=14.0, 7.3 Hz, 3H), 1.66(s, 1H), 1.53(d, J=9.2 Hz, 2H), 1.36-1.21(m, 2H), 0.88(t, J=7.3 Hz, 3H); LCMS: $[M+1]^+$ 1462.88 (theoretical value 1462.62).

**(4) Synthesis of PEG2- GK(Trt)-PAB-2505**

**Fmoc-PEG2-GK(Trt)-PAB-2505** (5.0 g, 3.36 mmol) was dissolved in DCM (30 mL). DBU (512.02 mg, 3.36 mmol, 502.97 μL) was added. The mixture was stirred at room temperature for 1 h. The reaction liquid was added to 300 mL of tert-butyl ether. A solid precipitated out. After filtration, a solid product was obtained, which was redissolved in 50 mL of DCM and then added to 500 mL of tert-butyl methyl ether. A solid precipitated out, which was filtered and dried to give **PEG2-GK(Trt)-PAB-2505** (4.5 g, 2.54 mmol, yield 75.45%, HPLC 93%); LCMS: $[M+1]^+$ 1240.90 (theoretical value 1240.55).

**(5) Synthesis of C2**

**PEG2-GK-PAB-2505** (100 mg, 80.56 μmol) was dissolved in DCM (2 mL). 2-[4,5-bis(bromomethyl)triazol-1-yl] acetic acid (25.21 mg, 80.56 μmol) and DIC (12.20 mg, 96.67 μmol, 14.97 μL) were added. The mixture was stirred at room temperature for 1 h. After the reaction was complete by detection (LCMS: $[M+2]^+$ 1535.82; theoretical value 1533.43), TFA (447.00 mg, 3.92 mmol, 0.3 mL) was added. The mixture was stirred at room temperature for 2 h, concentrated, and dissolved in 2 mL of DMF to give a clear solution, which was purified by chromatography on C18 Spherical 20-35 um 100A 40 g reversed-phase column. The mobile phases were acetonitrile (HPLC grade) and 0.1% TFA aqueous solution as mobile phases B2 and A2, respectively. HPLC preparation method (monitoring at wavelengths 254 nm and 214 nm) was used. After freeze drying, **C2** as a yellow solid powder product (58 mg, yield 51.7%, HPLC 93%) was obtained; $^1H$ NMR(600 MHz, DMSO-$d_6$)$\delta$ 10.07(s, 1H), 8.20(d, J=7.6 Hz, 2H), 7.85-7.61(m, 9H), 7.51(s, 1H), 7.24(s, 1H), 6.29(s, 2H), 5.43(s, 2H), 5.27(dd, J=12.2, 7.8 Hz, 3H), 5.14(s, 3H), 4.94(s, 1H), 4.84(s, 1H), 4.61(s, 1H), 4.60-4.56(m, 2H), 3.77(d, J=5.1 Hz, 2H), 3.57(ddd, J=31.8, 23.9, 8.3 Hz,12H), 2.99-2.92(m, 2H), 2.81-2.73(m, 2H), 2.41(s, 2H), 1.86(dq, J=13.6, 7.2 Hz, 3H), 1.54(d, J=7.8 Hz, 2H), 0.88(t, J=7.2 Hz, 3H); LCMS:$[M+2]^+$ 1293.61 (theoretical value 1291.32).

**Example 13: Synthesis of 4,5-dibromomethyltriazol-1-ylacetamidoethoxyethoxypropionyl-Gly-Val-Ala-ha-amino(methyl)oxyacetylexatecan (C3)**

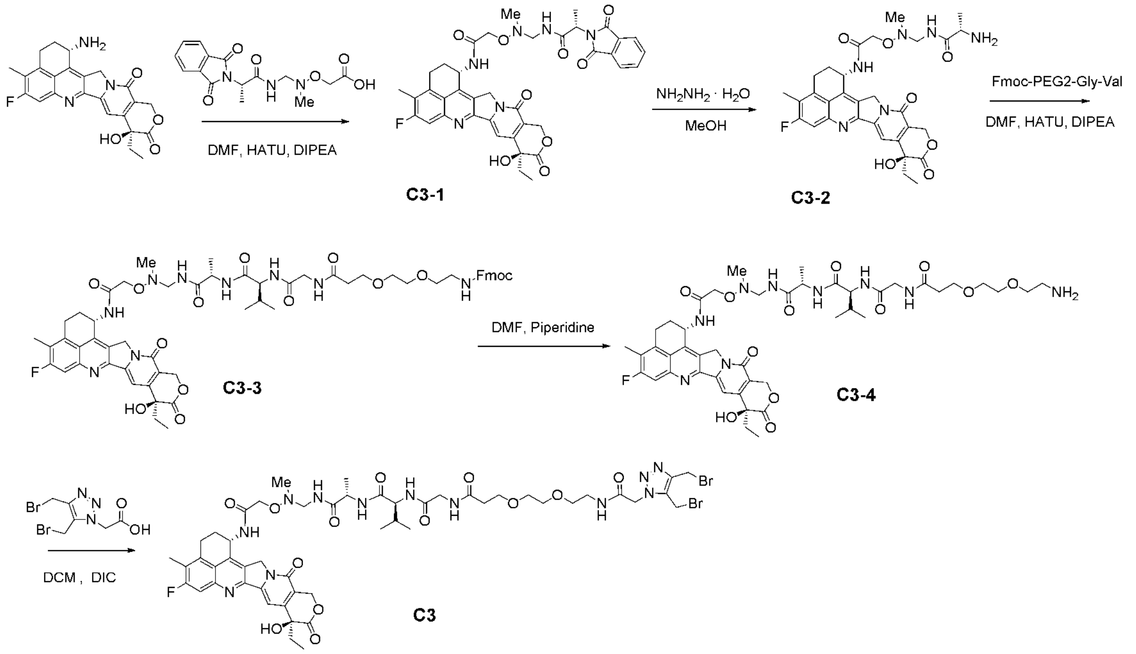


C3-1 C3-2 C3-3 C3-4 C3

**(1) Synthesis of C3-1**

Exatecan mesylate (0.26 g, 0.5 mmol) was added to 2 mL of DMF, followed by addition of HATU (0.19 g, 0.5 mmol), DIPEA (0.129 g, 1 mmol), and phthaloyl-Ala-ha-amino(N-methyl)oxyacetic acid (200 mg, 0.59 mmol). The mixture was reacted at room temperature for 15 h. The reaction mixture was diluted with 200 mL of DCM. After washing with a saturated sodium chloride aqueous solution, the resulting material was dried over anhydrous sodium sulfate and purified by silica gel column chromatography to give **C3-1** as a yellow solid (0.3 mg, yield 81%, HPLC purity 92%); LCMS: $[M+1]^+$ 753.12 (theoretical value: 752.26).

**(2) Synthesis of C3-2**

**C3-1** (0.1 g, 0.13 mmol) was dissolved in 5 ml of MeOH. Hydrazine hydrate (39.9 mg, 0.79 mmol) was added. The tube was sealed, heated to 60°C, and reacted for 2 h. The reaction mixture was cooled. The reaction liquid was poured into 100 mL of a saturated aqueous sodium chloride solution, extracted with 200 mL of ethyl acetate, and concentrated to give **C3-2** as a crude product (0.07 g, yield 77%, HPLC purity 86%); LCMS: $[M+1]^+$623.15 (theoretical value: 622.26).

**(3) Synthesis of C3-3**

**C3-2** (70 mg, 0.112 mmol) was dissolved in 2 mL of DMF. DIPEA (29 mg, 0.224 mmol) was added and stirred to give a clear solution. Fmoc-PEG2-Gly-Val (62.16 mg,0.112 mmol) and HATU (42.56 mg,0.112 mmol) were successively added to the clear solution. The mixture was reacted under stirring at room temperature for 1 h. The reaction liquid was directly concentrated. Silica gel was added for purification by silica gel column chromatography to give the compound **C3-3** (81 mg, yield 62%, HPLC purity 93%); LCMS: $[M+1]^+$ 1160.24 (theoretical value: 1159.50).

**(4) Synthesis of C3-4**

To a 10 mL single-necked flask was added **C3-3** (81 mg, 0.069 mmol), which was dissolved with 1 mL of DMF. 100 $\mu$L of piperidine was then added. After reaction at room temperature for 1 h, the reaction was complete. The reaction liquid was dropwise added to 20 mL of tert-butyl methyl ether. After centrifugation, the supernatant was removed. After drying, **C3-4** (32 mg, yield 49%, HPLC purity 95%) was obtained; LCMS: $[M+1]^+$ 938.02 (theoretical value: 937.43).

**(5) Synthesis of C3**

2-(4,5-Dibromomethyl-1-H-1,2,3-triazol-1-yl)acetic acid (129.7 mg, 0.416 mmol) and DIC (52.4 mg, 0.416 mmol) were added to a reaction flask, dissolved with DCM (2.5 mL), and stirred at room temperature for 5 min. The mixed liquid was added to a mixed solution of **C3-4** (300 mg, 0.320 mmol) dissolved in DCM/DMF (2.5 mL/1.0 mL). The mixture was

reacted at room temperature 25°C for 1 hour. The reaction liquid was dropwise added to 50 mL of tert-butyl methyl ether. A white solid precipitated out. The solid was dissolved with DMF (2 mL) and then purified by elution by medium-pressure reversed-phase C18 chromatography (gradient: acetonitrile / 0.1% TFA aqueous solution, 0% to 55%, time 100 min), followed by freeze drying to give **C3** as a yellow solid (151.0 mg, yield 38.3%, HPLC 97%); $^1$H NMR(500 MHz, DMSO-$d_6$) δ 8.60-8.47(m, 1H), 8.47-8.35(m, 11H), 8.33-8.20(m, 1H), 8.17-8.05(m, 1H), 8.05-7.92(m, 1H), 7.85-7.70(m, 2H), 7.30(s, 1H), 5.65-5.55(m, 1H), 5.42(s, 2H), 5.05-5.32(m, 4H), 4.62-4.42(m, 6H), 4.33-4.24(m, 1H), 4.24-4.01(m, 5H), 3.80-3.65(m, 2H), 3.64-3.54(m, 2H), 3.54-3.38(m, 6H), 3.32-3.04(m, 4H), 2.47(s, 2H), 2.42-2.30(m, 5H), 2.24-2.10(m, 2H), 1.92-1.76(m, 3H), 1.28-1.18(m, 1H), 1.17-1.08(m, 2H), 0.92-0.76(m, 5H), 0.76-0.62(m, 4H); LC-MS: [M+H]$^+$ 1232.54 (theoretical value 1232.31).

### Example 14: Synthesis of 2,3-dibromomethylquinoxaline-6-carboxamidoethoxyethoxypropanoyl-Gly-Lys-PAB-MMAE (C4)

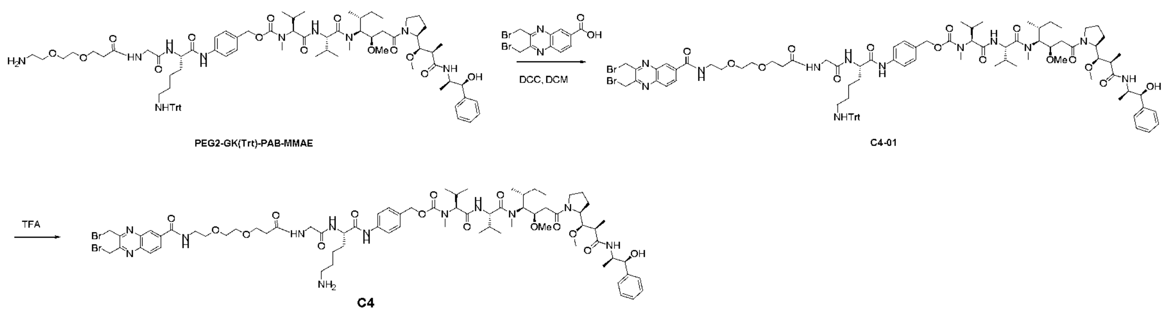


PEG2-GK(Trt)-PAB-MMAE

C4-01

C4

#### (1) Synthesis of C4-01

At room temperature, to a reaction flask were successively added 2,3-bis(bromomethyl)quinoxaline-6-carboxylic acid (148.57 mg, 412.70 μmol), DCC (42.58 mg, 206.35 μmol), and DCM (5 mL), which were reacted under stirring at room temperature for 1 h. After filtration, the resulting material was transferred to a 100 mL reaction flask, to which were successively added PEG-GK(Trt)-PAB-MMAE (300 mg, 206.35 μmol), DMF (3 mL), and DIPEA (26.67 mg, 206.35 μmol, 35.94 μL). The mixture was reacted under stirring at room temperature for 1 h. 5 g of silica gel powder was added to prepare a sand-like material. Purification by column chromatography using DCM and MeOH afforded **C4-01** as a light yellow solid (177 mg, yield 38.21%, HPLC 94%); LCMS: [M+1]$^+$ 1796.52 (theoretical value: 1795.87).

#### (2) Synthesis of C4

At room temperature, to a reaction flask were successively added **C4-1** (177 mg, 98.56 μmol), DCM (5 mL), and TFA (745.00 mg, 6.53 mmol, 500 μL). The mixture was reacted under stirring at room temperature for 1 h, concentrated, and dissolved in 3 mL of DMF to give a clear solution. The clear solution was purified by C18 reversed-phase column chromatography. The mobile phases were acetonitrile (HPLC grade) and 0.1% TFA aqueous solution as mobile phases B2 and A2, respectively. HPLC preparation method (monitoring at wavelengths 254 nm and 214 nm) was used. After freeze drying, **C4** as a white solid powder (29 mg, 17.92 μmol, yield 18.18%, HPLC 96%) was obtained; $^1$H NMR(600 MHz, DMSO-$d_6$)δ 9.99(s, 1H), 8.95(q, J=9.5, 7.5 Hz, 1H), 8.64-8.56(m, 1H), 8.29(dt, J=10.1, 7.3 Hz, 1H), 8.19-8.15(m, 1H), 8.13(d, J=8.1 Hz, 1H), 8.07(d, J=7.8 Hz, 1H), 7.89(d, J=8.7 Hz, 0H), 7.61(qd, J=9.2, 6.3, 4.9 Hz, 5H), 7.35-7.23(m, 4H), 7.16(s, 1H), 5.95(dd, J=14.0, 5.1 Hz, 1H), 5.41(s, 0H), 5.05(dd, J=13.3, 3.6 Hz, 3H), 4.73(s, 0H), 4.48(d, J=5.8 Hz, 1H), 4.41(d, J=11.3 Hz, 2H), 4.26(dd, J=19.2, 10.8 Hz, 1H), 3.99(s, 1H), 3.80-3.69(m, 2H), 3.64-3.53(m, 5H), 3.53-3.45(m, 4H), 3.26-3.19(m, 5H), 3.17(s, 1H), 3.11(s, 2H), 3.07-3.02(m, 0H), 2.97(s, 1H), 2.90-2.80(m, 3H), 2.77(q, J=6.8 Hz, 2H), 2.44-2.36(m, 2H), 2.26(dd, J=16.0, 9.5 Hz, 1H), 2.11(s, 1H), 1.99(dq, J=21.7, 8.3, 7.8 Hz, 1H), 1.81(s, 1H), 1.74(s, 3H), 1.53(q, J=8.4, 8.0 Hz, 2H), 1.49(s, 3H), 1.36(d, J=8.5 Hz, 1H), 1.30(s, 3H), 1.24(s, 1H), 1.06-0.96(m, 5H), 0.85(tt, J=12.9, 6.3 Hz, 5H), 0.79(s, 11H), 0.76(dt, J=11.7, 7.3 Hz, 3H); LCMS: [M+1]$^+$ 1554.32 (theoretical value: 1553.54).

### Example 15: Synthesis of 2,3-dibromomethylquinoxaline-6-carboxamidoethoxyethoxypropanoyl-Gly-Lys-PAB-(10,11-methylenedioxycamptothecin-7-ethyl)-(N-(S)-tetrahydrofuran-3-)amine (C5)

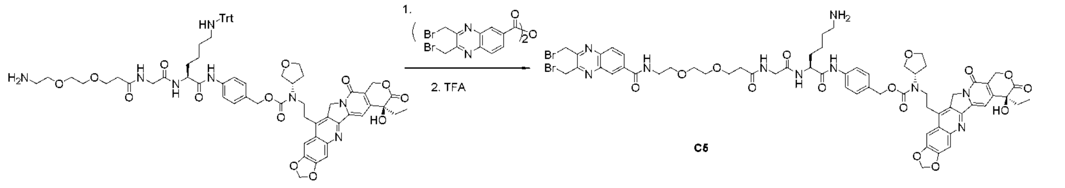


C5

At room temperature 25°C, a 25 mL single-necked flask was taken, to which were successively added DCM (2 mL), 2,3-bis(bromomethyl)quinoxaline-6-carboxylic acid (1.12 g, 3.10 mmol), and DCC (320 mg, 1.55mmol). The mixture was reacted under stirring at room temperature for 6 h. After filtration and rotary evaporation to dryness, 2,3-bis(bromomethyl)quinoxaline-6-carboxylic acid anhydride was obtained, which was directly used in the next reaction.

PEG2-GK(Trt)-PAB-2505 (260 mg, 209.44 μmol) was dissolved in DCM (5 mL). 2,3-bis(bromomethyl)quinoxaline-6-carboxylic acid anhydride (147.03 mg, 209.44 μmol) was added and stirred at room temperature for 1 h (LCMS: $[M+2]^+$ 1582.26 (theoretical value 1580.43). TFA (745.00 mg, 6.53 mmol, 0.5 mL) was added. The mixture was stirred at room temperature for 2 h. The reaction liquid was concentrated and dissolved with 2 mL of DMF to give a clear solution. The clear solution was purified by chromatography on C18 Spherical 20-35 um 100A 80 g reversed-phase column. The mobile phases were acetonitrile (HPLC grade) and 0.1% TFA aqueous solution as mobile phases B2 and A2, respectively. HPLC preparation method (monitoring at wavelengths 254 nm and 214 nm) was used. After freeze drying, **C5** as a yellow solid powder product (38 mg, 26.88 μmol, yield 12.83%, HPLC 94%) was obtained; 1H NMR(500 MHz, DMSO-d6)δ 8.14(dd, J=23.0, 15.1 Hz, 3H), 7.64(d, J=8.1 Hz, 6H), 7.41(s, 2H), 5.95(d, J=13.0 Hz, 1H), 5.43(s, 2H), 5.27(s, 2H), 5.13(s, 2H), 4.41(s, 1H), 3.82(d, J=71.8 Hz, 7H), 3.61-3.23(m, 21H), 2.77(d, J=6.0 Hz, 2H), 2.39(s, 2H), 2.14(d, J=8.0 Hz, 1H), 1.92-1.69(m, 4H), 1.68-1.47(m, 4H), 0.87(t, J=7.3 Hz, 3H); LCMS: $[M+2]^+$ 1340.51 (theoretical value 1338.32).

**Example 16: Synthesis of 2,3-dibromomethylquinoxaline-6-carboxamidoethoxyethoxypropanoyl-Gly-Val-Ala-ha-amino(methyl)oxyacetylexatecan (C6)**

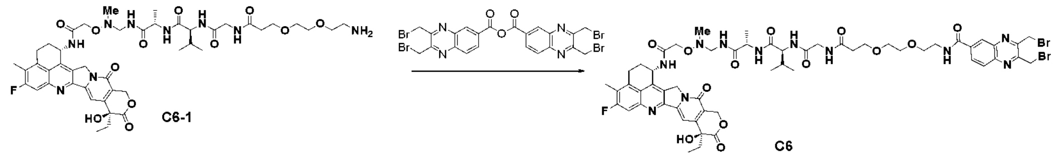

C6-1

C6

At room temperature, to a reaction flask were successively added DMF (5 mL), **C6-1** (0.94 g, 1 mmol), and 2,3-bis(bromomethyl)quinoxaline-6-carboxylic acid anhydride (0.7 g, 1 mmol), which were reacted under stirring at room temperature for 0.5 h. The reaction liquid was purified by reversed-phase preparative chromatography on C18 Spherical 20-35 um 100A 80 g reversed-phase column. The mobile phases were acetonitrile (HPLC grade) and 0.05% TFA aqueous solution as mobile phases B2 and A2, respectively. HPLC preparation method (monitoring at wavelengths 254 nm and 214 nm) was used. After freeze drying, **C6** as a yellow solid powder (456 mg, 0.36 mmol, yield 35%, HPLC 97%) was obtained; $^1$H NMR(500 MHz, DMSO-d*6*)δ 8.95(dt, J=10.0, 5.4 Hz, 1H), 8.71-8.49(m, 2H), 8.38-8.21(m, 1H), 8.25(s, 1H), 8.12(dt, J=8.3, 4.5 Hz, 1H), 7.98(d, J=7.2 Hz, 1H), 7.72(dd, J=9.7, 6.7 Hz, 2H), 7.29(d, J=3.8 Hz, 1H), 5.59(p, J=7.6, 6.0 Hz, 1H), 5.41(s, 2H), 5.30-5.20(m, 1H), 5.15-5.06(m, 1H), 4.80(s, 8H), 4.28(dd, J=13.2, 6.6 Hz, 1H), 4.24-4.11(m, 2H), 4.10(s, 3H), 4.08-3.94(m, 1H), 3.77-3.66(m, 2H), 3.69-3.59(m, 1H), 3.59(s, 1H), 3.59-3.51(m, 6H), 3.48(dt, J=11.5, 5.3 Hz, 4H), 3.11(d, J=17.2 Hz, 1H), 2.92(d, J=8.3 Hz, 1H), 2.47(s, 3H), 2.36(s, 4H), 2.34(d, J=6.3 Hz, 1H), 2.18(p, J=6.6, 5.6 Hz, 2H), 2.07(s, 1H), 1.98(s, 1H), 1.93-1.76(m, 4H), 1.25-1.11(m, 4H), 0.91-0.78(m, 4H), 0.70(dd, J=12.8, 6.8 Hz, 5H); LCMS: $[M+2]^+$ 1280.01 (theoretical value: 1277.32).

**Example 17: Synthesis of 2,6-dibromomethylpyridin-4-yloxyacetamidoethoxyethoxypropionyl-Gly-Lys-PAB-MMAE (C7)**

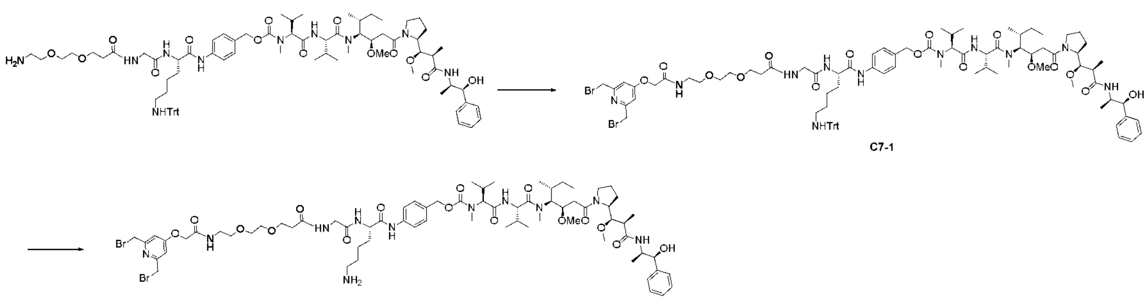

C7-1

C7

(1) Synthesis of C7-1

At room temperature 25°C, a 100 mL single-necked flask was taken, to which were successively added PEG-GK(Trt)-PAB-MMAE (150 mg, 103.17 μmol), **CL-06** (69.95 mg, 206.35 μmol), and DIC (13.02 mg, 103.17 μmol, 15.98 μL). The mixture was reacted under stirring at room temperature for 1 h. 10 g of silica gel powder was added to prepare a sand-like material. Purification by column chromatography using DCM and MeOH afforded **C7-1** as a brown solid product (130 mg, yield 70.99%); LCMS: $[M+1]^+$ 1775.69 (theoretical value: 1774.84).

(2) Synthesis of C7

At room temperature 25°C, a 100 mL single-necked flask was taken, to which were successively added **C7-1** (130 mg, 73.25 μmol), DCM (5 mL), and TFA (83.52 mg, 732.47 μmol, 56.05 μL). The mixture was reacted under stirring at room temperature for 1 h and purified by C18 Spherical 20-35 um 100A 120g reversed-phase column. The mobile phases were acetonitrile and 0.1% TFA aqueous solution as mobile phases B2 and A2, respectively. HPLC preparation method (monitoring at wavelengths 254 nm and 214 nm) was used. The product was collected and freeze-dried to give **C7** as a white solid product (54 mg, yield 46.66%, HPLC 97%); $^1$H NMR (600 MHz, DMSO-$d_6$) δ 10.02-9.97 (m, 1H), 8.25-8.12 (m, 3H), 8.10-8.04 (m, 1H), 7.61(dd, J=18.2, 10.4 Hz, 7H), 7.37-7.23 (m, 7H), 7.17 (td, J=11.7, 10.4, 4.2 Hz, 1H), 7.08 (s, 1H), 5.35(s, 1H), 5.04 (d, J=13.3 Hz, 2H), 4.61 (d, J=10.3 Hz, 5H), 4.49 (d, J=6.2 Hz, 1H), 4.41 (dt, J=8.6,4.5 Hz, 2H), 4.26 (dd, J=18.8, 10.7 Hz, 1H), 4.03-3.93 (m, 2H), 3.81-3.70 (m, 3H), 3.61 (t, J=6.5Hz, 3H), 3.48 (s, 5H), 3.43 (t, J=5.9 Hz, 2H), 3.30-3.16 (m, 9H), 3.12 (s, 2H), 2.97 (s, 2H), 2.86 (dd, J=22.4, 13.4 Hz, 3H), 2.77 (h, J=6.5 Hz, 3H), 2.40 (q, J=8.4, 6.4 Hz, 4H), 2.26 (t, J=13.0 Hz, 1H),2.12 (d, J=6.8 Hz, 2H), 1.78-1.75 (m, 3H), 1.75-1.70 (m, 2H), 1.54 (p, J=7.2 Hz, 4H), 1.36 (d, J=7.0 Hz, 1H), 1.33-1.29 (m, 3H), 1.06-0.96 (m, 7H), 0.95-0.72 (m, 14H); LCMS: $[M+1]^+$ 1533.38 (theoretical value: 1532.52).

**Example 18: Synthesis of 2,6-dibromomethylpyridin-4-yloxyacetamidoethoxyethoxypropionyl-Gly-Val-Ala-ha-amino(methyl)oxyacetylexatecan (C8)**

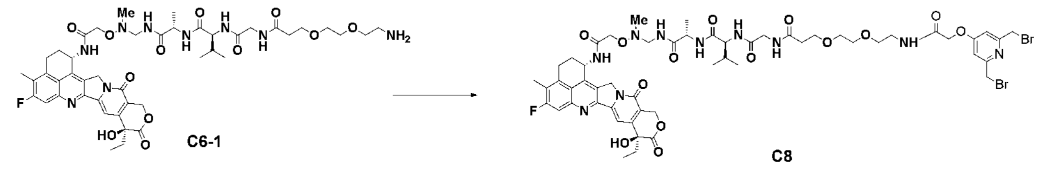

C6-1

C8

At 25°C, to a 10 mL single-necked eggplant-shaped flask were successively added **C6-1** (100 mg, 106.61 μmol), DCM (2 mL), and **CL-06** (36.14 mg, 106.61 μmol). The mixture was reacted under stirring at room temperature for 1 h. The reaction liquid was concentrated and dissolved in 3 mL of DMF to give a clear solution. The clear solution was purified by reversed-phase preparative chromatography on C18 Spherical 20-35 um 100A 40 g reversed-phase column. The mobile phases were acetonitrile (HPLC grade) and 0.1% TFA aqueous solution as mobile phases B2 and A2, respectively. HPLC preparation method (monitoring at wavelengths 254 nm and 214 nm) was used. A product was collected. The purity of the product was detected by HPLC (220 nm) and LCMS. The product was freeze-dried to give **C8** as a yellow solid powder (31 mg, 23.64 μmol, yield 22.17%, HPLC 96%); $^1$H NMR (600 MHz, DMSO-d6) δ 8.51 (d, J=8.9 Hz, 1H), 8.23 (dt, J=11.3, 6.0 Hz, 2H), 8.11 (t, J=5.7 Hz, 1H), 7.99 (d, J=7.1 Hz, 1H), 7.81-7.73 (m, 2H), 7.31 (s, 1H), 7.09 (s, 1H), 5.61 (dt, J=8.9, 6.0 Hz, 1H), 5.42 (s, 2H), 5.32-5.08 (m, 2H), 4.61 (d, J=11.3 Hz, 5H), 4.29 (dd, J=13.2, 6.7 Hz, 1H), 4.22-4.11 (m, 3H), 4.07 (dd, J=11.6, 5.3 Hz, 2H), 3.72 (td, J=16.2, 5.6 Hz, 2H), 3.58 (t, J=6.5 Hz, 2H), 3.48 (qd, J=5.0, 2.5 Hz, 4H), 3.43 (t, J=6.0 Hz, 2H),

3.27 (q, J=5.9 Hz, 2H), 3.23-2.98 (m, 1H), 2.47 (s, 3H), 2.41-2.33 (m, 5H), 2.19 (q, J=6.4 Hz, 2H), 1.93-1.79 (m, 3H), 1.16-1.09 (m, 3H), 0.87 (t, J=7.3 Hz, 3H), 0.71 (dd, J=15.6, 6.7 Hz, 5H). LCMS: $[M+1]^+$ 1260.30 (theoretical value: 1258.99).

**Example 19: Conjugation of C1 to HS627 antibody and preparation of dual-drug ADC (1) Preparation of HS627-$(C1)_2$ drug conjugate**

HS627 antibody (10 mg/mL, 5 mL) was taken. A TCEP.HCl (tris(2-carboxyethyl)phosphine hydrochloride) solution (2.86 mg/mL, 0.133 mL) was added and reacted at 0°C for 3 h. **C1** compound (10 mg/mL, 0.2 mL) dissolved in DMA (N,N-dimethylacetamide) was added to the above solution system. The mixture was uniformly mixed and reacted at 25°C for 4 h. After the reaction was complete, an NAC (N-acetyl-L-cysteine) solution (10 mg/mL, 0.11 mL) was added for quenching. Small molecules were removed by an ultrafiltration membrane to give an **HS627-$(C1)_n$** drug-antibody conjugate.

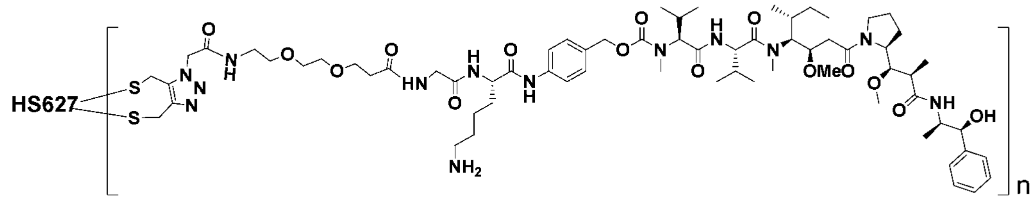

UV-HPLC, calculated DAR value: n = 2.1.

**(2) Preparation of HS627-$(C1)_4$ drug conjugate**

HS627 antibody (10 mg/mL, 5 mL) was taken. A TCEP.HCl solution (2.86 mg/mL, 0.2 mL) was added and reacted at 25°C for 4 h. Compound **C1** (10 mg/mL, 0.3 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-$(C1)_n$** drug conjugate.

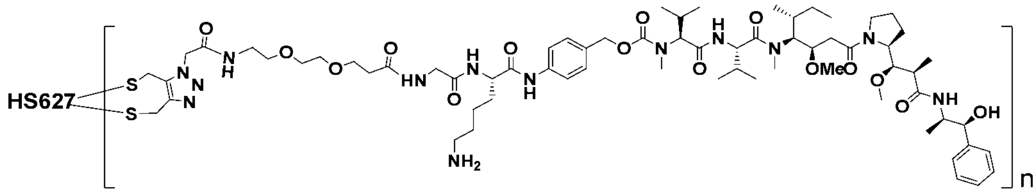

UV-HPLC, calculated DAR value: n = 3.9.

(3) **Preparation of $(C6)_4$-HS627-$(C1)_2$ dual-drug conjugate**

**(3.1)** Preparation of **C6**

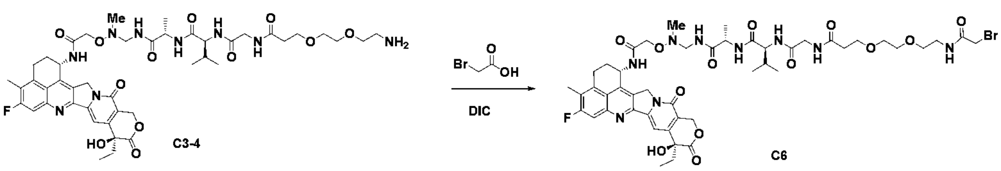


To a 10 mL single-necked flask were successively added DCM (3 mL), **C3-4** (32 mg, 0.034 mmol), **bromoacetic acid** (9.5 mg, 0.068 mmol), and DIC (8.6 mg, 0.068 mmol), which were reacted under stirring at room temperature for 90 min. The reaction mixture was concentrated and passed through a silica gel column (DCM:MeOH = 95:5) to give **C6** compound as a light yellow solid product (22 mg, yield 61%, HPLC 96%); $^1$H NMR(500 MHz, DMSO-$d_6$)δ 8.49(d, J=8.9 Hz, 1H), 8.33(t, J=5.7 Hz, 1H), 8.24(t, J=6.2 Hz, 1H), 8.11(t, J=5.8 Hz, 1H), 7.98(d, J=7.1 Hz, 1H), 7.75(d, J=8.5 Hz, 1H), 7.70(d, J=10.8 Hz, 1H), 7.28(s, 1H), 5.64-5.54(m, 1H), 5.40(s, 2H), 5.23(d, J=18.9 Hz, 1H), 5.04(d, J=18.9 Hz, 1H), 4.33-3.97(m,

6H), 3.85(s, 2H), 3.70(qd, J=16.5, 5.7 Hz, 3H), 3.57(t, J=6.5 Hz,4H), 3.52-3.37(m, 4H), 3.26-3.17(m, 4H), 3.14-3.06(m, 1H), 2.47(s, 3H), 2.41-2.28(m, 5H), 2.24-2.11(m, J=6.9, 6.0 Hz, 2H), 1.84(dh, J=20.8, 7.0 Hz, 3H), 1.14(d, J=7.1 Hz, 3H), 0.85(t, J=7.3 Hz, 3H), 0.70(dd, J=11.4, 6.8 Hz, 6H); LCMS: $[M+1]^+$ 1058.24 (theoretical value: 1057.36).

### (3.2) Preparation of $(C6)_4$-HS627-$(C1)_2$ dual-drug conjugate

The above **HS627-$(C1)_2$** drug-antibody conjugate (10 mg/mL, 2 mL) was taken. A TCEP.HCl solution (2.86 mg/mL, 0.08 mL) was added and reacted at 25°C for 4 h. **C6** compound (10 mg/mL, 0.15 mL) dissolved in DMA was added. The mixture was uniformly mixed and reacted at 25°C for 12 h. An NAC solution (10 mg/mL, 0.08 mL) was added for quenching. Small molecules were removed by an ultrafiltration membrane to give **$(C6)_m$-HS627-$(C1)_n$** drug-antibody conjugate.

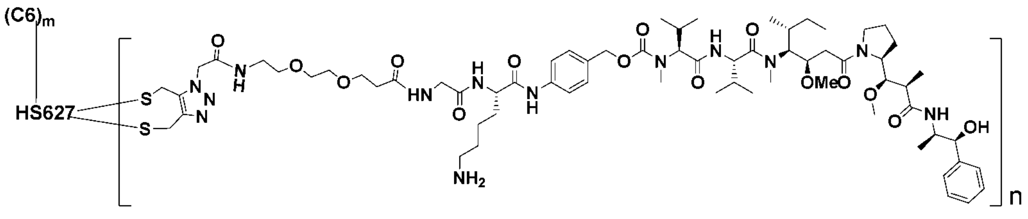

UV-HPLC, calculated DAR value: m+n = 5.9.

### Example 20: Conjugation of C2 to HS627 antibody and preparation of dual-drug ADC

### (1) Preparation of HS627-$(C2)_2$ drug conjugate

HS627 antibody (10 mg/mL, 5 mL) was taken. A TCEP.HCl solution (2.86 mg/mL, 0.133 mL) was added and reacted at 0°C for 3 h. **C2** (10 mg/mL, 0.17 mL) dissolved in DMA was added. The mixture was uniformly mixed and reacted at 25°C for 4 h. An NAC solution (10 mg/mL, 0.11 mL) was added for quenching. Small molecules were removed by an ultrafiltration membrane to give an **HS627-$(C2)_n$** drug-antibody conjugate.

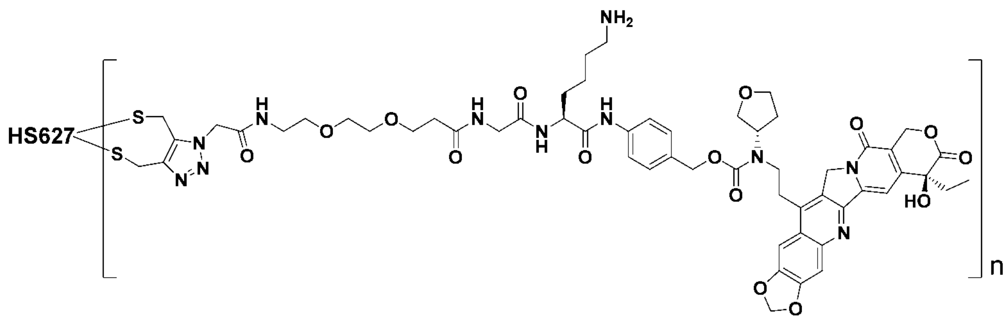

UV-HPLC, calculated DAR value: n = 1.9.

### (2) Preparation of HS627-$(C2)_4$ drug conjugate

HS627 antibody (10 mg/mL, 5 mL) was taken. A TCEP.HCl solution (2.86 mg/mL, 0.2 ml) was added and reacted at 25°C for 4 h. **C2** (10 mg/mL, 0.26 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-$(C2)_n$** drug-antibody conjugate.

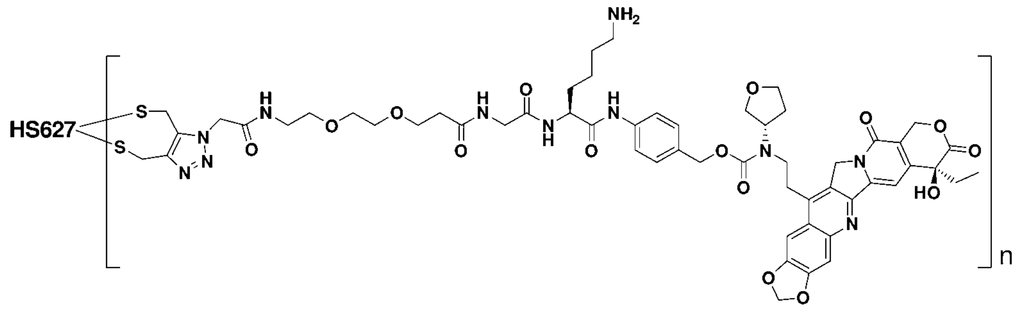

UV-HPLC, calculated DAR value: n = 3.8.

### (3) Preparation of $(CO)_4$-HS627-$(C2)_2$ dual-drug conjugate

The above **HS627-(C2)$_2$** (10 mg/mL, 2 mL) was taken. A TCEP.HCl solution (2.86 mg/mL, 0.08 mL) was added and reacted at 25°C for 4 h. **C0 (mc-VC-PABC-MMAE)** compound (10 mg/mL, 0.18mL) dissolved in DMA was added to the above solution system. The mixture was uniformly mixed and reacted at 25°C for 4 h. An NAC solution (10 mg/mL, 0.08 mL) was added for quenching. Small molecules were removed by an ultrafiltration membrane to give an **(CO)$_m$-HS627-(C2)$_n$** drug-antibody conjugate.

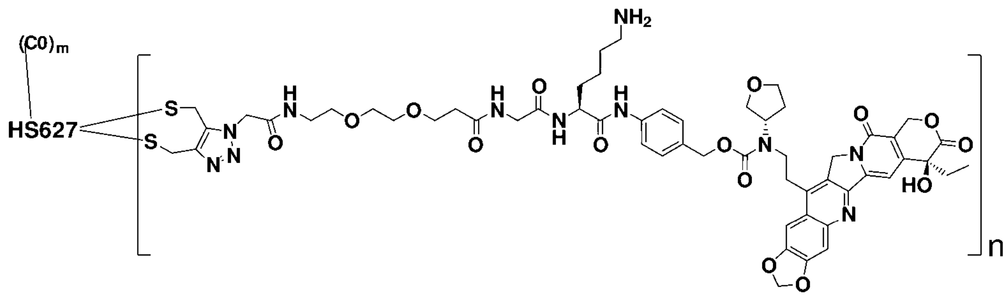

wherein, the structural formula of me-VC-PABC-MMAE was as follows:

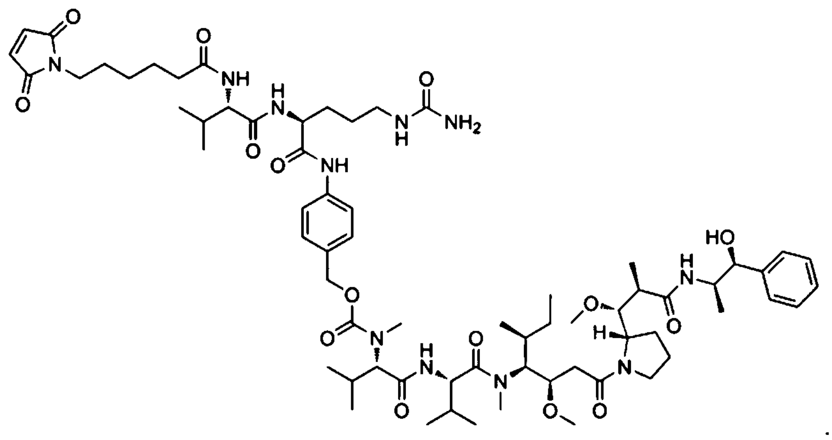

UV-HPLC, calculated DAR value: m+n = 5.7.

### Example 21: Conjugation of C3 to HS627 antibody and preparation of dual-drug ADC

### (1) Preparation of HS627-$(C3)_2$ drug conjugate

HS627 antibody (10 mg/mL, 5 mL) was taken. A TCEP.HCl solution (2.86 mg/mL, 0.133 mL) was added and reacted at 0°C for 3 h. **C3** (10 mg/mL, 0.16 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-(C3)$_n$** drug conjugate.

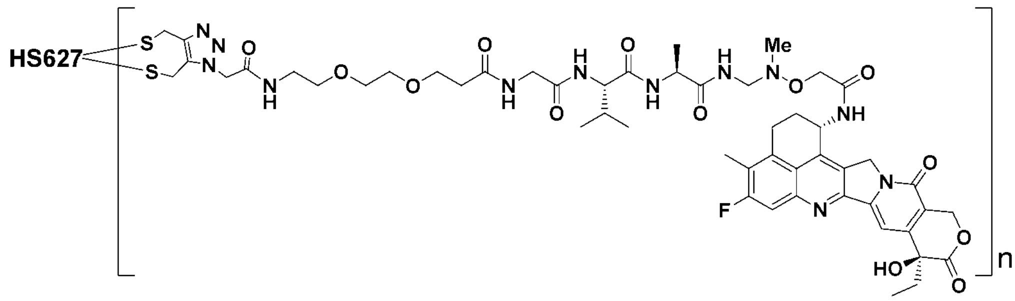

UV-HPLC, calculated DAR value: n = 2.1.

**(2) Preparation of HS627-(C3)$_4$ drug conjugate**

HS627 antibody (10 mg/mL, 5 mL) was taken. A TCEP.HCl solution (2.86 mg/mL, 0.2 mL) was added and reacted at 25°C for 4 h. **C3** (10 mg/mL, 0.25 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-(C3)$_n$** drug conjugate.

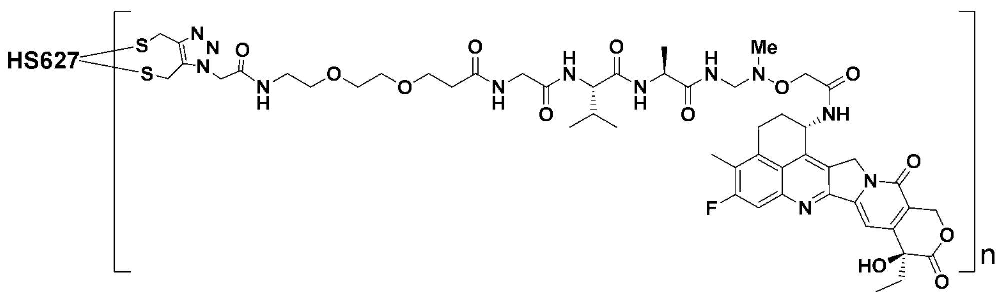

UV-HPLC, calculated DAR value: n = 3.8.

(3) **Preparation of (CO)$_4$-HS627-(C3)$_2$ dual-drug conjugate**

The above **HS627-(C3)$_2$** drug conjugate (10 mg/mL, 2 mL) was taken. A TCEP.HCl solution (2.86 mg/mL, 0.08 mL) was added and reacted at 25°C for 4 h. **C0** compound (10 mg/mL, 0.18 mL) dissolved in DMA was added to the above solution system. The mixture was uniformly mixed and reacted at 25°C for 4 h. An NAC solution (10 mg/mL, 0.08 mL) was added for quenching. Small molecules were removed by an ultrafiltration membrane to give an **(C0)$_m$-HS627-(C3)$_n$** drug conjugate.

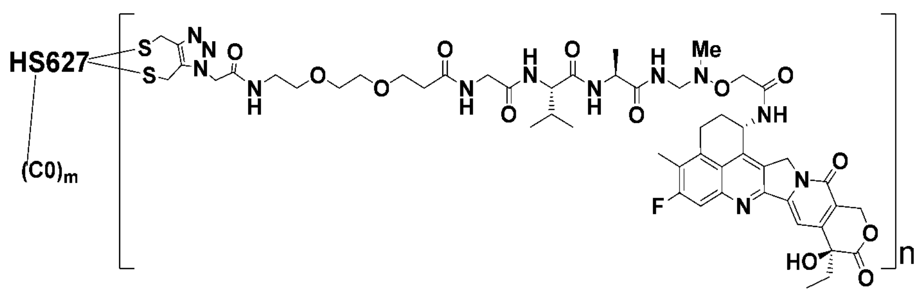

UV-HPLC, calculated DAR value: m+n = 5.8.

**Example 22: Conjugation of C4 to HS627 antibody and preparation of dual-drug ADC**

**(1) Preparation of HS627-(C4)$_2$ drug conjugate**

HS627 antibody (10 mg/mL, 5 mL) was taken. A TCEP.HCl solution (2.86 mg/mL, 0.133 mL) was added and reacted at 0°C for 3 h. **C4** compound (10 mg/mL, 0.2 mL) dissolved in DMA was added to the above solution system. The

mixture was uniformly mixed and reacted at 25°C for 4 h. An NAC solution (10 mg/mL, 0.11 mL) was added for quenching. Small molecules were removed by an ultrafiltration membrane to give an **HS627-(C4)$_n$** drug conjugate.

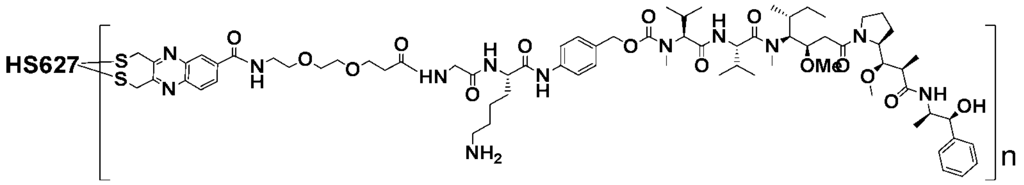

UV-HPLC, calculated DAR value: n = 1.9.

**(2) Preparation of HS627-(C4)$_4$ drug conjugate**

HS627 antibody (10 mg/mL, 5 mL) was taken. A TCEP.HCl solution (2.86 mg/mL, 0.2 mL) was added and reacted at 25°C for 4 h. C4 (10 mg/mL, 0.32 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-(C4)$_n$** drug conjugate.

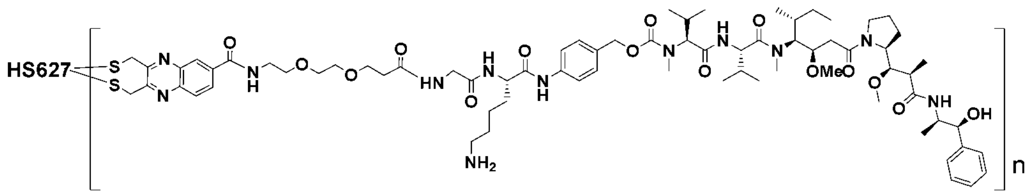

UV-HPLC, calculated DAR value: n = 3.7.

**(3) Preparation of (C$_6$)$_4$-HS627-(C4)$_2$ dual-drug conjugate**

The above **HS627-(C4)$_2$** (10 mg/mL, 2 mL) was taken. A TCEP.HCl solution (2.86 mg/mL, 0.08 mL) was added and reacted at 25°C for 4 h. **C6** compound (10 mg/mL, 0.15 mL) dissolved in DMA was added. The mixture was uniformly mixed and reacted at 25°C for 12 h. An NAC solution (10 mg/mL, 0.08 mL) was added for quenching. Small molecules were removed by an ultrafiltration membrane to give an **(C6)$_m$-HS627-(C4)$_n$** drug conjugate.

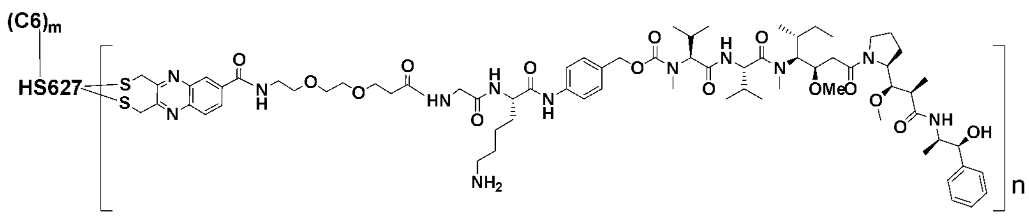

UV-HPLC, calculated DAR value: m+n = 5.7.

**Example 23: Conjugation of C5 to HS627 antibody and preparation of dual-drug ADC**

**(1) Preparation of HS627-(C5)$_2$ drug conjugate**

HS627 antibody (10 mg/mL, 5 mL) was taken. A TCEP.HCl solution (2.86 mg/mL, 0.133 mL) was added and reacted at 0°C for 3 h. **C5** (10 mg/mL, 0.17 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-(C5)$_n$** drug conjugate.

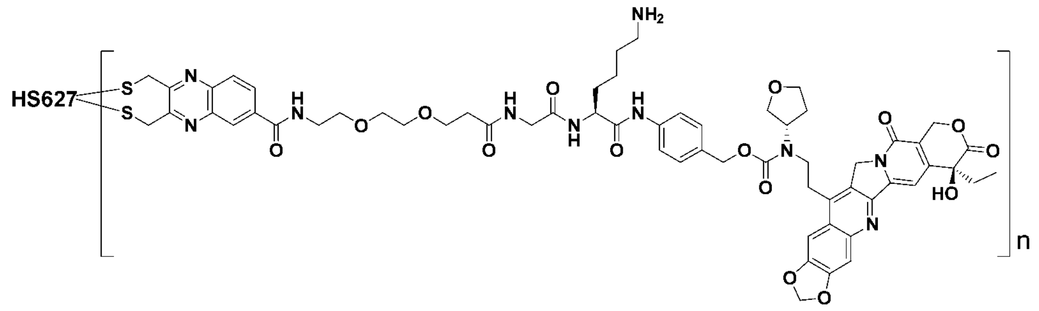

UV-HPLC, calculated DAR value: n = 2.0.

**(3) Preparation of HS627-(C5$_4$ drug conjugate**

HS627 antibody (10 mg/mL, 5 mL) was taken. A TCEP.HCl solution (2.86 mg/mL, 0.2 mL) was added and reacted at 25°C for 4 h. **C5** (10 mg/mL, 0.28 mL) dissolved in DMA was added to the above solution system. The mixture was uniformly mixed and reacted at 25°C for 4 h. An NAC solution (10 mg/mL, 0.11 mL) was added for quenching. Small molecules were removed by an ultrafiltration membrane to give an **HS627-(C5)$_n$** drug conjugate.

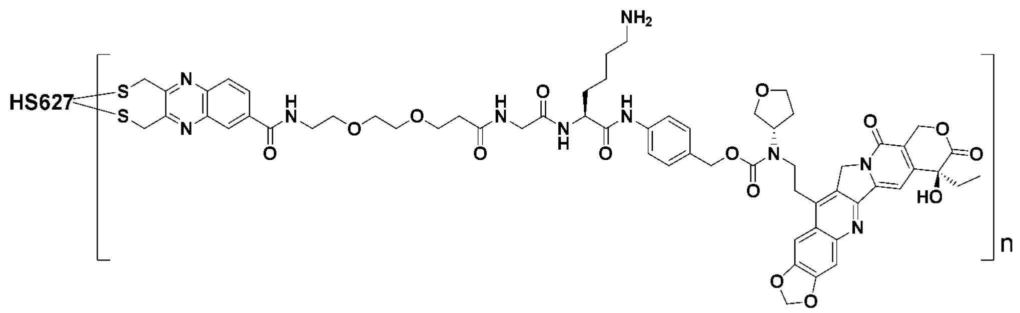

UV-HPLC, calculated ADR value: n = 3.9.

**(4) Preparation of (CO)$_4$-HS627-(C5)$_2$ dual-drug conjugate**

The above **HS627-(C5)$_2$** (10 mg/mL, 2 mL) was taken. A TCEP.HCl solution (2.86 mg/mL, 0.08 mL) was added and reacted at 25°C for 4 h. **C0** compound (10 mg/mL, 0.18 mL) dissolved in DMA was added. The mixture was uniformly mixed and reacted at 25°C for 4 h. An NAC solution (10 mg/mL, 0.08 mL) was added for quenching. Small molecules were removed by an ultrafiltration membrane to give an **(CO)$_m$-HS627-(C5)$_n$** drug conjugate.

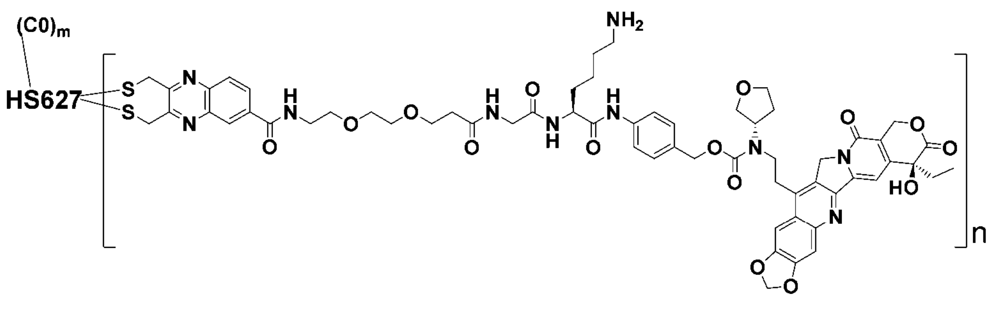

UV-HPLC, calculated DAR value: m+n = 5.8.

**Example 24: Conjugation of C6 to HS627 antibody and preparation of dual-drug ADC**

**(1) Preparation of HS627-(C6)$_2$ drug conjugate**

HS627 antibody (10 mg/mL, 5 mL) was taken. A TCEP.HCl solution (2.86 mg/mL, 0.133 mL) was added and reacted at 0°C for 3 h. **C6** (10 mg/mL, 0.16 mL) dissolved in DMA was added to the above solution system. The mixture was

uniformly mixed and reacted at 25°C for 4 h. An NAC aqueous solution (10 mg/mL, 0.11 mL) was added for quenching. Small molecules were removed by an ultrafiltration membrane to give an **HS627-(C6)$_n$** drug conjugate.

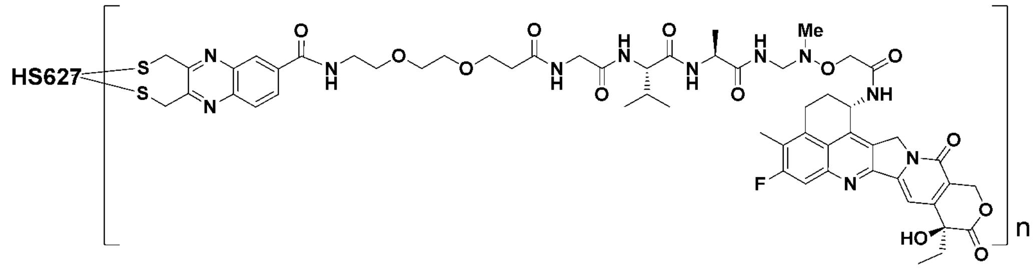

UV-HPLC, calculated DAR value: n = 1.9.

**(2) Preparation of HS627-(C6)$_4$ drug conjugate**

HS627 antibody (10 mg/mL, 5 mL) was taken. A TCEP.HCl solution (2.86 mg/mL, 0.2 mL) was added and reacted at 25°C for 4 h. C6 (10 mg/mL, 0.27 mL) dissolved in DMA was added to the above solution system. The mixture was uniformly mixed and reacted at 25°C for 4 h. An NAC aqueous solution (10 mg/mL, 0.11 mL) was added for quenching. Small molecules were removed by an ultrafiltration membrane to give an **HS627-(C6)$_n$** drug conjugate.

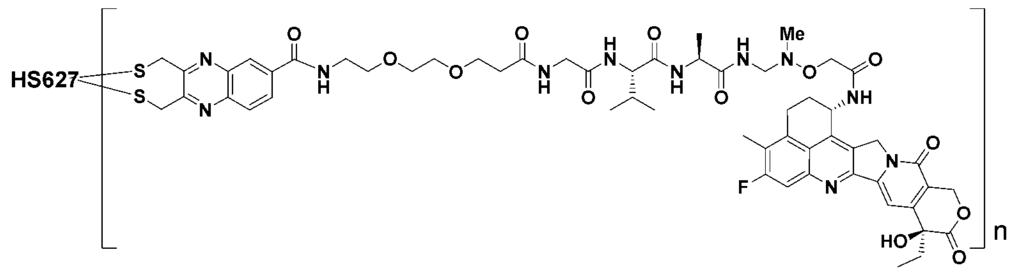

UV-HPLC, calculated DAR value: n = 3.8.

**(3) Preparation of (C0)$_4$-HS627-(C6)$_2$ dual-drug conjugate**

The above **HS627-(C6)$_2$** (10 mg/mL, 2 mL) was taken. A TCEP.HCl solution (2.86 mg/mL, 0.08 mL) was added and reacted at 25°C for 4 h. **C0** compound (10 mg/mL, 0.18 mL) dissolved in DMA was added to the above solution system. The mixture was uniformly mixed and reacted at 25°C for 4 h. An NAC solution (10 mg/mL, 0.08 mL) was added for quenching. Small molecules were removed by an ultrafiltration membrane to give an **(C0)$_m$-HS627-(C6)$_n$** drug conjugate.

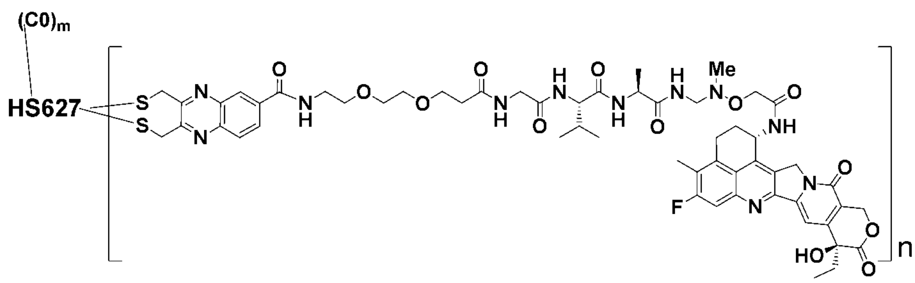

UV-HPLC, calculated DAR value: m+n = 5.9.

**Example 25: Conjugation of C7 to HS627 antibody and preparation of dual-drug ADC**

**(1) Preparation of HS627-(C7)$_2$ drug conjugate**

HS627 antibody (10 mg/mL, 5 mL) was taken. A TCEP.HCl solution (2.86 mg/mL, 0.133 mL) was added and reacted at 10°C for 3 h. **C7** (10 mg/mL, 0.20 mL) dissolved in DMA was added to the above solution system. The mixture

was uniformly mixed and reacted at 25°C for 4 h. After the reaction was complete, an NAC solution (10 mg/mL, 0.11 mL) was added for quenching. Small molecules were then removed by an ultrafiltration membrane to finally give an **HS627-(C7)$_n$** drug conjugate.

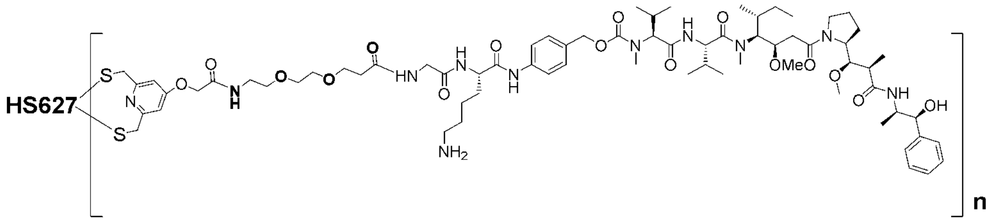

UV-HPLC, calculated average DAR value: n = 2.0.

**(2) Preparation of HS627-(C7)$_4$ drug conjugate**

HS627 antibody (10 mg/mL, 5 mL) was taken. A TCEP.HCl solution (2.86 mg/mL, 0.2 mL) was added and reacted at 25°C for 4 h. **C7** compound (10 mg/mL, 0.33 mL) dissolved in DMA was added to the above solution system. The mixture was uniformly mixed and reacted at 25°C for 4 h. After the reaction was complete, an NAC solution (10 mg/mL, 0.11 mL) was added for quenching. Small molecules were then removed by an ultrafiltration membrane to finally give an **HS627-(C7)$_n$** drug conjugate.

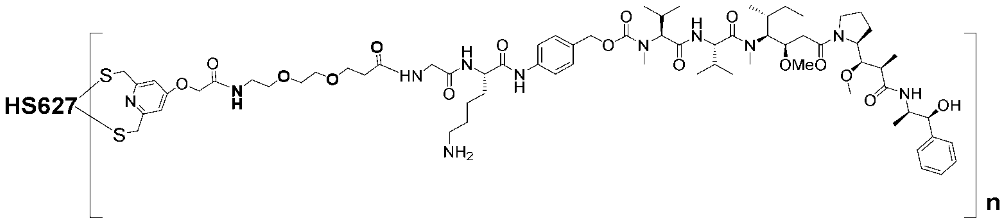

UV-HPLC, calculated average DAR value: n = 4.1.

**(3) Preparation of (C6)$_4$-HS627-(C7)$_2$ drug conjugate**

The above **HS627-(C7)$_2$** drug conjugate (10 mg/mL, 2 mL) was taken. A TCEP.HCl solution (2.86 mg/mL, 0.08 mL) was added and reacted at 25°C for 4 h. **C6** compound (10 mg/mL, 0.15 mL) dissolved in DMA was added. The mixture was uniformly mixed and reacted at 25°C for 12 h. An NAC solution (10 mg/mL, 0.08 mL) was added for quenching. Small molecules were removed by an ultrafiltration membrane to give a DAR (2+4) product **(C6)$_m$-HS627-(C7)$_n$** drug conjugate.

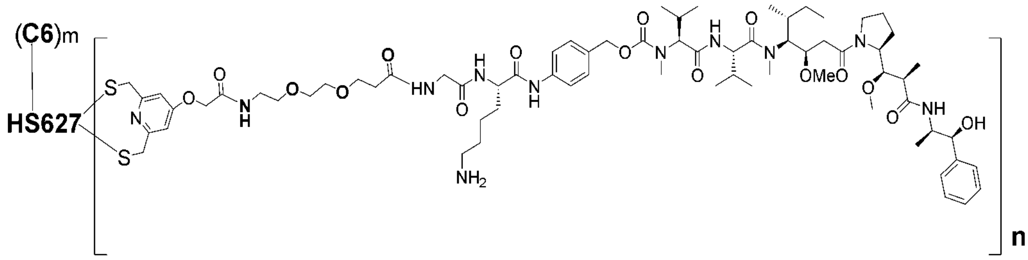

UV-HPLC, calculated average DAR value: m+n = 5.9.

**Example 26: Conjugation of C8 to HS627 antibody and preparation of dual-drug ADC**

**(1) Preparation of HS627-(C8)$_2$ drug conjugate**

HS627 antibody (10 mg/mL, 5 mL) was taken. A TCEP.HCl solution (2.86 mg/mL, 0.133 mL) was added and reacted at 10°C for 3 h. **C8** (10 mg/mL, 0.16 mL) dissolved in DMA was added to the above solution system. The mixture was uniformly mixed and reacted at 25°C for 4 h. After the reaction was complete, an NAC solution (10 mg/mL, 0.11 mL)

was added for quenching. Small molecules were then removed by an ultrafiltration membrane to finally give an **HS627-(C8)$_n$** drug conjugate.

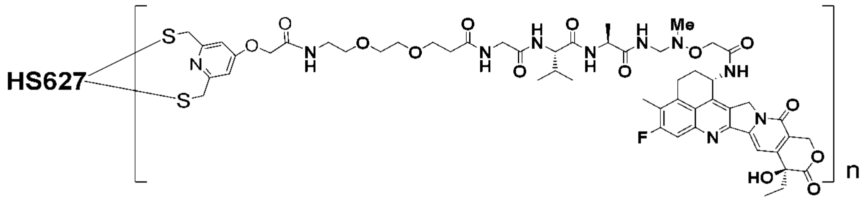

UV-HPLC, calculated average DAR value: n = 1.9.

**(2) Preparation of HS627-(C8)$_4$ drug conjugate**

HS627 antibody (10 mg/mL, 5 mL) was taken. A TCEP.HCl solution (2.86 mg/mL, 0.2 mL) was added and reacted at 25°C for 4 h. **C8** (10 mg/mL, 0.27 mL) dissolved in DMA was added to the above solution system. The mixture was uniformly mixed and reacted at 25°C for 4 h. After the reaction was complete, an NAC solution (10 mg/mL, 0.11 mL) was added for quenching. Small molecules were then removed by an ultrafiltration membrane to finally give an **HS627-(C8)$_n$** drug conjugate.

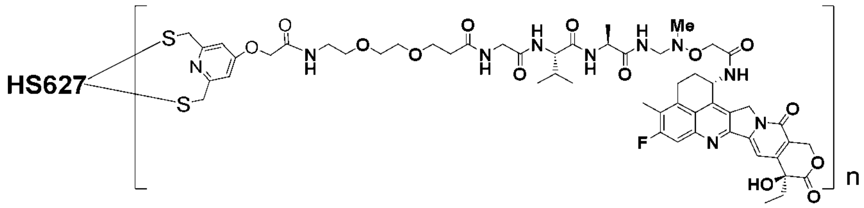

UV-HPLC, calculated average DAR value: n = 4.1.

**(3) Preparation of (C0)$_4$-HS627-(C8)$_2$ drug conjugate**

The above **HS627-(C8)$_2$** drug conjugate (10 mg/mL, 2 mL) was taken. A TCEP.HCl solution (2.86 mg/mL, 0.08 mL) was added and reacted at 25°C for 4 h. **C0** (MC-VC-PAB-MMAE) compound (10 mg/mL, 0.18 mL) dissolved in DMA was added to the above solution system. The mixture was uniformly mixed and reacted at 25°C for 4 h. After the reaction was complete, an NAC solution (10 mg/mL, 0.08 mL) was added for quenching. Small molecules were then removed by an ultrafiltration membrane to finally give a **(CO)$_m$-HS627-(C8)$_n$** drug conjugate.

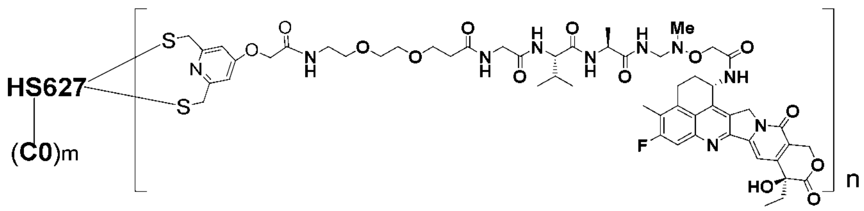

UV-HPLC, calculated average DAR value: m+n = 6.1.

**Effect Example 1: DAR value of ADC**

The measured DAR values of the ADCs prepared in the examples are shown in Table 1 below.

Table 1. Measured DAR results of ADC compounds involved in the examples of the present disclosure

| Entry | ADC | Measured DAR value | | |
|---|---|---|---|---|
| | | DAR2 | DAR4 | DAR2+4 |
| 1 | HS627-(C1)$_2$ | 2.1 | - | - |

(continued)

| Entry | ADC | Measured DAR value | | |
|---|---|---|---|---|
| | | DAR2 | DAR4 | DAR2+4 |
| 2 | HS627-$(C1)_4$ | - | 3.9 | - |
| 3 | $(C6)_4$-HS627-$(C1)_2$ | - | - | 5.9 |
| 4 | HS627-$(C2)_2$ | 1.9 | - | - |
| 5 | HS627-$(C2)_4$ | - | 3.8 | - |
| 6 | $(C0)_4$-HS627-$(C2)_2$ | - | - | 5.7 |
| 7 | HS627-$(C3)_2$ | 2.1 | - | - |
| 8 | HS627-$(C3)_4$ | - | 3.8 | - |
| 9 | $(C0)_4$-HS627-$(C3)_2$ | - | - | 5.8 |
| 10 | HS627-$(C4)_2$ | 1.9 | - | - |
| 11 | HS627-$(C4)_4$ | - | 3.7 | - |
| 12 | $(C6)_4$-HS627-$(C4)_2$ | - | - | 5.7 |
| 13 | HS627-$(C5)_2$ | 2.0 | - | - |
| 14 | HS627-$(C5)_4$ | - | 3.9 | - |
| 15 | $(C0)_4$-HS627-$(C5)_2$ | - | - | 5.8 |
| 16 | HS627-$(C6)_2$ | 1.9 | - | - |
| 17 | HS627-$(C6)_4$ | - | 3.8 | - |
| 18 | $(C0)_4$-HS627-$(C6)_2$ | - | - | 5.9 |
| 19 | HS627-$(C7)_2$ | 2.0 | - | - |
| 20 | HS627-$(C7)_4$ | - | 4.1 | - |
| 21 | $(C6)_4$-HS627-$(C7)_2$ | - | - | 5.9 |
| 22 | HS627-$(C8)_2$ | 1.9 | - | - |
| 23 | HS627-$(C8)_4$ | - | 4.1 | - |
| 24 | $(C0)_4$-HS627-$(C8)_2$ | - | - | 6.1 |

**Effect Example 2: Inhibition of tumor growth activity *in vivo* by ADCs**

Testing method for *in vitro* inhibitory activity of ADCs:

Human pancreatic cancer CFPAC-1 cells with low Her2 expression and breast cancer MCE-7 cells were subjected to monolayer culturing *in vitro.* When cell saturation reached 80%-90%, the cells were digested with trypsin-EDTA and centrifuged. The supernatant was discarded. The cells were re-suspended in PBS. The cell suspension was adjusted to an appropriate concentration. The cells (2-10 $\times$ $10^6$ cells / 0.1 mL) were subcutaneously inoculated into BALB/c nude mice. The animals and the growth of transplanted tumors were observed regularly. When the tumor volume reached approximately 100-200 $mm^3$, the mice were randomly divided into groups based on tumor volume and body weight. The groups included a vehicle control group (or Vehicle group, injected with normal saline) and ADC administration groups (dissolved in normal saline), with 6 animals per group. The animals were administered via intravenous injection with an administration frequency of Q7D. A total of 2 doses were administered (the time of the first administration was denoted Day 1, and the second administration was conducted on Day 7, with the administered dose being 2 mg/kg or 5 mg/kg). Twice a week, the longer diameter a (mm) and shorter diameter b (mm) of the tumor were measured with a vernier caliper and the body weight of the mice were measured. The tumor volume (V) was calculated according to the following formula: $V = 1/2 \times a \times b^2$ ($mm^3$), in which a and b represented the length and width of the tumor, respectively. A tumor growth curve was plotted. Finally, the tumor was excised and weighed. Statistical analysis was performed based on the tumor volume and body weight data of tumor-bearing mice at the end of the experiment by using GraphPad Prism software to obtain tumor inhibition results (FIGS. 1, 2, 3, and 4). HS627-Dxd was used as a control, and the structural formula thereof was as below:

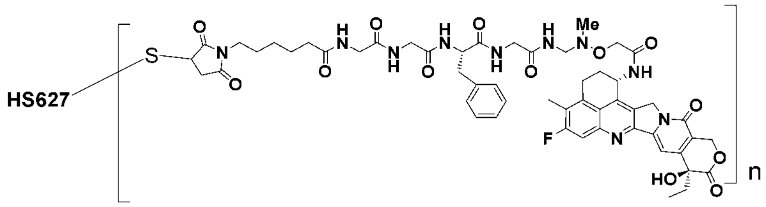

n = 8. The primary evaluation indicator was:
tumor growth inhibition (TGI):

$$TGI\ (\%) = [1-(avT_{i-0}/avC_{i-0})]\times 100\%,$$

in which $avT_{i-0}$ was the average tumor volume of the administration group on a specified day minus the average tumor volume of the administration group on the day of starting administration, and avCi-0 was the average tumor volume of the vehicle control group on the specified day minus the average tumor volume of the vehicle control group on the day of starting administration.

Table 2. Grouping and administration regimen

| Tumor model | | Administered drug | Administered dose | Administration time | Tumor inhibition |
|---|---|---|---|---|---|
| CFPAC-1 | Normal saline | Normal saline | - | Day0, Day7 | - |
| | Positive control | HS627-Dxd (DAR8) | 1 mg/kg | | 42.75% |
| | | | 5 mg/kg | | 95.56% |
| | Experimental group | Entry5 (HS627-$(C2)_4$) | 2 mg/kg | | 72.82% |
| | | | 5 mg/kg | | 96.97% |
| | | Entry8 (HS627-$(C3)_4$) | 2 mg/kg | | 78.17% |
| | | | 5 mg/kg | | 95.69% |
| | | Entry 17 (HS627-$(C6)_4$) | 2 mg/kg | | 66.90% |
| | | | 5 mg/kg | | 90.21% |
| MCF-7 | Normal saline | Normal saline | - | Day0, Day7 | - |
| | Positive control | HS627-Dxd (DAR8) | 2 mg/kg | | 41.39% |
| | | | 5 mg/kg | | 79.07% |
| | Experimental group | Entry18 ($(C0)_4$-HS627-$(C6)_2$) | 2 mg/kg | | 54.06% |
| | | | 5 mg/kg | | 83.84% |
| | | Entry2 (HS627-$(C1)_4$) | 2 mg/kg | | 23.55% |
| | | | 5 mg/kg | | 39.21% |
| | | Entry24 ($(C0)_4$-HS627-$(C8)_2$) | 2 mg/kg | | 37.07% |
| | | | 5 mg/kg | | 95.86% |

The tumor inhibition was calculated based on the tumor volume on Day 42 for the CFPAC-1 model and on the tumor volume on Day 35 for the MCF-7 model.

As can be seen from the results, the exemplary ADC compounds Entry2 (DAR2), Entry5 (DAR4), Entry8 (DAR4), Entry17 (DAR4), Entry18 (DAR2+4), and Entry24 (DAR2+4) of the present disclosure all demonstrated better tumor inhibition effects in pancreatic and breast cancer tumor models.

## Claims

1. A compound represented by formula (I) or a pharmaceutically acceptable salt, stereoisomer, or prodrug thereof:

M-L-D (I)

wherein,

M is a bifunctional linker linked to an antibody or an antigen-binding fragment thereof;
L is a linker between the bifunctional linker M and D;
D is a cytotoxic drug moiety;
said M represents a structure represented by formula (II) below:

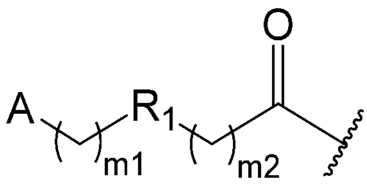
(II)

in formula (II), A is selected from alkenyl, a monocyclic group, or a fused ring group, wherein the monocyclic group is aryl or heteroaryl, and the fused ring group is formed by fusing heteroaryl to a group selected from aryl and heteroaryl; and $R_1$ represents a single bond, O, N, S, or alkylene; said A is optionally substituted with one or more substituents selected from X, wherein X is selected from halogen, halogen-substituted or unsubstituted alkyl, $-NH-S(O)_2$-alkenyl, or $-C_1-C_6$ heteroalkyl-heterocycloalkyl, the heterocycloalkyl being optionally substituted with one or more halogen-substituted alkyl or -C(O)-halogen-substituted alkyl;
in formula (II), m1 and m2 are each independently selected from an integer from 0 to 10; preferably, A is selected from $C_2-C_6$ alkenyl, a monocyclic group, or a fused ring group, wherein the monocyclic group is 6- to 12-membered aryl or 5- to 12-membered heteroaryl, the 5- to 12-membered heteroaryl containing 1-3 nitrogen atoms; and the fused ring group is a ring formed by fusing 5- to 12-membered heteroaryl to a group selected from 6- to 12-membered aryl and 5- to 12-membered heteroaryl, the 5- to 12-membered heteroaryl containing 1-3 nitrogen atoms as ring atoms;
preferably, A is selected from a monocyclic group or a fused ring group, wherein the monocyclic group is a 6-membered aromatic ring, a 5-membered heteroaromatic ring, or a 6-membered heteroaromatic ring, and the fused ring group is formed by fusing 6-membered heteroaryl to 6-membered aryl, the 5-membered heteroaryl or 6-membered heteroaryl containing 1-3 nitrogen atoms;
preferably, A is selected from vinyl,

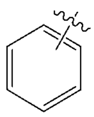 , 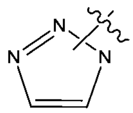 , 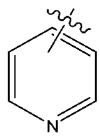 , 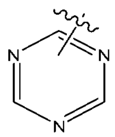

or

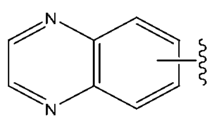 ;

preferably, X represents halogen, halogen-substituted $C_1-C_6$ alkyl, $-NH-S(O)_2-C_2-C_6$ alkenyl, or $-C_1-C_6$ heteroalkyl-3- to 8-membered heterocycloalkyl, the 3- to 8-membered heterocycloalkyl being optionally substituted with one or more halogen-substituted $C_1-C_6$ alkyl or -C(O)-halogen-substituted $C_1-C_6$ alkyl;
preferably, M is selected from

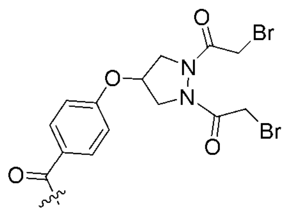 , 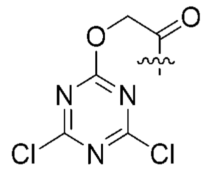 , 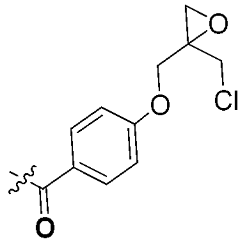 , 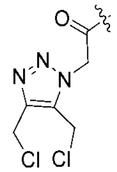 , 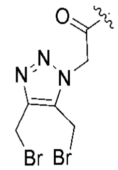 ,

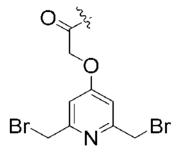 , 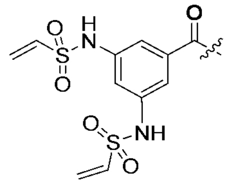 , 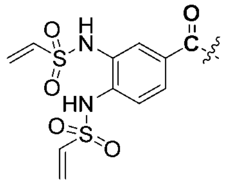 , 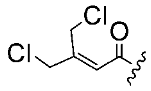 , 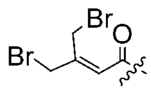

or

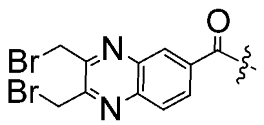 ;

preferably, L is selected from a divalent structure composed of one or more of: Val-Cit, Val-Ala, Val-Lys, Val-Lys(Ac), Phe-Lys, Phe-Lys(Ac), Gly -Lys, Gly-Phe, Gly-Leu, Gly -Gly- Lys, Gly-Val-Ala, Gly-Val- Cit, Gly-Phe-Gly, Val-Ala-Gly, D-Val-Leu-Lys, Gly-Gly-Arg, Ala-Ala-Asn, Ala-Ala-Ala, Val-Lys-Ala, Gly-Gly-Gly, Gly-Leu-Gly, Gly-Gly-Phe-Gly, Gly-Gly-Leu-Gly, Gly-Val-Ala-Gly, Gly-Val-Cit-Gly, and Gly-Gly-Gly-Gly-Gly;
preferably, L is selected from Val-Cit, Val-Ala, Gly-Gly-Lys, Gly-Phe-Gly, Gly-Lys, Gly-Phe, Gly-Leu, Val-Ala-Gly, Gly-Gly-Phe-Gly, Gly-Val-Ala-Gly, Ala-Ala-Asn, and Gly-Leu-Gly; and preferably, L is selected from Gly-Lys and Gly-Val-Ala.

**2.** The compound or the pharmaceutically acceptable salt, stereoisomer, or prodrug thereof according to claim 1, wherein D is selected from at least one of an anti-tubulin agent, a DNA intercalator, a DNA topoisomerase inhibitor, a DNA synthesis inhibitor, an RNA polymerase inhibitor, and a Spliceosome inhibitor, preferably camptothecin or a camptothecin derivative, a pyrrolobenzodiazepine (PBD) dimer, methylauristatin E, or methylauristatin F.

**3.** The compound or the pharmaceutically acceptable salt, stereoisomer, or prodrug thereof according to claim 1 or 2, wherein said L is directly linked to D, or
a spacer unit E is linked between said L and said D, the spacer unit E being selected from p-aminobenzyl carbamate, bis(p-aminobenzyl) carbonate, aminomethyl ether, and methylenediamine.

**4.** The compound or the pharmaceutically acceptable salt, stereoisomer, or prodrug thereof according to any one of claims 1 to 3, wherein said M is directly linked to L, or
a spacer unit F is linked between said M and said L, the spacer unit F being selected from $-NH-(CH_2CH_2-O)_q-CH_2CH_2-C(=O)-$, wherein q represents an integer from 1 to 6.

**5.** The compound or the pharmaceutically acceptable salt, stereoisomer, or prodrug thereof according to any one of claims 1 to 4, wherein the compound is selected from the group consisting of:

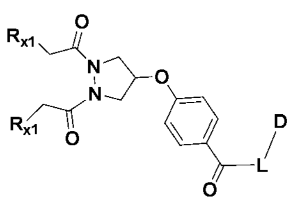 , 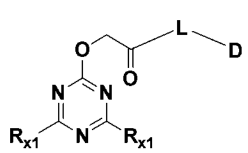 , 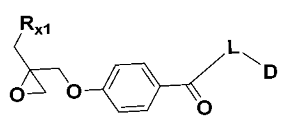 , 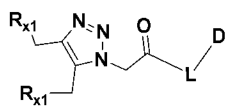 ,

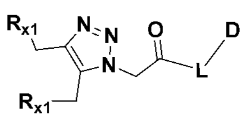 , 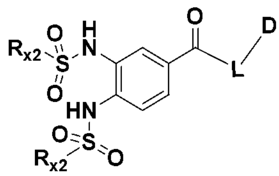 , 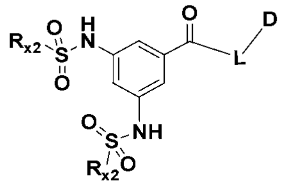 , 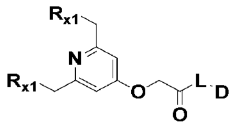 ,

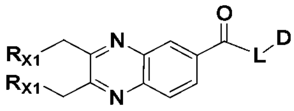 , and 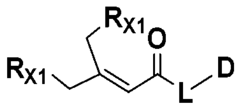 ,

wherein, L and D are as previously defined;
$R_{X1}$ and $R_{X2}$ are each independently selected from halogen or $C_2$-$C_6$ alkenyl;
preferably, $R_{X1}$ is selected from bromine or chlorine, and $R_{X2}$ is vinyl;
preferably, the compound is selected from the group consisting of:

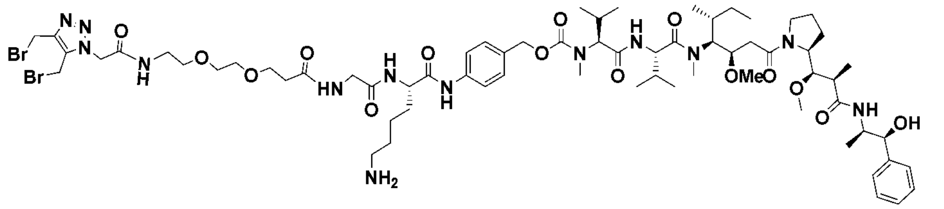

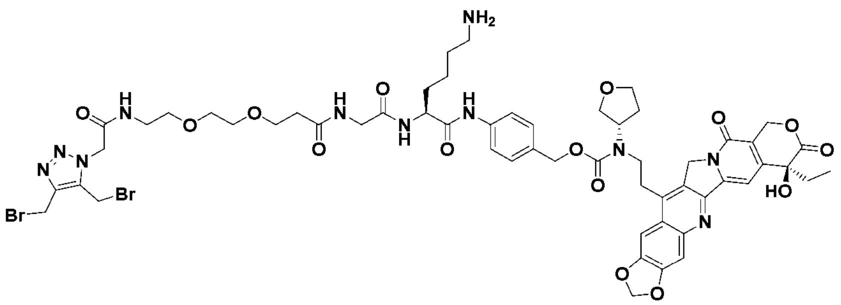

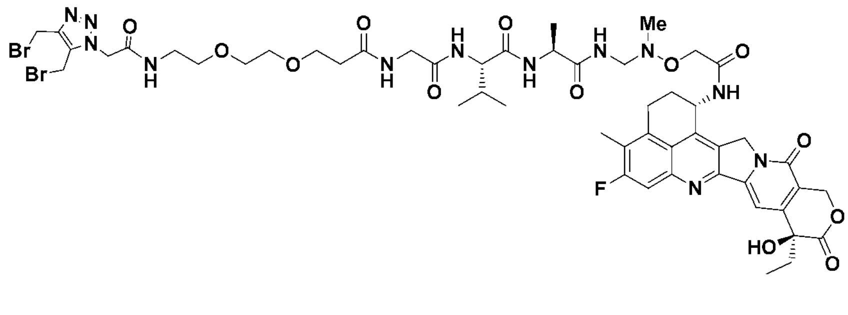

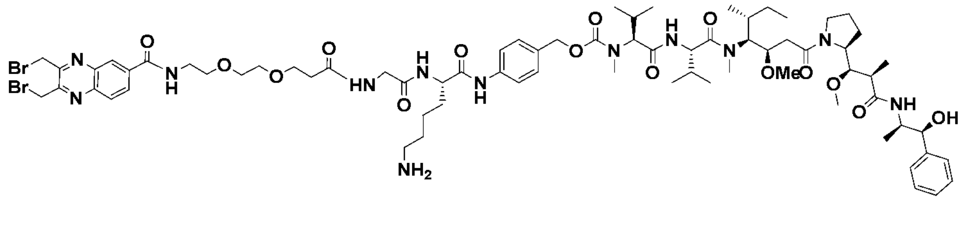

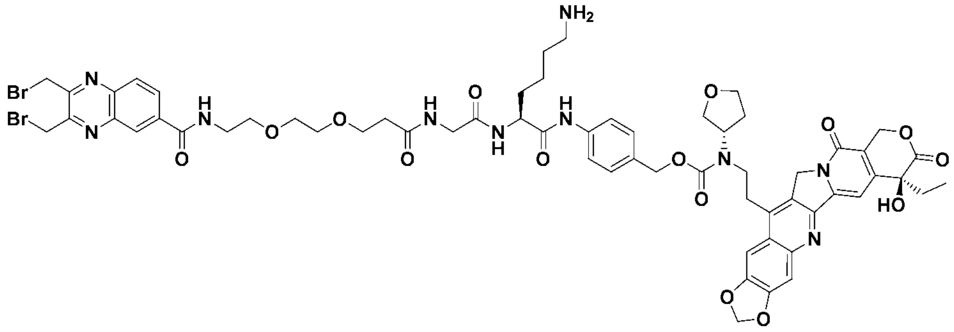

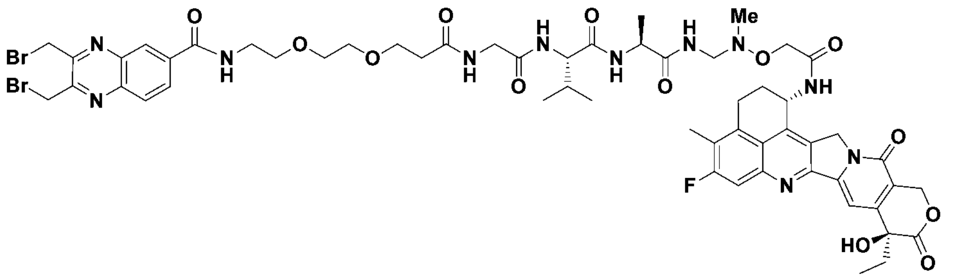

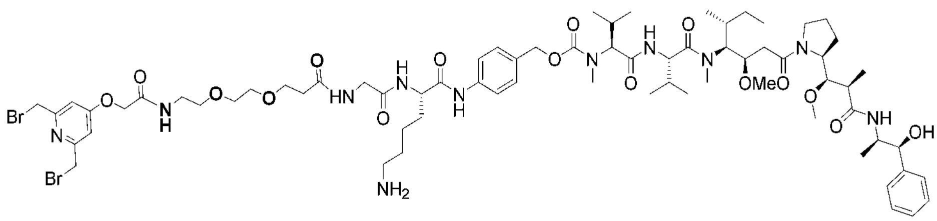

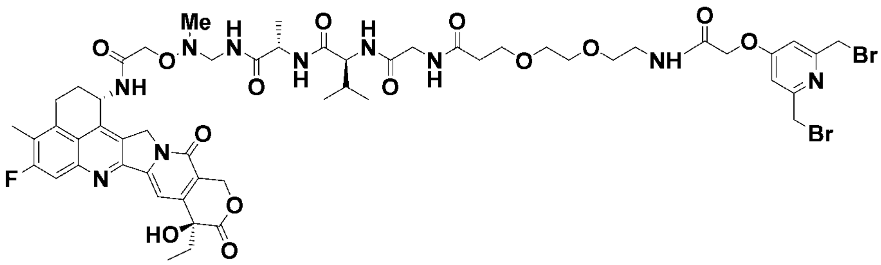

.

**6.** A drug-antibody conjugate represented by formula (III) or a pharmaceutically acceptable salt thereof:

Ab-M-L-D (III)

wherein, M, L, and D are each as defined for the compound of formula (I); and Ab is an antibody or an antigen-binding fragment thereof;
preferably, the Ab antibody is an antibody against a tumor-associated antigen;
preferably, the Ab is an anti-Her2 antibody;
preferably, the Ab is pertuzumab;
preferably, the drug-antibody conjugate compound is further linked to an additional drug having cytotoxicity;
preferably, the drug-antibody conjugate compound has a structure represented by formula (IV):

D'-L'-Ab-M-L-D (IV)

wherein, said L' is a linker between the antibody and D', and is the same as or different from L;
D' is a cytotoxic drug moiety that is the same as or different from D;
preferably, the drug-antibody conjugate represented by formula (III) or the pharmaceutically acceptable salt thereof is the following drug-antibody conjugate compounds:

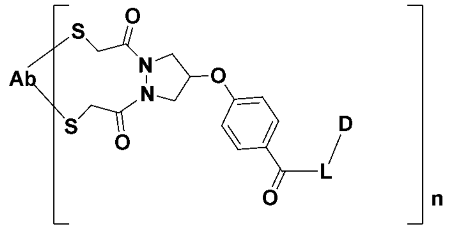 , 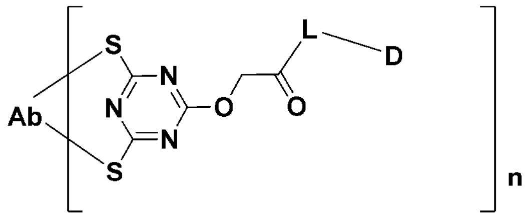 ,

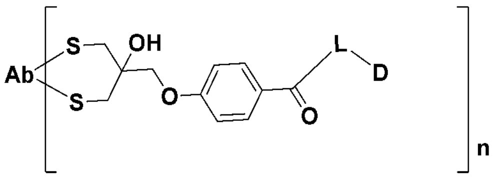 , 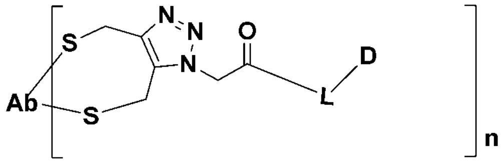 ,

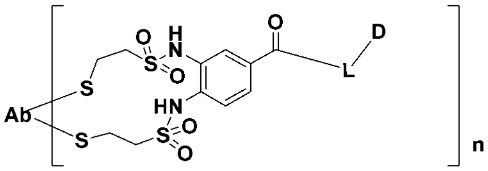 , 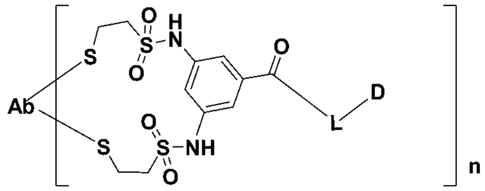 ,

and

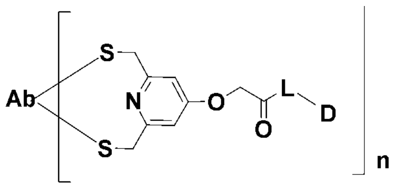 ;

wherein, L and D are each as defined for the compound of formula (I), and Ab is an antibody or an antigen-binding fragment thereof;
n is an integer selected from 1 to 12, preferably an integer selected from 2 to 4; preferably, the antibody is a monoclonal antibody or a polyclonal antibody.

**7.** A bifunctional linker compound of

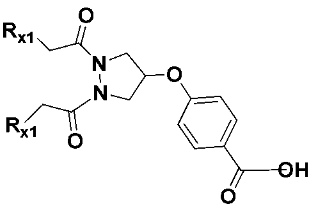 , 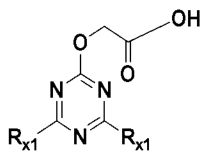 , 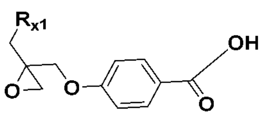 , 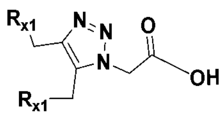 ,

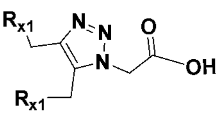 , 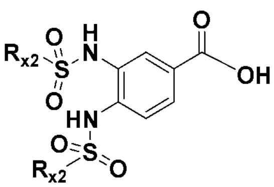 , 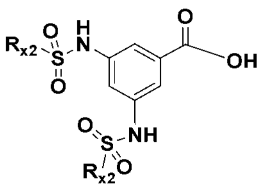 , 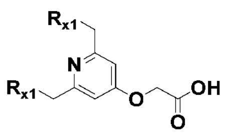 ,

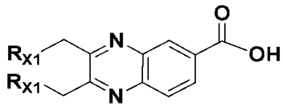, or 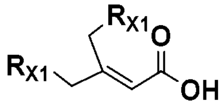;

wherein, $R_{X1}$ and $R_{X2}$ are each independently selected from halogen or $C_2$-$C_6$ alkenyl;
preferably, $R_{X1}$ is selected from bromine or chlorine, and $R_{X2}$ is vinyl.

**8.** A method for preparing the drug-antibody conjugate compound represented by formula (III) or the pharmaceutically acceptable salt thereof according to claim 6, comprising the following step:
linking a compound represented by formula (I) or a pharmaceutically acceptable salt, stereoisomer, or prodrug thereof to the antibody or the antigen-binding fragment thereof.

**9.** A pharmaceutical composition, comprising the compound or the pharmaceutically acceptable salt, stereoisomer, or prodrug thereof according to any one of claims 1-6 or the antibody-drug conjugate according to claim 6, and a pharmaceutically acceptable excipient.

**10.** Use of the compound or the pharmaceutically acceptable salt, stereoisomer, or prodrug thereof according to any one of claims 1-5, the drug-antibody conjugate according to claim 6, the bifunctional linker compound according to claim 7, or the pharmaceutical composition according to claim 9 in the manufacture of a medicament for treating cancer,
preferably, the cancer comprises gastric cancer, esophageal cancer, cardia cancer, breast cancer, ovarian cancer, colon cancer, rectal cancer, primary liver cancer, pancreatic cancer, acute and chronic granulocytic leukemia, chorionic epithelial cancer, lung cancer, bladder cancer, intestinal cancer, or small cell lung cancer; more preferably, the cancer is esophageal cancer, pancreatic cancer, or gastric cancer, and further preferably, the cancer is pancreatic cancer.

**11.** A method for treating cancer, comprising the step of administering to a patient in need thereof a therapeutically effective amount of the compound or the pharmaceutically acceptable salt, stereoisomer, or prodrug thereof according to any one of claims 1-5, the drug-antibody conjugate according to claim 6, the bifunctional linker compound according to claim 7, or the pharmaceutical composition according to claim 9,
preferably, the cancer comprises gastric cancer, esophageal cancer, cardia cancer, breast cancer, ovarian cancer, colon cancer, rectal cancer, primary liver cancer, pancreatic cancer, acute and chronic granulocytic leukemia, chorionic epithelial cancer, lung cancer, bladder cancer, intestinal cancer, or small cell lung cancer; more preferably, the cancer is esophageal cancer, pancreatic cancer, or gastric cancer, and further preferably, the cancer is pancreatic cancer.

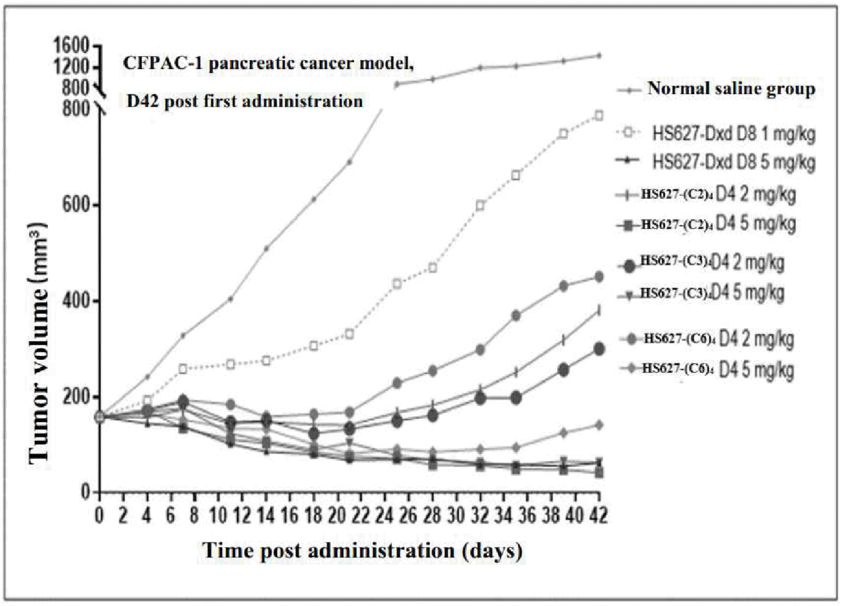


Figure 1

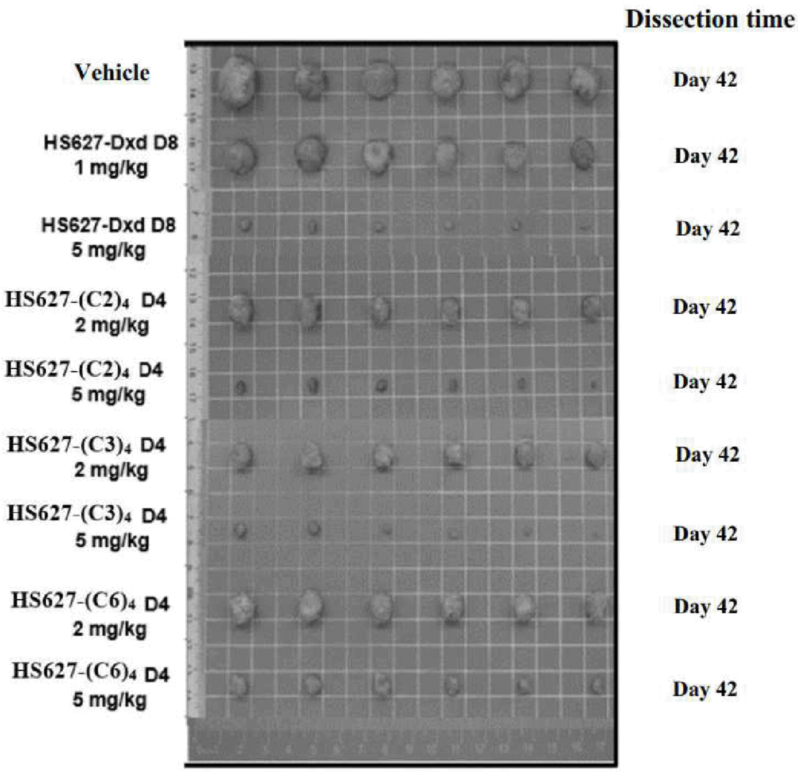


Figure 2

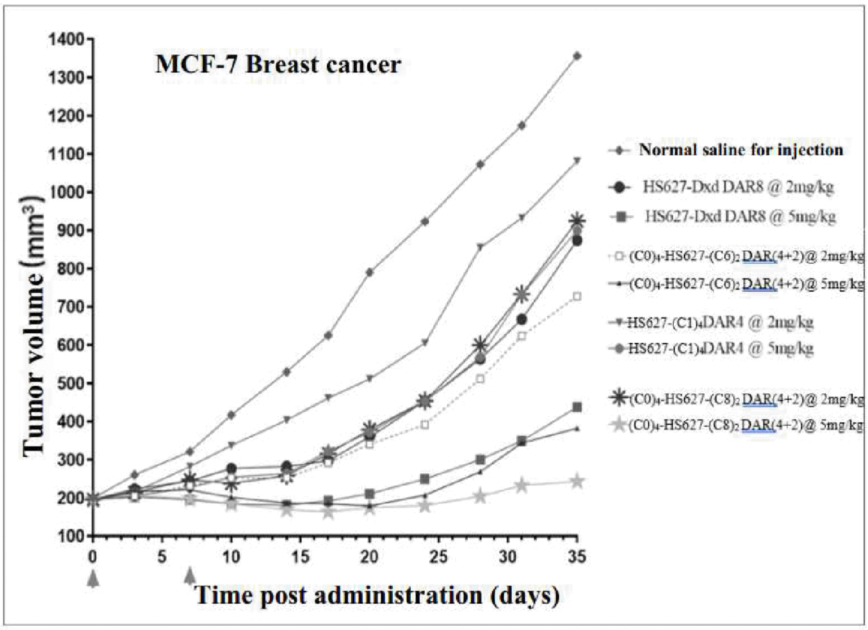


Figure 3

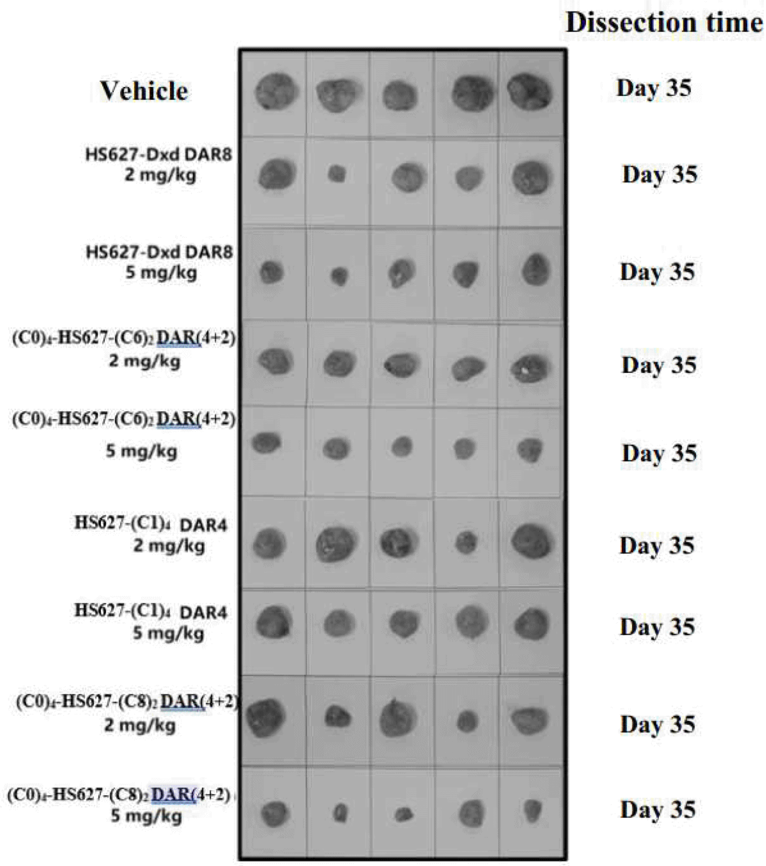


Figure 4

**INTERNATIONAL SEARCH REPORT**

International application No. **PCT/CN2024/110323**

**A. CLASSIFICATION OF SUBJECT MATTER**

C07K 5/027(2006.01)i; A61K 47/68(2017.01)i; A61K 38/07(2006.01)i; A61P 35/00(2006.01)i; A61P 35/02(2006.01)i; C07K 5/062(2006.01)i; C07K 5/083(2006.01)i

According to International Patent Classification (IPC) or to both national classification and IPC

**B. FIELDS SEARCHED**

Minimum documentation searched (classification system followed by classification symbols)

IPC:C07K A61K A61P

Documentation searched other than minimum documentation to the extent that such documents are included in the fields searched

Electronic data base consulted during the international search (name of data base and, where practicable, search terms used)

CNABS; CNTXT; ENTXTC; ENTXT; STN; Web of Science; DWPI; CNKI: 抗体药物偶联, 抗体偶联药物, 抗体-药物偶联, 配体药物偶联, 双功能, 双接头, bis, conjugate, antiboby, drug, ADC, 肿瘤, 癌症, cancer, tumor, structural formula search

**C. DOCUMENTS CONSIDERED TO BE RELEVANT**

| Category* | Citation of document, with indication, where appropriate, of the relevant passages | Relevant to claim No. |
|---|---|---|
| PX | CN 117069793 A (HANGZHOU ADCORIS BIOPHARMA CO., LTD.) 17 November 2023 (2023-11-17)<br>claims 1-11 | 1-11 |
| PX | WO 2023173026 A1 (SORRENTO THERAPEUTICS, INC.) 14 September 2023 (2023-09-14)<br>embodiment S48, and claims 50-53 and 120-121 | 1-11 |
| X | Kay, G. et al. "Coupling of Enzymes to Cellulose Using Chloro-s-triazines" *Nature,* Vol. vol. 216, 01 November 1967 (1967-11-01), pp. 514-515<br>ISSN: 1476-4687,<br>Table 1 | 7 |
| X | CN 108472284 A (VENATORX PHARMACEUTICALS INC.) 31 August 2018 (2018-08-31)<br>embodiment 32 | 7 |
| X | CN 115243727 A (SYNAFFIX B.V.) 25 October 2022 (2022-10-25)<br>embodiment 42, and claims 1, 9 and 13 | 1-11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 October 2024** | **16 November 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/110323**

**C. DOCUMENTS CONSIDERED TO BE RELEVANT**

| Category* | Citation of document, with indication, where appropriate, of the relevant passages | Relevant to claim No. |
|---|---|---|
| X | ZHENG, Y.C., et al. "Triazole-Dithiocarbamate Based Selective Lysine Specific Demethylase 1 (LSD1) Inactivators Inhibit Gastric Cancer Cell Growth, Invasion, and Migration" *J. Med. Chem,* Vol. vol. 56, 16 October 2013 (2013-10-16), pp. 8543-8560 ISSN: 1520-4804, Scheme 2 | 7 |
| X | WO 2023078273 A1 (HANGZHOU DAC BIOTECH CO., LTD.) 11 May 2023 (2023-05-11) claims 1, 3-4, 6-14 and 17-19 | 1-11 |
| A | WO 2022022649 A1 (CHENGDU SCIMOUNT PHARMATECH CO., LTD.) 03 February 2022 (2022-02-03) claims 1-19 | 1-11 |
| A | WO 2023124963 A1 (KUNSHAN XINYUNDA BIOTECH CO., LTD.) 06 July 2023 (2023-07-06) claims 1-18 | 1-11 |

Form PCT/ISA/210 (second sheet) (July 2022)

**INTERNATIONAL SEARCH REPORT**

International application No.
**PCT/CN2024/110323**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **11**
because they relate to subject matter not required to be searched by this Authority, namely:

Claim 11 relates to a cancer treatment method, which falls within methods for treatment of a human or animal body by therapy, and falls within subject matter for which no search is required by the International Searching Authority as defined in PCT Rule 39.1(iv). The search is carried out on the basis that the method is reasonably expected to be the subject matter of a pharmaceutical use claim, i.e. "the use for the preparation of a drug for treating cancer".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/110323**

| Patent document cited in search report | Publication date (day/month/year) | Patent family member(s) | Publication date (day/month/year) |
|---|---|---|---|
| CN 117069793 A | 17 November 2023 | None | |
| WO 2023173026 A1 | 14 September 2023 | None | |
| CN 108472284 A | 31 August 2018 | WO 2017044828 A1 | 16 March 2017 |
| | | EP 3347008 A1 | 18 July 2018 |
| | | EP 3347008 A4 | 10 April 2019 |
| | | EP 3347008 B1 | 09 March 2022 |
| | | US 2017073360 A1 | 16 March 2017 |
| | | US 10399996 B2 | 03 September 2019 |
| | | US 2020055877 A1 | 20 February 2020 |
| | | US 11046716 B2 | 29 June 2021 |
| CN 115243727 A | 25 October 2022 | WO 2021144314 A1 | 22 July 2021 |
| | | EP 4090379 A1 | 23 November 2022 |
| | | JP 2023510850 A | 15 March 2023 |
| | | US 2023114866 A1 | 13 April 2023 |
| WO 2023078273 A1 | 11 May 2023 | WO 2022078524 A2 | 21 April 2022 |
| | | WO 2022078524 A3 | 25 August 2022 |
| | | WO 2022078524 A4 | 08 December 2022 |
| | | CA 3236754 A1 | 11 May 2023 |
| | | WO 2023078021 A1 | 11 May 2023 |
| | | TW 202334217 A | 01 September 2023 |
| | | CA 3236930 A1 | 21 April 2022 |
| | | KR 20240095442 A | 25 June 2024 |
| | | CA 3236852 A1 | 11 May 2023 |
| | | KR 20240095316 A | 25 June 2024 |
| | | EP 4426729 A1 | 11 September 2024 |
| | | EP 4426727 A2 | 11 September 2024 |
| | | EP 4426741 A1 | 11 September 2024 |
| | | AU 2022383265 A1 | 13 June 2024 |
| | | AU 2022381163 A1 | 13 June 2024 |
| | | AU 2021362997 A1 | 16 May 2024 |
| WO 2022022649 A1 | 03 February 2022 | None | |
| WO 2023124963 A1 | 06 July 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023109887077
- US 5821337 A